(19) Europäisches Patentamt
European Patent Office
Office européen des brevets

(11) EP 1 811 840 B1

(12) EUROPEAN PATENT SPECIFICATION

(45) Date of publication and mention of the grant of the patent:
**13.04.2016 Bulletin 2016/15**

(21) Application number: **05810263.3**

(22) Date of filing: **07.09.2005**

(51) Int Cl.:
***A01N 43/08*** *(2006.01)* ***A01N 43/16*** *(2006.01)*
***A61K 31/7012*** *(2006.01)* ***A61P 35/00*** *(2006.01)*
***A61P 9/14*** *(2006.01)*

(86) International application number:
**PCT/US2005/031900**

(87) International publication number:
**WO 2006/029221 (16.03.2006 Gazette 2006/11)**

(54) **ANTI-TUMOR COMPOUNDS WITH ANGELOYL GROUPS**

ANTITUMORZUSAMMENSETZUNGEN MIT ANGELOYL-GRUPPEN

COMPOSES ANTITUMORAUX COMPRENANT DES GROUPES ANGELOYL

(84) Designated Contracting States:
**AT BE BG CH CY CZ DE DK EE ES FI FR GB GR HU IE IS IT LI LT LU LV MC NL PL PT RO SE SI SK TR**

(30) Priority:
**07.09.2004 US 607858 P**
**27.09.2004 US 613811 P**
**08.10.2004 US 617379 P**
**08.10.2004 PCT/US2004/033359**
**23.12.2004 PCT/US2004/043465**
**14.02.2005 US 906303**
**27.04.2005 US 117760**
**27.04.2005 US 675282 P**
**27.04.2005 US 675284 P**
**17.05.2005 US 131551**

(43) Date of publication of application:
**01.08.2007 Bulletin 2007/31**

(73) Proprietor: **Mak, May Sung**
**Kowloon**
**Hong Kong (CN)**

(72) Inventors:
- **CHAN, Pui-Kwong**
  **Sugarland, TX 77429 (US)**
- **MAK, May, Sung**
  **Kowloon, Hong Kong (CN)**
- **WANG, Yun**
  **Dunedin, New Zealand (NZ)**

(74) Representative: **Lecca, Patricia S. et al**
**Cabinet Lecca**
**21 rue de Fécamp**
**75012 Paris (FR)**

(56) References cited:
**WO-A-2005/037200 US-A1- 2003 096 030**
**US-B2- 6 616 943**

- **JIANG Z ET AL: "Six triterpenoid saponins from Mmaesa laxiflora." JOURNAL OF NATURAL PRODUCTS JUN 1999, vol. 62, no. 6, June 1999 (1999-06), pages 873-876, XP002510553 ISSN: 0163-3864**
- **KONOSHIMA T ET AL: "ANTITUMOR AGENTS 82. CYTOTOXIC SAPOGENOLS FROM AESCULUS-HIPPOCASTANUM" JOURNAL OF NATURAL PRODUCTS (LLOYDIA), vol. 49, no. 4, 1986, pages 650-656, XP002510554 ISSN: 0163-3864**
- **D'ACQUARICA I ET AL: "Isolation and structure elucidation of four new triterpenoid estersaponins from fruits of Pittosporum tobira ait" TETRAHEDRON, ELSEVIER SCIENCE PUBLISHERS, AMSTERDAM, NL, vol. 58, no. 51, 16 December 2002 (2002-12-16), pages 10127-10136, XP004396869 ISSN: 0040-4020**
- **SEO YOUNGWAN ET AL: "A new triterpene saponin from Pittosporum viridiflorum from the Madagascar rainforest." JOURNAL OF NATURAL PRODUCTS JAN 2002, vol. 65, no. 1, January 2002 (2002-01), pages 65-68, XP002510555 ISSN: 0163-3864**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



**(Cont. next page)**

- **CHEN Y ET AL: "STUDIES ON THE CONSTITUENTS OF XANTHOCERAS SORBIFOLIA BUNGE. II. MAJOR SAPOGENOL AND A PROSAPOGENIN FROM THE FRUITS OF XANTHOCERAS SORBIFOLIA BUNGE" CHEMICAL AND PHARMACEUTICAL BULLETIN, PHARMACEUTICAL SOCIETY OF JAPAN, TOKYO, vol. 32, no. 9, 1 January 1984 (1984-01-01), pages 3378-3383, XP001206882 ISSN: 0009-2363**
- **CHEN Y ET AL: "Studies on the constituents of xanthoceras sorbifolia BUNGE. III. Minor prosapogenins from the fruits of xanthoceras sorbifolia BUNGE" CHEMICAL AND PHARMACEUTICAL BULLETIN, PHARMACEUTICAL SOCIETY OF JAPAN, TOKYO, vol. 33, no. 1, 1 January 1985 (1985-01-01), pages 127-134, XP002961625 ISSN: 0009-2363**
- **VOUTQUENNE L ET AL: "Haemolytic acylated triterpenoid saponins from Harpullia austro-caledonica" PHYTOCHEMISTRY, PERGAMON PRESS, GB, vol. 66, no. 7, 1 April 2005 (2005-04-01), pages 825-835, XP004846755 ISSN: 0031-9422**
- **DIZES C ET AL: "Harpuloside a triterpenoid saponin from Harpullia ramiflora" PHYTOCHEMISTRY, PERGAMON PRESS, GB, vol. 48, no. 7, 1 August 1998 (1998-08-01), pages 1229-1232, XP004289959 ISSN: 0031-9422**
- **D'ACQUARICA I. ET AL.: 'Isolation and Structure Elucidation of Four New Triterpenoid Estersaponins from Fruits of Pittosporum Tobira AIT' TETRAHEDRON vol. 58, no. 51, 2002, pages 10127 - 10136, XP004396869**

## Description

This application is a Continuation-In-Part of U.S. Serial No. 11/131,551, filed May 17, 2005, a Continuation-In-Part of U.S. Serial No. 11/117,760, filed April 27, 2005, Continuation-In-Part application of U.S. Serial No. 10/906,303, filed February 14, 2005, Continuation-In-Part application of International Application No. PCT/US04/43465, filed December 23, 2004, which is a Continuation-In-Part application of Int'l App'l No. PCT/US04/33359, filed October 8, 2004, which claims the benefit of U.S. Serial Nos. 60/532,101, filed December 23, 2003, and 60/509,851, filed October 9, 2003; and which claims the benefit of U.S. Serial Nos. 60/617,379, filed October 8, 2004, 60/613,811, filed September 27, 2004, and 60/607,858, filed September 7, 2004, 60/675,282, Filed April 27, 2005, and 60/675,284, Filed April 27, 2005.

## **FIELD** OF THE INVENTION

This invention relates to novel anti-tumor compounds obtainable from Xanthoceras sorbifolia and plants from the sapindaceae family.

## BACKGROUND OF THE INVENTION

Wenguanguo is a species of the sapindaceae family. Its scientific name is Xanthoceras sorbifolia Bunge. Wenguanguo is the common Chinese name. Others are Wenguannguo, Wenguanmu, Wenguanhua, Xilacedeng, Goldenhorn and Yellowhorn. Wenguanguo is grown in Liaoning, Jilin, Hebei, Shandong, Jiangsu, Henan, Shanxi, Shaanxi, Gansu, Ningxia and Inner Mongolia, China. Its seeds, leaves and flowers are edible and have been used as a folk or traiditional medicine for centuries. Its branches and woods are also used as a folk or traditional medicine.

Yingjie Chen, Tadahiro Takeda and Yukio Ogihara reported in Chem. Pharm. Bull 33(4)1387-1394(1985) describes a study on Xanthoceras sorbifolia Bunge. See Section V. Saponins from the Fruits of Xanthoceras sorbifolia. Four new saponin compounds were isolated from the fruits of Xanthoceras sorbifolia Bunge. These structures are bunkankasaponins A, B, C and D. Yingjie Chen, Tadahiro Takeda and Yukio Ogihara reported in Chem. Pharm. Bull 33(3)1043-1048(1985) is another a study focused on the Xanthoceras sorbifolia Bunge. See Section IV. Structures of the Miner Prosapogenin. Yingjie Chen, Tadahiro Takeda and Yukio Ogihara. Chem. Pharm. Bull 33(1)127-134(1985) describes yet another study on the Xanthoceras sorbifolia Bunge. See Section III. Minor Prosapogenins Saponins from the Fruits of Xanthoceras sorbifolia Bunge. Other studies which have focused on saponin compounds include: Laurence Voutquenne, Cecile Kokougan. Catherine Lavaud, Isabelle Pouny, Marc Litaudon."Triterpenoid saponins and Acylated prosapogenins from Harpullia austro-caledonica." Phytochemistry 59 (2002) 825-832. Laurence Voutquenne et al. "Haemolytic acylated triterpenoil saponins from Harpullia austro-caledonica" Phytochemistry 66(2005)825-835. Zhong Jaing, Jean-francois Gallard, Marie-Therese Adeline, Vincent Dumontet, Mai Van Tri, Thierry Sevenet, and Mary Pais "Six Triterpennoid Saponins from Maesa laxiflora." .J. Nat. Prod. (1999), 62, 873-876. Young Seo, John M. Berger, Jennine Hoch, Kim M Neddermann, Isia Bursuker, Steven W. Mamber and David G. Kingston. "A new Triterpene Saponin from Pittosporum viridiflorum from the Madagascar Rainforest ". J. Nat. Prod. 2002, 65, 65-68. Xiu-Wei Yang, Jing Zhao, Xue-Hui Lui, Chao-Mei Ma, Masao Hattori, and Li He Zhang "Anti-HIV-1 Protease Triterpenoid Saponins from the Seeds of Aesculus chinensis." J. Nat. Prod. (1999), 62, 1510-1513. Yi Lu, Tatsuya Umeda, Akihito Yagi, Kanzo Sakata, Tirthankar Chaudhuri, D.K. Ganguly, Secion Sarma. "Triterpenoid Saponins from the roots of the tea plant (Camellia sinensis var. Assamica)." Phytochchemistry 53 (2000) 941-946. Sandra Apers, Tess E. De Bruyne, Magda Claeys, Arnold J. Viletinck, Luc A.C. Pieters. "New acylated triterpenoid saponins from Maesa laceceolata." Phytochemistry 52 (1999) 1121-1131. Ilaria D'Acquarica, Maria Cristina, Di Giovanni, Francesco Gasparrini, Domenico Misiti, Claudio D' Arrigo, Nicolina Fagnano, Decimo Guarnieri, Giovanni Iacono, Giuseppe Bifulco and Raffaele Riccio. "Isolation and structure elucidation of four new triterpenoid estersaponins from fruits of the Pittosporumtobira AIT." Tetrahedron 58 (2002) 10127-10136.

However, these references do not disclose saponin compounds having the same structure as the compounds of this invention which are capable of inhibiting cancer or tumor cell growth.

Cancer cells are defined by two heritable properties: (1) they reproduce in defiance of normal restraints on cell division; and (2) they invade and colonize territories normally reserved for other cells. Cancers require mutations of one to many genes for its development, and they are classified according to the tissue and cell type from which they arise. Cancers arising from epithelial cells are named carcinomas; those arising from connective tissue or muscle cells are named sarcomas. In addition, there are cancers called leukemias, which are derived from hemopaietic cells. Cancers can also develop from cells of the nervous system.

Ovarian cancer is the 5th leading cause of cancer death in women, and the leading cause of death from gynecologic malignancies. In the United States, females have a 1.4 to 2.5%, or 1 out of 40-60 women, lifelong chance of developing ovarian cancer. Older women are at highest risk. More than half of the deaths from ovarian cancer occur in women between 55 and 74 years of age, and approximately one quarter of ovarian cancer deaths occur in women between 35 and 54 years of age. *See MedlinePlus Encyclopedia on ovarian cancer at* http://www.nlm.nih.gov/medlineplus/ency/article/000889.htm.

Ovarian cancer is disproportionately deadly for a number of reasons. First, symptoms are vague and non-specific, so women and their physicians frequently attribute them to more common conditions. By the time the cancer is diagnosed, the tumor has often spread beyond the ovaries. Also, ovarian cancers shed malignant cells that frequently implant on the uterus, bladder, bowel, and lining of the bowel wall (omentum). These cells can begin forming new tumor growths before cancer is even suspected. Second, because no cost-effective screening test for ovarian cancer exists, more than 50 percent of women with ovarian cancer are diagnosed in the advanced stages of the disease.

Therefore, it is an object of this invention to provide compounds and/or compositions extracted from Xanthoceras sorbifolia or plants, or chemically or biochemically synthesized, which have substantial potency against ovarian cancer.

## SUMMARY OF THE INVENTION

In accordance with these and other objects of the invention, a brief summary of the present invention is presented. Some simplifications and omission may be made in the following summary, which is intended to highlight and introduce some aspects of the present invention, but not to limit its scope. Detailed descriptions of a preferred exemplary embodiment adequate to allow those of ordinary skill in the art to make and use the invention concepts will follow in later sections.

The invention provides four novel compounds of the structure (Y2, Y or Y3, Y8, Y10) as shown in Figure 1. As used herein, "Structure Y" is also referred to as "Structure Y3".

**Y**

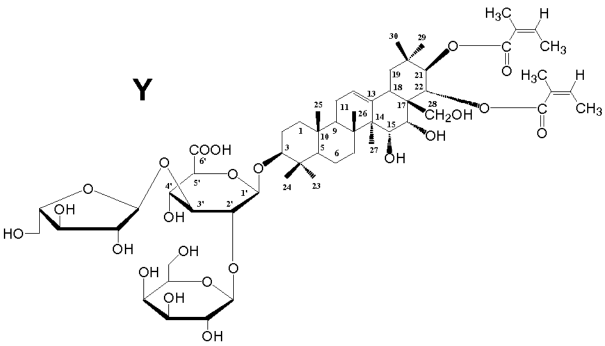

**Y2**

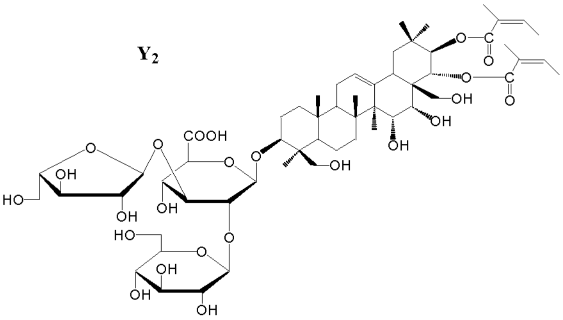

**Y-8**

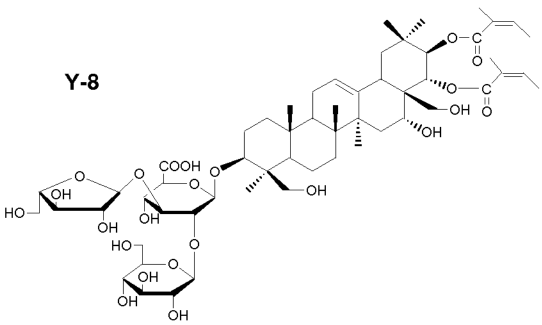

**Y-10**

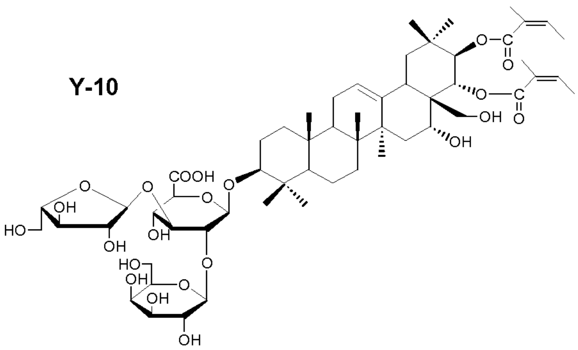

The formula, chemical name and common name of these compounds are presented in Table 1 below.

**Table 1. Formula, Chemical Name and Common Name of Four Novel Compounds of Structure (Y, Y2, Y8, Y10)**

| Names | Formula | Chemical Name |
|---|---|---|
| Xanifolia-**Y (Y3)** | $C_{57}H_{88}O_{23}$ | 3-O-[β-D-galactopyranosyl(1→2)]-α-L-arabinofuranosyl(1→3)-β-D-glucuronopyranosyl-21,22-O-diangeloyl-3β, 15α, 16α, 21β, 22α, 28-hexahydroxyolean-12-ene, |
| Xanifolia-**Y2** | $C_{57}H_{88}O_{24}$ | 3-O-[β-D-glucopyranosyl-(1→2)]-α-L-arabinofuranosyl(1→3)-(-D-glucuronopyranosyl-21,22-O-diangeloyl-3*β*, 15*α*, 16*α*, 21*β*, 22*α*, 24*β*, 28-heptahydroxyolean-12-ene |
| Xanifolia-**Y8** | $C_{57}H_{88}O_{23}$ | 3-O-[*β*-galactopyranosyl (1→2)]-α-arabinofuranosyl (1→3)-*β*-glucuronopyranosyl-21, 22-O-diangeloyl-3*β*, 16*α*, 21*β*, 22*α*, 24*β*, 28-hexahydroxyolean-12-ene |

(continued)

| Names | Formula | Chemical Name |
|---|---|---|
| Xanifolia-**Y10** | $C_{57}H_{88}O_{22}$ | 3-O-[$\beta$-galactopyranosyl (1→2)]-$\alpha$-arabinofuranosyl (1→3)-$\beta$-glucuronopyranosyl-21, 22-O-diangeloyl-3$\beta$, 16$\alpha$, 21$\beta$, 22$\alpha$, 28-pentahydroxyolean-12-ene |

The above four compounds (Y, Y2, Y8, and Y10) have anti-cancer effect. These compounds inhibit the growth of human ovarian and other cancer cells. See Figure 2, 3 and 4.

A concensus sub-structure was derived or identified from the bioactive compounds (Y, Y2, Y8, and Y10) of the invention. A concensus sub-structure of the compounds of the invention is a biangeloyl group located on adjacent carbons. For example, in structure Y, Y2, Y8 and Y10, the biangeloyl group is located at 21β and 22α of the triterpene backbone. In an embodiment, the biangeloyl group of the bioactive compounds (Y, Y2, Y8, and Y10) of the invention is situated in a trans-position in adjacent carbons of a structure. In another embodiment, a sugar moiety or sugar chain is located at C3 of the triterpene. In a further embodiment, the sugar moiety or sugar chain selected from the group consisting of: D-glucose, D-galactose, L-rhamnose, L-arabinose, D-xylose, alduronic acid, D-glucuronic acid and D-galacturonic acid.

Studies on the structural and functional relationship of the four compounds of the invention indicate that changes to or substitutions of the functional groups located at C15 and C24 of the triterpene do not affect anticancer activity. The compounds (Y, Y2, Y8, and Y10) of the invention have activity in inhibition of turmor growth. See Figure 2, 3 and 4. The compounds are prepared by a chromatograpy purification process involving FPLC and HPLC as described in Figure 6, 7, 8, 9, 10, 11. Compound Y is purified using a procedure described in this application. Figure 9A shows the NMR peaks of compound Y.Comparision of Figure 2 and Figure 12 shows that the purified compound Y possesses potency (IC50 =1.5 ug/ml) 10 times higher than the original extract (IC50 = 25ug/ml). Compound Y also has a high selectivity toward ovarian cancer. See Figure 13.

The purified compound Y, Y1, Y2, Y8, Y9, and Y10 also show inhibitory activity toward human cancer cells with a higher potency toward ovarian carcinoma. See Figure 3 and 4. The plant extract containing compound Ys also shows inhibitory activity toward human cancer cells. For example, studies were performed on eleven human cancer cell lines, and the plant extract of the invention shows a higher potency toward ovarian carcinoma. See Figures 12, 13 and 14 and Table 3.1 for a comparison of the activities of the plant extract of the invention among the different cancer cell lines. As used herein, "Ys" or "compound Ys" is used to denote compound Y or Y3, Y1, Y2, Y8, Y9, Y10, or other bioactive compounds obtainable from a Xanthoceras sorbifolia extract of the invention.

This invention provides an extract of Xanthoceras sorbifolia capable of inhibiting cancer growth. The cancer includes, but is not limited to ovary cancer, bladder cancer, prostate cancer, leukocytes cancer, and bone cancer. Compounds of the invention, shown to be effective against cancer, can be isolated from the plant called Xanthoceras sorbifolia, synthesized chemically, or extracted from other biological sources, including plants from the sapindaceae family.

This invention provides a process of producing bioactive compounds from husks, leaves, branches or stems, fruit-stems, seed shell, roots and barks of the Wenguanguo. The extraction of the bioactive compounds of the invention from different parts of the Wenguangu plant can be performed separately or simultaneously. This invention further discloses methods of obtain the bioactive compounds of the invention. In addtion to saponin comopunds, the extracts of the invention also contain saccharides, proteins, glycosides, flavonoids, curmarin extracts, alkaloid extracts, organic acid extracts, tannin and other bioactive compounds. The saponin compounds obtainable from the extract of the invention were investigated, and have been shown to possess inhibitory activity against cancer growth.

The compounds, extracts or compositions of the present invention is capable of regulating many cellular pathways, including the receptors or components of a cell, such as G-protein receptor, Fas protein, receptor Tyrosine Kinases, Mitogen, mitogen receptor. The compounds of the invention can be isolated from the plant called Xanthoceras sorbifolia, synthesized chemically, or extracted from other biological sources, including plants from the sapindaceae family.

This invention provides compounds, including compound of structures Y, Y1, Y2, Y8, Y9 and Y10, obtainable from Xanthoceras sorbifolia and capable of inhibiting cancer growth. In an embodiment, the cancer includes but is not limited to bladder cancer, cervix cancer, prostate cancer, lung cancer, breast cancer, leukocytes cancer, colon cancer, liver cancer, bone cancer, brain cancer, and ovary cancer. This invention provides a compound of oleanene triterpenoidal saponin comprising a side chain at Carbon 21 and Carbon 22 of said compound, wherein the side chain comprises angeloyl groups. In an embodiment, the compound comprises one or more sugars, wherein C3 and C4 of the sugar are acylated with angeloyl groups. This invention provides a triterpinoidal saponin compound comprising a triterpene backbone and a biangeloyl group, wherein the one angeloyl group is attached to 21β and one angeloyl group is attached to 22α of the triterpene backbone, and wherein the presence of the biangeloyl group produces anticancer activity. This

invention provides a triterpenoidal saponin compound comprising a triterpene backbone and a sugar moiety or rhamnose, wherein the sugar moiety or rhamnose is attached to the triterpene backbone, wherein the sugar moiety or rhamnose further comprises a biangeloyl group, and wherein the presence of the biangeloyl group produces anticancer activity. This invention provides a triterpenoidal saponin compound comprising a triterpene backbone, said triterpene backbone is acylated at either 21β or 22α position or at both 21β and 22α position with a sugar moiety or sugar chain, and wherein at least one sugar in the sugar moiety or sugar chain comprises angeloyl groups attached to the C3 and C4 position of said sugar. In an embodiment, the two angeloyl groups are in a trans-position on a structure, and the presence of the biangeloyl group produces anticancer activity. In an embodiment, the two angeloyl groups are in a trans-position in adjacent carbons on a structure, and the presence of the biangeloyl group produces anticancer activity. This invention provides a salt of the above-described compounds. This invention provides a pharmaceutical composition comprising an effective amount of the above-described compounds and a pharmaceutically acceptable carrier(s).

This invention provides a method for isolating compounds from Xanthoceras sorbifolia comprising the steps of: extracting Xanthoceras sorbifolia powder with an appropriate amount of an organic solvent for an appropriate amount of time to obtain an extract, identifying the bioactive compounds in the extract; purifying the bioactive compounds in the extract with FPLC to obtain a fraction of the bioactive compounds; and isolating the purified individual bioactive compound from the fraction of the bioactive compounds with preparative HPLC. This invention provides a compound having a structure verified by NMR spectral data derived from proton NMR, carbon NMR, 2D NMR of the Heteronuclear Multiple Quantum Correlation (HMQC), Heteronuclear Multiple Bond Correlation (HMBC), NOESY and COSY, and Mass spectral data derived from MALDI-TOF and ESI-MS.

This invention provides a compound and its derivatives which are effective against cancer. The compounds or compositions of the present invention are capable of regulating various cellular pathways, including but not limiting the following: receptors or components, such as G-protein receptor, Fas protein, receptor for Tyrosine Kinases, mitogens, mitogen receptors, TGF Beta-smad, FGF, TGF-beta and TGF-alpha, ras-GTPase-MAP kinase, jun-fos, Src-fyn, Jak-Jnk-STAT, BMP, Wnt, or myc-cell proliferation. The compounds and composition of the invention derivied from Xanthoceras Sorbifolia is also capable of regulating the components and receptors associated with cell death, and are capable of (re)activating the cell death program or pathways.

## DETAILED DESCRIPTION OF THE FIGURES

**Figure 1** shows the structures of six bioactive anticancer saponin compound of the invention.

**Figure 2** shows the anticancer activity of purified Compound Y. The experiment was performed on ovarian cancer cells (OCAR-3) and the inhibition activity was determined by MTT assay. For details, refer to Experiment 3. Abscissa: Concentration (ug/ml). Ordinate: % Cell Growth. The IC50 is approximately 1-1.5 ug/ml. A: Point scale. B: Linear scale.

**Figure 3** shows the inhibition of the purified Compound Y1 and Compound Y2 on the growth of ovarian cancer cells.

**Figure 4** shows the anticancer activity of Y, Y8, Y9 and Y10 on ovarian cancer cells as determined by MTT assay.

**Figure 5** shows a consensus structure derived from four bioactive anticancer saponin compounds of the invention (i.e., Y, Y2, Y8 and Y10).

**Figure 6** shows the separation of the components of Xanthoceras sorbifolia extract by HPLC with a μbondapak C18 column. Details of the experiment were presented in Experiment 2.

**Figure 7** shows the elution profile of an extract of Xanthoceras sorbifolia in FPLC with 10-80% gradient. Ordinate: Optical density (at 245nm). Abscissa: Fractions (5ml/fraction).

**Figure 8** shows the results of the screening of cell growth activity using fractions obtained from FPLC chromatography. The assay was conducted in bladder cells. The fractions obtained from FPLC as shown in Figure 9 were used. As shown in this figure, different components of Xanthoceras sorbifolia extracts cause either growth or inhibition effects on cells. Only fraction 5962 (Fraction Y) causes cell inhibition. Fractions 610, 1116 and 1724 cause minor stimulation of cell growth. Abscissa: concentration (ug/ml). Ordinate: % Cell Growth (determined by MTT assay).

**Figure 9** shows HPLC profile of Fraction Y with 45% acetonitrile isocratic elution in a preparative C18 column (Delta Pak C18). Under these conditions, fractions Y (Y3), Y1 and Y2 are well separated from each other and they are subsequently purified. A and B shows the purity of the collected Y3 and Y2 by HPLC under same conditions.

**Figure 10** shows the separation profile of Y8, Y9 andγ10 with 45% acetonitrile isocratic elution in a preparative C18 column (Delta Pak C18).

**Figure 11** shows the HPLC profiles of purified Y8, Y9 and γ10.

**Figure 12** shows the growth curves of ovarian cancer cells after treatment with the crude extract of Xanthoceras sorbifolia as determined by the MTT assay. The two curves in the figure are two experiments results. This study determined the efficacy of the extract of Xanthoceras sorbifolia on cancer cells. In these experiments, cancer cell

lines from 11 different human organs were employed. This figure shows that ovary cancer cells are the most sensitive cancer cells in responding to Xanthoceras Sorbifolia extract of the invention. Results of other cancer cells were represented in Figures 16A, 16B, 16C. Abscissa: concentration (ug/ml). Ordinate: % Cell Growth (determined by MTT assay).

**Figure 13** shows the comparison of potency of Compound Y in ovarian cancer cells and cervical cancer cells. Ovarian cancer cells are much more sensitive than the cervical cancer cells. The IC50 for Compound Y in ovary cells is about 1.5ug/ml while the IC50 in cervical cancer cells is over 20 ug/ml.

**Figures 14A** shows the growth curves of sensitive group of bladder and bone cancer cells as determined by MTT assay. The curves in the figure are the repeated experiments results. Abscissa: concentration (ug/ml). Ordinate: % Cell Growth (determined by MTT assay).

**Figures 14B** shows the growth curves of semi-sensitive group: leukocyte, liver; prostate, breast and brain cancer cells as determined by MTT assay. The curves in the figure are the repeated experiments results. Abscissa: concentration (ug/ml). Ordinate: % Cell Growth (determined by MTT assay).

**Figures 14C** shows the growth curves of least sensitive: colon, cervix and lung cancer cells as determined by MTT assay. The curves in the figure are the repeated experiments results. Abscissa: concentration (ug/ml). Ordinate: % Cell Growth (determined by MTT assay).

**Figure 15** shows the structure of Compound Y having the formula of $C_{57}H_{88}O_{23}$ and the chemical name of 3-O-[β-D-galactopyranosyl(1→2)]-α-L-arabinofuranosyl(1→3)-β-D-glucuronopyranosyl-21,22-O-diangeloyl-3β, 15α, 16α, 21β, 22α, 28-hexahydroxyolean-12-ene.

**Figure 16**shows the sprectrum of proton NMR of Compound Y.

**Figure 17** shows 2D NMR (HMQC) results of Compound Y.

**Figure 18** shows 2D NMR (HMBC) results of Compound Y.

**Figure 19** shows the mass spectrum of compound Y with MALDI-TOF (high mass): Y + Matrix (CHCA) + Angiotensin 1 "two point calibration".

**Figure 20** shows the mass spectrum of compound Y with ESI-MS.

**Figure 21** shows the structure of Compound Y1 having the formula of $C_{65}H_{100}O_{27}$ and the chemical name of 3-O-[β-D-galactopyranosyl(1→2)]-α-L-arabinofuranosyl(1→3)-β-D-glucuronopyranosyl-21-O-(3,4-diangeloyl)-α-L-rhamnophyranosyl-22-O-acetyl-3β,16α, 21β, 22α, 28-pentahydroxyolean-12-ene.

**Figure 22** shows the Proton NMR spectrum of Compound Y1.

**Figure 23** shows the 2D NMR (HMQC) results of Compound Y1.

**Figure 24** shows the 2D NMR (HMBC) results of Compound Y1.

**Figure 25** shows COSY-NMR profile of Compound Y1.

**Figure 26** shows the chemical structure and the chemical name of Compound Y2.

**Figure 27** shows the proton NMR spectrum of Y2.

**Figure 28** shows the 2D NMR spectrum of Y2 (HMQC)-level-1.

**Figure 29** shows the C13 NMR spectra of compound Y2.

**Figure 30** shows the 2D NMR (HMBC)-level-1 spectra of compound Y2.

**Figure 31** shows the 2D NMR HOHAHA (TOCSY)-level-1 spectrum of compound Y2.

**Figure 32**-shows the mass spectrum of compound Y2 +Matrix + Standards.

**Figure 33** shows the chemical structure of Y8.

**Figure 34** shows H-NMR spectrum of Y8.

**Figure 35** shows C13-NMR spectrum of Y8.

**Figure 36** shows 2D NMR HMQC (level 1) spectrum of Y8.

**Figure 37** shows the chemical structure of Y9.

**Figure 38** shows H-NMR spectrum of Y9.

**Figure 39** shows 2D NMR HMQC (level 1) spectrum of Y9.

**Figure 40** shows 2D NMR HMBC (level1) spectrum of Y9.

**Figure 41** shows the chemical structure of Y10.

**Figure 42** shows H-NMR spectrum of Y10.

**Figure 43** -shows C13 NMR spectrum of Y10.

**Figure 44** shows 2D NMR HMQC (level 1) spectrum of Y10.

**Figure 45** shows the absorption spectrum of Xanthoceras sorbifolia extract of the invention. Abscissa: Wavelength in nm. Ordinate: Optical Density. The extract has three absorption maximum at 207nm, 278nm and 500nm.

**Figure 46** shows the effect of compound X on the growth of OCAR-3 cells.

**Figure 48** shows a comparision of MTT and Haemolytic activities of saponin compound and Compound Ys of the invention. (A) and (B) shows hemolytic activities. (C) and (D) show MTT activities.

**Figure 49** (A) shows a compound of the invention without angeloyl groups. (B) shows a compound of the invention without sugar moiety.

**Figure 50** shows the saponin compounds of the invention.

## DETAILED DESCRIPTION OF THE INVENTION

This invention provides a compound selected from a compound of formula (1):

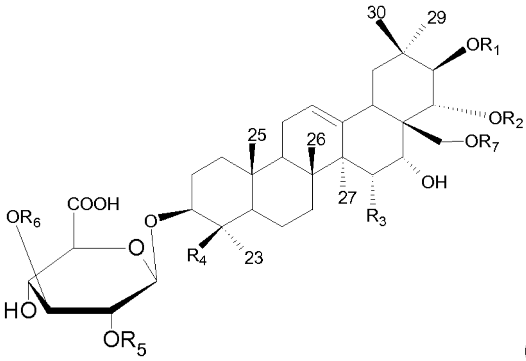

(1)

or a salt, ester or derivative thereof,wherein R1 represents angeloyl group; R2 represents angeloyl group; R3 represents OH or H; R4 represents CH3 or CH2OH; R7 represents H; and R5 represents D-glucose or D-Galactose; and R6 represents L-arabinose.

This invention provides a compound selected from a compound of formula (3A):

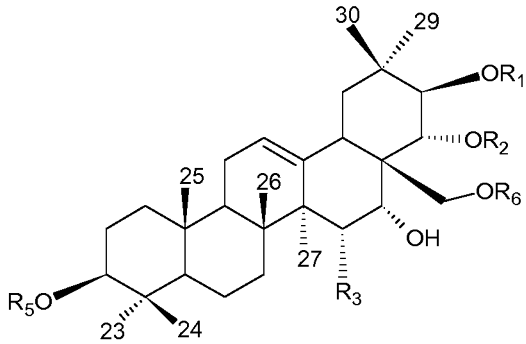

(3A)

or a salt, ester or derivative thereof, wherein R1 represents angeloyl group; R2 represents angeloyl group; R3 represents OH or H; Positions 23, 24, 25, 26, 27, 29, 30 of the compound independently comprise CH3, or CH2OH, or CHO, or COOH, alkyls group, or acetyl group, or derivative; R6 represents Ac or H; and R5 represents sugar moiety, wherein the sugar moiety comprises at least one sugar, or D-glucose, or D-galactose, or L-rhamnose, or L-arabinose, or D-xylose, or alduronic acid, or D-glucuronic acid, or D-galacturonic acid, or their derivative thereof, or the combination thereof. In an embodiment, R5 represents a compound capable of performing the function of the sugar moiety. In another embodiment the sugar moiety comprises L-arabinose, D-glucose and/or D-galactose, or combinations thereof. In a further embodiment, any two of R1, R2 or R6 are angeloyl groups, or any one of R1, R2 or R6 is attached to a sugar moiety in which two angeloyl groups are attached to adjacent carbons of the monosaccharides. In a further embodiment, R1, R2, and R6 comprises angeloyl group, acetyl group, tigloyl group, senecioly group, or an acid with two to five carbons or combibation thereof. In a further embodiment, at least one of R1, R2 or R6 is attached a sugar moiety or rhamnose, wherein sugar moiety or rhamnose comprises two angeloyl group, acetyl group, tigloyl group, senecioly group, acid having two to five carbons, or combinations thereof.

This invention provides a compound selected from a compound of formula (5):

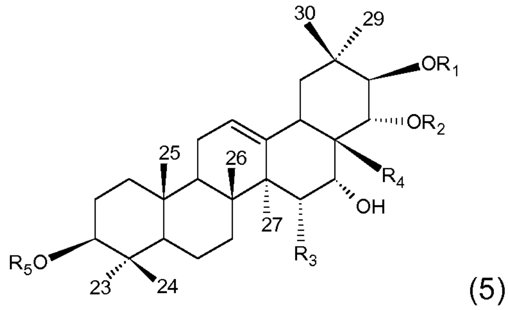

(5)

Or a salt, ester, metabolite or derivative thereof, wherein R1 and R2 represent angeloyl group; R3 represents H or OH; R4 represent CH2OR6; and wherein R6 is H; R5 represents at least one sugar moiety or its derivatives. In an embodiment, R1 and R2 represent angeloyl group; R3 represents H or OH; R4 represents COOR6 wherein R6 is H; R5 represents at least one sugar moiety or its derivatives. In an embodiment, R1 represents H; R2 represents angeloyl group; R3 represents H or OH; R4 represents CH2OR6 or COOR6; wherein R6 is an angeloyl group; and R5 represents at least one sugar moiety or its derivatives. In another embodiment, at least two of R1, R2, and R6 comprise an angeloyl group or acid having five carbons; R3 represents H or OH; R4 represents CH2OR6 or COOR6; and wherein R6 is angeloyl group; R5 represents at least one sugar moiety or its derivatives. In a further embodiment, at least one angeloyl of R1 or R2 is replaced by acetyl group, tigloyl group, senecioly group, or an acid having two to five carbons; R3 represents H or OH; R4 represents CH2OR6 or COOR6; and wherein R6 is angeloyl group; R5 represents at least one sugar moiety or its derivatives. In a further embodiment, at least one of R1, R2, and R6 is a sugar moiety or rhamnose comprising at least two angeloyl groups, acetyl group, tigloyl group, senecioly group, or an acid having two to five carbons or combination thereof. In a further embodiment, positions 23, 24, 25, 26, 29, 30 of the compound independently comprise CH3, CH2OH, CHO, COOH, alkyls group, acetyl group or derivative thereof. In a further embodiment, R5 represents sugar moiety comprising glucose, galactose or arabinose. In a further embodiment, R5 represents sugar moiety, wherein the sugar moiety comprises at least one sugar, or D-glucose, D-galactose, or L-rhamnose, or L-arabinose, or D-xylose, or alduronic acid, or D-glucuronic acid or D-galacturonic acid, or derivative thereof, or the combination thereof. In an embodiment, R5 represents a compound capable of performing the function of the sugar moiety. In a further embodiment, the R5 represents H. In a further embodiment, R4 represents H or OH or CH3.

Substitution, deletion and/or addition of any group in the above-described compounds will be apparent to one of ordinary skill in the art based on the teaching of this application. In a further embodiment, the substitution, deletion and/or addition of the group(s) in the compound of the invention does not substantially affect the biological function of the compound. In a further embodiment, the angeloyl groups are in a trans-position on a structure.

This invention provides a composition for inhibiting tumor cell growth, comprising the above-described compounds. In an embodiment, the composition comprises a suitable carrier. In another embodiment, the composition comprises a pharmaceutically suitable carrier. This invention provides a method for treating ovarian cancer in a subject, comprising administering to said subject an effective amount of the above-described compositions.

This invention provides a method for isolating compounds from Xanthoceras sorbifolia herb, or plants from the sapindaceae family comprising the steps of: (a) extracting Xanthoceras sorbifolia or plant powder with organic solvents to obtain an organic extract; (b) collecting the organic extract; (c) refluxing the organic extract to obtain a second extract; (d) removing the organic solvent from the second extract; (e) drying and sterilizing the second extract to obtain a crude extract powder; (f) fractionating the crude extract powder into components using HPLC and FPLC chromatography with silica gel, C18 and other equivalent solid phase materials; (g) monitoring absorption wavelength at 207nm or 254nm; (h) identifying the bioactive components of the crude extract powder; (i) purifying one or more bioactive components of the crude extract powder with FPLC to obtain one or more fraction of the bioactive component; and (j) isolating the desired fraction of the bioactive component with preparative HPLC.

**Compound Y or Y3**

This invention provides a compound comprising the following structure, i.e., see Figure 15, having the formula of $C_{57}H_{88}O_{23}$ and the name of 3-O-[β-D-galactopyranosyl(1→2)]-α-L-arabinofuranosyl(1→3)-β-D-glucuronopyranosyl-21,22-O-diangeloyl-3β, 15α, 16α, 21β, 22α, 28-hexahydroxyolean-12-ene, also known as Xanifolia-Y. This compound is obtainable from Xanthoceras sorbifolia or plants from the sapindaceae family.

$Y_3$

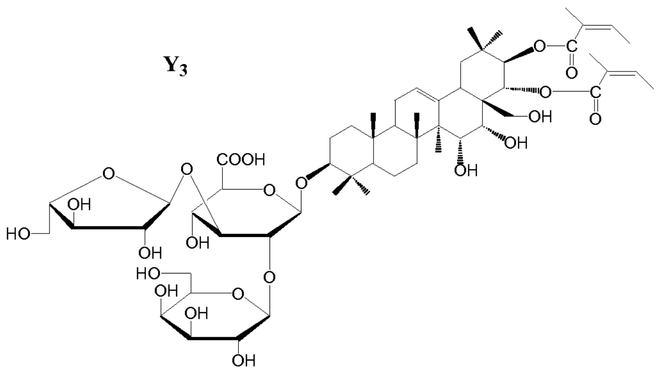

This compound belongs to an oleanene triterpenoidal saponin with a trisaccharide chain attached at C-3 of the aglycone and two angeloyl groups acylated at C-21 and C-22. This compound has anti-cancer activity. The assignment of this structure is supported by spectral data, i.e., H-NMR, 2D NMR (HMBC, HMQC), and MS (MALDI-TOF, EMS). Accordingly, this compound has the characteristic property as shown in Figures 16-20 or Table 5.1.

**Compound Y1**

This invention provides another compound comprising the following structure, i.e., see Figure 21, having the formula of $C_{65}H_{100}O_{27}$ and the name of 3-O-[β-D-galactopyranosyl(1→2)]-α-L-arabinofuranosyl(1→3)-β-D-glucuronopyranosyl-21-O-(3,4-diangeloyl)-α-L-rhamnophyranosyl-22-O-acetyl-3β,16α, 21β, 22α, 28-pentahydroxyolean-12-ene, also known as Xanifolia-Y1.

$Y_1$

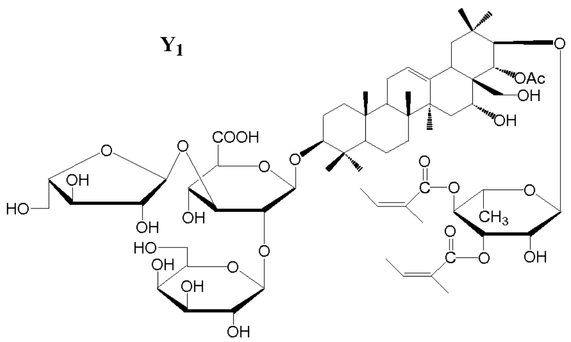

This compound is a bisdesmosidic polyhydroxyoleanene triterpenoidal saponin with a trisaccharide chain at C-3 of the backbone and a monosaccharide moiety at C-21 where two angeloyl groups were acylated at C-3 and C-4 position. This compound has anti-cancer activity. The assignment of this structure is supported by spectral data, i.e., H-NMR, 2D NMR (HMBC, HMQC, COSY), and MS (MALDI-TOF). Accordingly, this compound has the characteristic property as shown in Figures 22-25.

**Compound Y2**

This invention provides a third compound comprising the following structure, i.e., see Figure 26, having the formula of $C_{57}H_{88}O_{24}$ and chemical name of 3-O-[β-D-glucopyranosyl-(1→2)]-α-L-arabinofuranosyl(1→3)-β-D-glucuronopyranosyl-21,22-O-biangeloyl-3β, 15α, 16α, 21β, 22α, 24β, 28-heptahydroxyolean-12-ene, also known as Xanifolia-Y2.

Y2

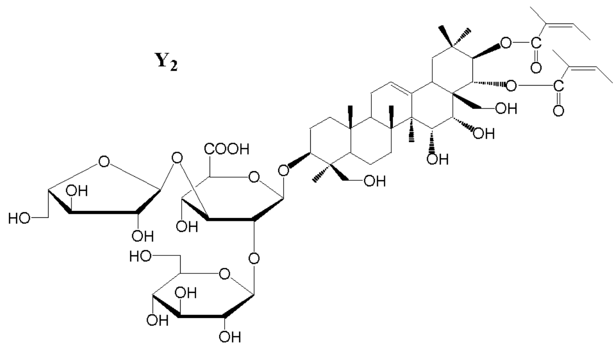

This compound (Y2) belongs to saponins comprising a triterpene, a sugar moiety and angeloyl groups linked to the backbone. The angeloyl groups are linked to the backbone at C21 and C22 positions. This compound has anti-cancer activity. The assignment of this structure is supported by spectral data, i.e., H-NMR, C-NMR, 2D NMR (HMBC, HMQC, TOCSY), and MS (MALDI-TOF). Accordingly, this compound has the characteristic property as shown in Figures 27-30.

**Compound Y8**

This invention provides a fourth active compound Y8 and the structure was determined by 1D NMR, 2D NMR, and MS analysis. The compound comprises the following structure, i.e. see Figure 33, having the formula of $C_{57}H_{87}O_{23}$ and chemical name of 3-*O*-[*β*-glucopyranosyl (1→2)]-α-arabinofuranosyl (1→3)-*β*-glucuronopyranosyl-21, 22-*O*-diangeloyl-3*β*, 16*α*, 21*α*, 22*α*, 24*β*, 28-hexahydroxyolean-12-ene, also known as Xanifolia-Y8.

Y-8

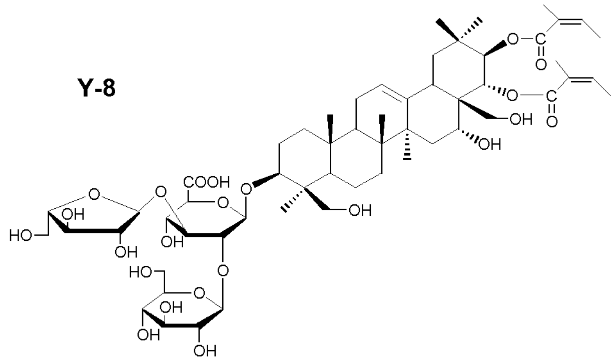

The assignment of this structure is supported by spectral data, i.e., H-NMR, C13-NMR and 2D NMR (HMQC). Accordingly, this compound has the characteristic property as shown in figures 34-36.

**Compound Y9**

This invention provides a fifth active compound Y9 and the structure was determined by 1 D NMR, 2D NMR, and MS analysis. The compound comprises the following structure, i.e., see Figure 37, having chemical name 3-O-[*β*-galactopyranosyl (1→2)]-*α*-arabinofuranosyl (1→3)-*β*-glucuronopyranosyl-21-*O*-(3,4-diangeloyl)-*α*-rhamnopyranosyl-28-*O*-acety1-3*β*, 16*α*, 21*β*, 22*α*, 28-pentahydroxyolean-12-ene, also known as Xanifolia-Y9.

$Y_9$

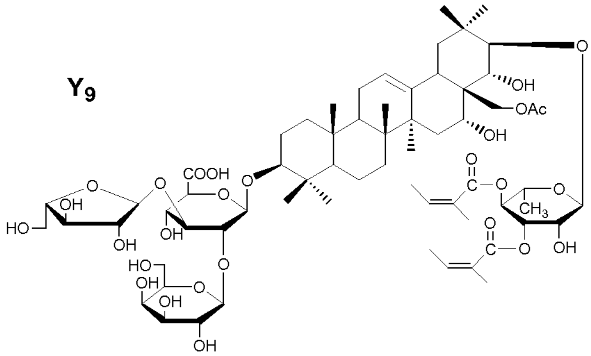

The assignment of this structure is supported by spectral data, i.e., H-NMR, 2D NMR (HMQC and HMBC). Accordingly, this compound has the characteristic property as shown in Figures 36-40.

**Compound Y10**

This invention provides a sixth active compound Y10 and the structure was determined by 1D NMR, 2D NMR and MS analysis. The compound comprises the following structure, i.e., see Figure 41, having the formula of $C_{57}H_{57}O_{22}$ and chemical name of 3-O-[$\beta$-galactopyranosyl (1→2)]-$\alpha$-arabinofuranosyl (1→3)-$\beta$-glucuronopyranosyl-21, 22-*O*-di-angeloyl-3$\beta$, 16$\alpha$, 21$\beta$, 22$\alpha$, 28-pentahydroxyolean-12-ene, also known as Xanifolia-Y10.

Y-10

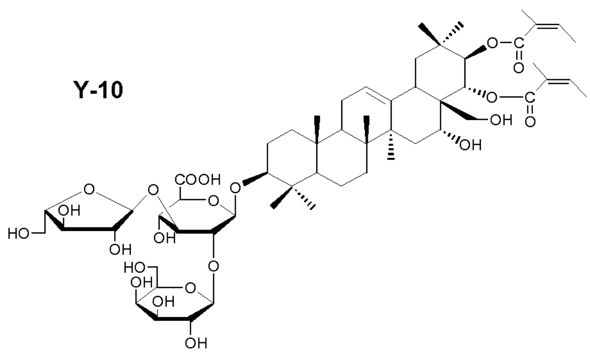

The assignment of this structure is supported by spectral data, i.e., H-NMR, C13-NMR and 2D NMR (HMQC). Accordingly, this compound has the characteristic property as shown in figures 42-44.

This invention provides a compound comprising a sugar moiety and a triterpene or Sapogenin, wherein the triterpene or sapogenin is acylated at Carbon 21 and 22 with angeloyl groups. In an embodiment, the triterpene or sapogenin is acylated at Carbon 21 and/or 22 with a sugar moiety which comprises two angeloyl groups. In another embodiment, the compound comprises one or more sugars. In another embodiment, the compound comprises at least two angeloyl groups. In a further embodiment, the sugar moiety comprises at least one sugar, or D-glucose, or D-galactose, or L-rhamnose, or L-arabinose, or D-xylose, or alduronic acid, or D-glucuronic acid or D-galacturonic acid, derivative thereof, or the combination thereof. In a further embodiment, a compound capable of performing the function of the sugar moiety is attached to the triterpene or sapogenin. In a further embodiment, the angeloyl group may be replaced by tigloyl group, senecioly group, an acid having two to five carbons, or combinations thereof. Substitution, deletion and/or addition of any group in the above-described compounds will be apparent to one of ordinary skill in the art based on the teaching of this application. In a further embodiment, the substitution, deletion and/or addition of the group(s) in the compound of the invention does not substantially affect the biological function of the compound. In a further embodiment, the angeloyl groups are in a trans-position on a structure. In a further embodiment, the angeloyl groups are in an adjacent trans-position on a structure.

Extracts obtained from Xanthoceras sorbifolia having anticancer activity is disclosed. Experiments for determining anti-cancer activity of the extract of the invention employ human cells lines derived from eleven human organs, i.e., (HTB-9 (bladder), HeLa-S3 (cervix), DU145 (prostate), H460 (lung), MCF-7 (breast), K562 (leukocytes), HCT116 (colon), HepG2 (liver), U2OS (bone), T98G (brain) and OVCAR-3 (ovary)). The response of the 11 cell lines toward the extract of the invention can be categorized into four groups: (A) most sensitive: Ovary, see Figure 12; (B) Sensitive: bladder, bone, (C) Semi-sensitive: prostate, leukocyte, liver, breast, and brain; and (D) lease sensitive: colon, cervix,

and lung. See Figure 14A, B, C. The respective IC50 values are listed in Table 3.1.

**Table 3.1. IC50 values of Xanthoceras Sorbifolia Extract Determined in Different Cancer Cells**

| Cancer cells from different organs | IC50 determined by MTT assay (ug/ml) |
|---|---|
| Ovary (most sensitive) | 15-15 |
| Bladder (sensitive) | 45-50 |
| Bone | 40-55 |
| Prostate (semi-sensitive) | 40-50 |
| Leukocyte | 45-50 |
| Liver | 45-65 |
| Breast | 65 |
| Brain | 70-85 |
| Colon (least sensitive) | 90 |
| Cervix | 115 |
| Lung | 110 |

In order to identify the active compounds of Xanthoceras sorbifolia, the extracts from Xanthoceras sorbifolia were separated by chromatography comprising FPLC (Fast Protein Liquid Chromatography) and HPLC (High Preferment Liquid Chromatography). Multiple fractions were obtained by FPLC procedures, i.e., see Figure 7, and HPLC, i.e., see Figure 6. Analysis of the fractions by HPLC shows that the extract comprises 26 identifiable fractions, designated as a to z, which are shown in Figure 6. Anti-cancer activities of these fractions were determined by the MTT assay.

FPLC fraction 5962, i.e., see Figure 8, which coresponds to fraction Y in HPLC, i.e., see Figure 6, has anti-cancer activity. Compound Y can be purified from fraction Y. Fraction 5962 was further separated into 4 components, designated as Y1 to Y4. See Figure 9. Compounds Y1-Y4 can be purified from the fraction 5962. Fraction 6365 was further seperated into 5-6 components, designated as Y5-Y10. See Figure 10. Compounds Y5-Y10 can be purified from Fraction 6365. Compounds Y or Y3, Y1 and Y2 show strong anti-tumor activity, i.e., see Figure 2-3, and were therefore isolated and purified. Similarly, compounds Y8, Y9 and Y10 also show strong anti-tumor activity, i.e., see Figure 4, and were therefore isolated and purified. See Figure 11. Accordingly, the structures of these active compounds, i.e., Y, Y1, Y2, Y8, Y9 and Y10 and their uses are the subject of this application.

The inhibition effects of the compounds of the present invention on ovarian cancer cells were evaluated with the MTT assay. Compound Y shows at least 10 times higher potency (IC50 = 1.5 ug/ml), i.e., see Figure 2, than the original crude extract as shown in Figure 12 (IC50 = 20 ug/ml). The selectivity of compound Y toward different cell lines was tested, and it was found that compound Y has a much higher potency toward ovarian cancer cells as compared to the cervical cancer cells. See Figure 13.

This invention provides a method for identifying and isolating the bioactive compounds from plants, herbs or plant extracts. In an embodiement, the extracts include extracts of Xanthoceras sorbifolia or of plants from the sapindaceae family. This invention provides the chemical structure of six bioactive compounds isolated from Xanthoceras sorbifolia or plants from the sapindaceae family. The structure of the compounds of the invention is shown in Figure 1. This invention provides spectral data including H-NMR, C-13-NMR, 2D NMR (HMBC, HMQC, COSY, TOCSY), and MS (MALDI-TOF, ESI-MS) in supporting the assigned structures.

This invention provides a consensus sub-structure or functional group derived from the bioactive compounds purified from fraction Y. The compounds, such as Y or Y3, Y1, Y2, Y8, Y9 and Y10, isolated from fraction Y are collectively referred to as "Ys" and their common name is Xanifolia-Ys. The consensus sub-structure or functional group of these compounds is the biangeloyl group located on adjacent carbons. For example, in compound Y, Y2, Y8 and Y10, the biangeloyl group is located at 21$\beta$ and 22$\alpha$ of the triterpene backbone. See Figure 5. In compound Y1 and Y9, the biangeloyl group is located at C3 and C4 of the sugar ring. Accordingly, the biangeloyl group of the bioactive compounds of the invention is situated in a trans-position with respect to each other on a structure. It has been shown that the bioactive functional group of the compounds of the invention is a biangeloyl group attached in-trans to adjacent carbons located in a structure. This invention provides a salt of the above-described compounds.

This invention provides a composition comprising the above-described compounds and a suitable carrier. This invention provides a pharmaceutical composition comprising an effective amount of the above-described compounds and a pharmaceutically acceptable carrier. This invention provides an anti-ovarian cancer agent or composition comprising the above-described compositions. This invention provides a composition effective against cancer growth. The cancer includes but is not limited to bladder cancer, bone cancer and ovary cancer. This invention provides a composition effective against skin cancer and KB cancer growth. This invention provides a composition comprising the above-described compounds and their salts, esters, derivatives or metabolites capable of inhibiting tumour growth. This invention provides a composition for inhibiting virus growth and/or activities comprising the above-described compounds and their salts, esters, derivatives or metabolites.

This invention provides a composition comprising the above-described compounds and their salts, esters, derivatives or metabolites capable of hemolytic activities. See Figure 48. It has been shown that a compound of the invention having two angeloyl groups has a stronger anticancer activity than a compound with one angeloyl group.See Figure 65, 2, 3. A compound with two angeloyl groups shows more haemolytic activity than a compound with one angeloyl group. See Figure 46A. The compound loses hemolytic activity when the angeloyl groups are removed See Figure 46B. The compounds of the invention or their derivatives are also obtainable by chemical systhesis or isolated from natural or biological sources, including plants from the sapindaceae family. It has also been shown that saponin compounds of the invention having two angeloyl groups are more potent than Escin for treating diseases relating to hamoylic activities. See Figure 46A.

This invention provides a composition for treating chronic venous insufficiency, peripheral edema, antilipemic, chronic venous disease, varicose vein disease, varicose syndrome,venous stasis, Expectorant, cerebro-organic convulsion, cerebral circulation disorder, cerebral edema, psychoses, dysmenorrheal, hemorrhoids, episiotomies, haemonhoids, peripheral oedema formation or postoperative swelling; for reducing symptoms of leg pain, pruritis, lower leg volume, thrombosis, thromophlebitis; and for preventing gastric ulcers antispasmotic. This invention provides a composition for AntiMS, antianeurysm, antiasthmatic, antibradykinic, anticapillarihemorrhagic, anticephalagic, anticervicobrachialgic, antieclamptic, antiedemic, antiencaphalitic, antiepiglottitic, antiexudative, antiflu, antifracture, antigingivitic, antihematomic, antiherpetic, antihistaminic, antihydrathritic, antimeningitic, antioxidant, antiperiodontic, antiphlebitic, antipleuritic, antiraucedo, antirhinitic, antitonsilitic, antiulcer, antivaricose, antivertiginous, cancerostatic, corticosterogenic, diuretic, fungicide, hemolytic, hyaluronidase inhibitor, lymphagogue, natriuretic, pesticide, pituitary stimulant, thymolytic, vasoprotective, venotonic.

**Compound-X**

This invention provides an active compound, designated as "compound X", isolated from extract of Xanthoceras Sorbifolia. Compound X is an oleanene triterpenoidal saponin with a trisaccharide chain attached at C-3 of the aglycone and one angeloyl group acylated at C-22. The formula of compound X is $C_{58}H_{92}O_{22}$, and the chemical name of compound X is: 3-O-{[$\beta$-D-galactopyranosyl (1→2)]-[-$\alpha$-arabinofuranosyl (1→3)]-$\beta$-D-glucuronopyranoside butyl ester}-21-O-acetyl-22-O-angeloyl-3$\beta$,16$\alpha$,21$\beta$,22$\alpha$,28-pentahydroxyolean-12-ene. The chemical structure of compound X is shown below.

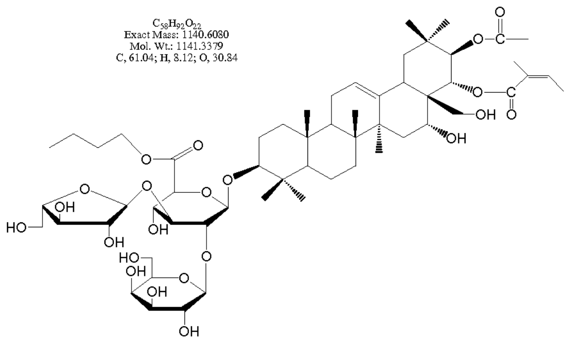

This invention provides a composition comprising the compounds of the invention for treating enuresis and frequency micturition, and for improving the functions of the central nervous system including signaling the bladder to wake up from deep sleep or to relaxe the bladder so that it can store more urine. The compounds of the invention can be used to relax the detrusor tension caused by aging, stress, nervousness, overactivity, instability, hyper-reflexia, and uninhibited bladder. In another embodiment, the compounds may be used for relaxing the contracted bladder tissue induced by acetylcholine (Ach). The compounds identified and isolated from extract of Xanthoceras sorbifolia may be used as acetylcolinesterase, an AChE inhibitor, for regulating Antidiuretic hormone (ADH), which reduces the volume of urine, and as an antiinflammatory agent.

The compounds of the invention can be used for accelerating the growth of bladder, for suppressing deep sleep, for increasing alterness in a sleeping subject, for modulating the release, breakdown and uptake of Antidieuretic hormone (ADH) and its receptors, for modulating the secretion, breakdown and uptake of Adrenocorticotropic hormone

(ACTH) and its receptors, for modulating the release, breakdown and uptake of 5-hydroxytryptamine and its receptors, for modulating the release, breakdown and uptake of Acetycholine (Ach) and its receptors, for modulating the release, breakdown and uptake of Adrenaline (AD) and its receptors, for modulating the release, breakdown and uptake of Dopamine (DA) and its receptors, for modulating the release, breakdown and uptake of Norepinephrine (NE) and its receptors, for preventing sleep paralysis, for modulating the formation, release, breakdown and activity of neuropeptides and their receptors.

This invention provides a composition comprising the compounds of the invention for treating cancers; for inhibiting virus; for preventing cerebral aging; for improving memory; improving cerebral functions, for curing enuresis, frequent micturition, urinary incontinence,dementia, Alzheimer's disease, autism, brain trauma, Parkinson's disease or other diseases caused by cerebral dysfunctions; for treating arthritis, rheumatism, poor circulation, arteriosclerosis, Raynaud's syndrome, angina pectoris, cardiac disorder, coronary heart disease, headache, dizziness, kidney disorder; cerebrovascular diseasea; inhibiting NF-Kappa B activation; for treating brain edema, sever acute respiratory syndrome, respiratory viral diseases, chronic venous insufficiency, hypertension, chronic venous disease, anti-oedematous, anti inflammatory, haemonhoids, peripheral oedema formation, varicose vein disease, flu, post traumatic edema and postoperative swelling; for inhibiting ethanol absorption; for lowering blood sugar; for regulating the adreocorticotropin and corticosterone level; and for treating impotence or premature ejaculation or diabetes. See U.S. Serial No. 10/906,303, filed February 14, 2005, International Application No. PCT/US04/43465, filed December 23, 2004, International Application No. PCT/US04/33359, filed October 8, 2004, and U.S. Serial No. 11/131551, filed May 17, 2005.

This invention provides a composition capable of regulating the enzymatic activities of bladder cell. The Xanthoceras Sorbifolia derived compounds and/or compositions of the invention are capable of regulating the various components or receptors of a cell and strengthening the cell growth process. The Xanthoceras Sorbifolia derived compound and/or composition regulates the activities of cell's enzymes. See U.S. Serial No.60/675,284, filed April 27, 2005. This invention provides compounds isolated from Xanthoceras Sorbifolia, or its derivatives or metabolites capable of regulating pathways involved in cell proliferation.See U.S. Serial No. 10/906,303, filed February 14, 2005, International Application No. PCT/US04/43465, filed December 23, 2004, International Application No. PCT/US04/33359, filed October 8, 2004.

This invention provides the methods and uses of triterpenoidal saponins purified and isolated from plants. This invention provides compositions comprising the triterpenoidal saponins or their derivatives for inhibition of tumor growth. The compounds of the invention comprise angeloyl group(s) or tigloyl group(s) or senecioyl group(s) or combinations thereof which are attached to carbon 21 and 22 of their sapongenines. In an embodiment, the compounds may comprise any two angeloyl groups or tigeloyl groups or senecioyl groups or combinations thereof attached to a sugar moiety which bonds to carbon 21 or 22 of their sapongenines. The bioactive compounds can be isolated from natural plants, including plants in the Sapindaceae family, which has between about 1400-2000 species with 140-150 genera. See U.S. Serial No.60/675,282, filed filed April 27, 2005.

## Summary

This invention provides methods for identifying and purifying bioactive compounds from the plant extract of Xanthoceras sorbifolia. Eight bioactive compounds have so far been identified and purified, and six of them have been shown to have anticancer activity. These compounds are collectively referred to as triterpenoidal saponins. A consensus sub-structure was identified or derived from the structure of the bioactive compounds of the invention. The consensus sub-structure or active functional groups of the bioactive compounds is the biangeloyl group located on adjacent carbons. The angeloyl groups are located at 21$\beta$ and 22$\alpha$ of the triterpene backbone, i.e., see Figure 5, or located at C3 and C4 of the sugar ring. Accordingly, the biangeloyl group of these bioactive compounds is acylated in a trans-position in adjacent carbons of a structure. The compound with single angeloyl group shows weaker anticancer activity than a compound with two angeloyl groups, i.e., sees Figure 46, 2, 3. The compound with single angeloyl group shows less haemolytic activity than a compound with two angeloyl groups, i.e., see Figure 48. The structures or derivatives of the compounds of the present invention are also obtainable by chemical systhesis or from biological sources.

This invention will be better understood from examples which follow. However, one skilled in the art will readily appreciate that the specific methods and results discussed are merely illustrative of the invention as described more fully in the claims which follow thereafter.

## EXPERIMENTAL DETAILS

### Experiment 1: Herb Extraction

(a) extracting Xanthoceras sorbifolia powder of husks or branches or stems or leaves or kernels or roots or barks with organic solvent at ratio of 1:2 for 4-5 times for 20-35 hours each time to form an organic extract; (b) collecting the organic extract; (c) refluxing the organic extract for 2-3 times at 80°C to form second extract; (d) removing the organic

solvent from the second extract; and (e) drying and sterilizing the second extract to form a Xanthoceras sorbifolia extract powder.

**Experiment 2: Analysis of Xanthoceras Sorbifolia Extract Components by HPLC Chromatography**

**Methods**

HPLC. A C-18 reverse phase μbondapak column (Water P/N 27324) was equilibrated with 10% acetonitrile, 0.005% Trifluoroacetic acid (equilibration solution). An extract of Xanthoceras sorbifolia prepared using the methods described in Experiment 1 was dissolved in equilibration solution (1 mg/ml) before applying into the column. 20ug of samples was applied into column. Elution conditions: Fractions were eluted (with flow rate 0.5 ml/min.) with acetonitrile gradient from 10% to 80% in 70 min, and then remains at 80% for 10 min. The acetonitrile concentration then decreased to 10% and remained at 10% for 25 min. The fractions were monitored at 207 nm and recorded in chart with a chart speed of 0.25 cm/min and with OD full scale of 0.128.

Instruments. Waters Model 510 Solvent Delivery System; Waters 484 tunable Absorbance Detector; Waters 745/745B Data Module.

Absorbance analysis. The absorption profile of Xanthoceras Sorbifolia extract at various wavelengths was determined. An extract of Xanthoceras sorbifolia of the present invention was dissolved in 10% acetonitrile/TFA and scanned at 200-700 nm with a spectrophotometer [Spectronic Ins. Model Gene Sys2].

**Results**

HPLC. About 60-70 peaks can be accounted for in the profile. Among them four are major peaks, 10 are of medium size and the rest are small fractions. The peaks are labelled with a to z following increased concentration of acetonitrile elution. See Figure 6.

Absorption maximum. Three absorption maximum were identified for Xanthoceras sorbifolia plant extract; 207nm, 278nm and 500nm. See Figure 45.

**Experiment 3: Determination of the cell-growth activity effected by Xanthoceras Sorbifolia Extract with Cancer Cells Derived from Different Human Organs using MTT Assay**

**Methods and Materials**

Cells. Human cancer cell lines were obtained from American Type Culture Collection: HTB-9 (bladder), HeLa-S3 (cervix), DU145 (prostate), H460 (lung), MCF-7 (breast), K562 (leukocytes), HCT116 (colon), HepG2 (liver), U2OS (bone), T98G (brain) and OVCAR-3 (ovary). Cells were grown in culture medium (HeLa-S3, DU145, MCF-7, Hep-G2 and T98G in MEN (Earle's salts); HTB-9, H460, K562, OVCAR-3 in RPMI-1640; HCT-116, U2OS in McCoy-5A) supplemented with 10% fetal calf serum, glutamine and antibiotics in a 5% CO2 humidified incubator at 37°C.

MTT assay. The procedure for MTT assay followed the method described in (Carmichael et al., 1987) with only minor modifications. Cells were seeded into a 96-wells plate at concentrations of 10,000/well (HTB-9, HeLa, H460, HCT116, T98G, OVCAR-3), 15,000/well (DU145, MCF-7, HepG2, U2OS), or 40,000/well (K562), for 24 hours before drug-treatment. Cells were then exposed to drugs for 48 hours (72 hours for HepG2, U2OS, and 96 hours for MCF-7). After the drug-treatment, MTT (0.5 mg/ml) was added to cultures for an hour. The formation of formazan (product of the reduction of tetrazolium by viable cells) was dissolved with DMSO and the O.D. at 490nm was measured by an ELISA reader [Dynatech. Model MR700]. The MTT level of cells before drug-treatment was also measured (TO). The % cell-growth (%G) is calculated as:

$$\%G = (TD\text{-}T0 / TC\text{-}T0) \times 100 \qquad (1)$$

where TC or TD represent O.D. readings of control or drug-treated cells. When T0 > TD, then the cytotoxicity (LC) expressed as % of the control is calculated as:

$$\%LC = (TD\text{-}T0 / T0) \times 100. \qquad (2)$$

**Results**

Among the 11 cell lines studies, inhibition of cell-grwoth after exposure of plant extract was observed. However, their sensitivity toward Xanthoceras sorbifolia extract is different. The response of the cell lines to the Xanthoceras extract can be categorized into four groups: Most sensitive, i.e., Ovary; Sensitive, i.e., bladder, bone; Semi-sensitive, i.e., prostate, leukocyte, liver, breast, and brain; and least sensitive, i.e., colon, cervix, and lung. See Figure 12, 13 and 14A-D. Their IC50 values are listed in Table 3.1 above.

In addition to cell-growth inhibition, the Xanthoceras sorbifolia plant extract also stimulate a minor cell growth at low concentrations in bladder, bone and lung cells. Results indicate that there is a cell or tissue stimulation component(s) in the extract. See Figures 14A and 14D.

To investigate the inhibition components of the Xanthoceras sorbifolia plant extract, the plant extract was fractionated. Figure 8 shows the results of the screening of fractions obtained after FPLC chromatography for cell growth-inhibition activity. The assay was conducted with bladder cells. The fractions obtained from FPLC, as shown in Figure 7, were used. As shown in Figure 7, different components of Xanthoceras sorbifolia extract caused either growth or inhibition effects on cells. Only fractions 5962, designated as Fraction Y, cause cell growth inhibition. Abscissa: concentration (ug/ml). Ordinate: % Cell Growth (determined by MTT assay).

**Experiment 4: Purification of the Inhibition Components in the Xanthoceras Sorbifolia Extract.**

**(A) Fractionation of plant extracts with FPLC**

**Methods**

Column. Octadecyl functionalized silica gel. Column dimension: 2cm x 28cm; equilibrated with 10% acetonitrile - 0.005% TFA before use.
Sample loading: 1-2 ml, concentration: 100mg/ml in 10% acetonitrile/TFA.
Gradient elution condition: 10-80% acetonitrile in a total volume of 500 ml.
Monitor absorption wavelength: at 254nm.
Fraction Collector: 5 ml/fractions (collect from 10% to 72% acetonitrile)
Instrument: AKTA-FPLC, P920 pump; Monitor UPC-900; Frac-900.

**Results**

The elution profile of the chromatography shows 4-5 broad fractions. See Figure 7. These fractions were analyzed with HPLC. Specific components, corresponding to a-z as specified in Figure 6, are then assigned in these FPLC fractions. FPLC fractions are then grouped into 7 pools and analyzed for cell growth activity in bladder cells with MTT assay. *See* Experiment 3. It was found that only pool #5962, corresponding to fraction Y in HPLC, contains inhibition activity. *See* Figure 8. It was also found in later experiments that fractions beyond 62 also show inhibition activity. The components isolated from fractions 63-65 showed inhibition activities. *See* Figure 4, 10 and 13.

**(B) Isolation of Component Ys with Preparative HPLC**

**Methods**

Column: A preparative HPLC column (Waters Delta Pak C18-300A);
Elution conditions: 45% acetonitrile isocratic elution with flow rate of 1 ml/min.
Fractions are monitored at 207nm and were collected and lyophilized.

**Results**

Final separation of Y fractions was achieved by HPLC with a preparative column. See Figure 9 and 10. These fractions, which include compound Y1, Y2, Y or Y3 and Y4, were collected. Re-chromatography of compound Y showed a single peak in HPLC with a C18 reverse phase column. See Figure 9A and 9B. Re-chromatography of the compound Y8, Y9 and Y10 showed a single peak in HPLC with a C18 reverse phase column. See Figure 11.

**(C) Appearance and solubility**

The pure compound Ys is an amorphous white powder, soluble in aqueous alcohol, i.e., methanol or ethanol,

50% acetonitrile and 100% pyridine.

**(D) Inhibition analysis of Compound Ys with MTT assay**

Inhibition analysis of compound Y was determined with MTT assay. Figure 2 shows that compound Y has activity against ovarian cancer cells (OCAR-3) with IC50 value of 1.5 ug/ml which is 10-15 times more potent than the unpurified extract shown in Figure 12. Figure 13 shows the selectivity of compound Y to ovarian cancer cells compared with cervical cancer cells (HeLa). Figure 3 shows the inhibition activities of compound Y1 and Y2 on the growth of ovarian cancer cells (OCAR-3). Figure 4 shows the inhibition activities of compound Y, Y8, Y9 and Y10 on the growth of ovarian cancer cells (OCAR-3).

**Experiment 5: Determination of the Chemical Structure**

**Methods**

NMR analysis. The pure compound Y of Xanthoceras sorbifolia was dissolved in pyridine-D5 with 0.05% v/v TMS. All NMR spectra were acquired using a Bruker Avance 600 MHz NMR spectrometer with a QXI probe (1H/13C/15N/31P) at 298 K. The numbers of scans for 1 D 1 H spectra were 16 to 128, depending on the sample concentration. 2D HMQC spectra were recorded with spectral widths of 6000 x 24,000 Hz and data points of 2024 x 256 for t2 and t1 dimensions, respectively. The number of scans was 4 to 128. 2D HMBC were acquired with spectral widths of 6000 x 30,000 Hz and data points of 2024 x 512 for t2 and t1 dimensions, respectively. The number of scans was 64. The 2D data were zero-filled in t1 dimension to double the data points, multiplied by cosine-square-bell window functions in both t1 and t2 dimensions, and Fourier-transformed using software XWIN-NMR. The final real matrix sizes of these 2D spectra are 2048x256 and 2048x512 data points (F2xF1) for HMQC and HMBC, respectively.

Mass spectral analysis. The mass of samples was analyzed by (A) MALDI-TOF Mass Spectrometry and by (B) ESI-MS Mass spectrometry. (A) Samples for MALDI-TOF were first dissolved in acetonitrile, and then mixed with the matrix CHCA, i.e., Alpha-cyano-4-hydroxycinnamic acid, 10mg CHCA/mL in 50:50 water/acetonitrile and 0.1% TFA in final concentration. The molecular weight was determined by the high resolution mass spectroscope analysis with standards. (B) For ESI, the sample was analyzed with LCQ DECA XP Plus machine made by Thermo Finnigan. It is ionized with ESI source and the solvent for the compound is acetonitrile.

**Results**

The profile of the proton NMR is presented in Figure 18. The 2D NMR profiles of HMQC and HMBC are shown in Figures 19 and 20, respectively. Table 5.1 summarizes the 2D NMR chemical shift data and the assignment of functional groups derived from these data. Based on these data and analysis, the structure of compound Y (Y3) is assigned as shown below.

**Structure of Compound Y**

$Y_3$

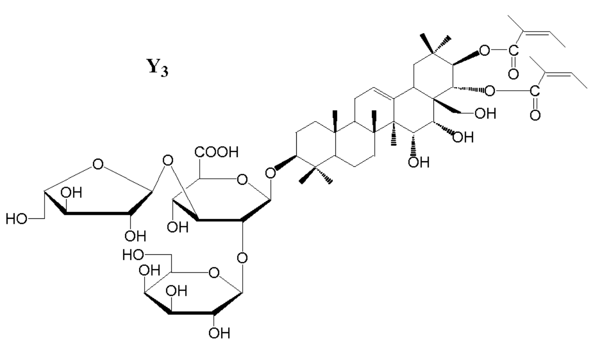

The chemical name of compound Y is: 3-O-[β-D-galactopyranosyl(1→2)]-α-L-arabinofuranosyl(1→3)-β-D-glucuronopyranosyl-21,22-O-diangeloyl-3β, 15α, 16α, 21β, 22α, 28-hexahydroxyolean-12-ene. The mass spectrum of compound Y as determined by MALDI-TOF and ESI-MS, i.e., see Figure 19, 20, indicates that the mass of compound Y is 1140.57 which agree with the theoretical mass of the compound Y.

**Conclusion**

The active compound Y isolated from extract of Xanthoceras sorbifolia is an oleanene triterpenoidal saponin with a trisaccharide chain attached at C-3 of the aglycone and two angeloyl groups acylated at C-21 and C-22. The formula of Y is $C_{57}H_{88}O_{23}$, and the chemical name of Compound Y is: 3-O-[β-D-galactopyranosyl(1→2)]-α-L-arabinofuranosyl(1→3)-β-D-glucuronopyranosyl-21,22-O-diangeloyl-3β, 15α, 16α, 21β, 22α, 28-hexahydroxyolean-12-ene.

**Experiment 6: Determination of the Chemical Structure of Compound Y1 of Xanthoceras Sorbifolia Extract**

**Methods**

The method for NMR and MS analysis for compound Y1 is similar to the method described in Experiment 5.

**Results**

The spectrum of the H-NMR is presented in Figure 22. The 2D NMR spectra of HMQC, HMBC and COSY are shown in Figures 23-25, respectively. Based on these data and analysis, the structure of compound Y1 is assigned and shown below.

**Structure of Y1**

$Y_1$

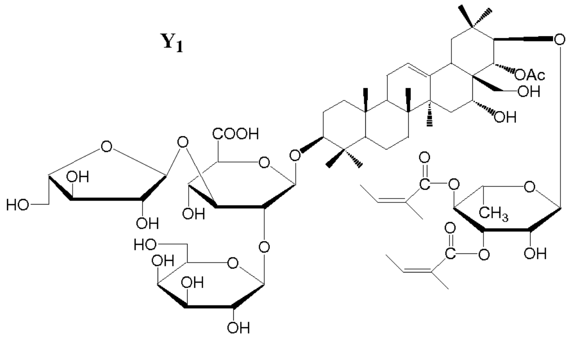

The chemical name of Y1 is: 3-O-[β-D-galactopyranosyl(1→2)]-α-L-arabinofuranosyl(1→3)-β-D-glucuronopyranosyl-21-O-(3,4-diangeloyl)-α-L-rhamnophyranosyl-22-O-acetyl-3β,16α, 21β, 22α, 28-pentahydroxyolean-12-ene.

**Conclusion**

Compound Y1 isolated from extract of Xanthoceras sorbifolia is a bisdesmosidic polyhydroxyoleanene triterpenoidal saponin with a trisaccharide chain at C-3 of the backbone and a monosaccharide moiety at C-21 where two angeloyl groups were acylated at C-3 and C-4 position. The formula of Y1 is $C_{65}H_{100}O_{27}$,

**Experiment 7: Determination of the Chemical Structure of Compound Y2 of Xanthoceras Sorbifolia Extract.**

**Methods**

The method for NMR and MS analysis for compound Y2 is similar to the method described in Experiment 5.

**Results**

The 1 D and 2 D NMR spectra of H-NMR, C-13 NMR, HMQC, HMBC and (TOCSY) and MS (MALDI-TOF) of Y2 are showed in Figures 27-32. Table 7.1 summarizes the 1 D and 2D NMR chemical shift data and the assignment of functional groups derived from these data.

## Conclusion

Based on these data and analysis, the compound Y2 isolated from extract of Xanthoceras sorbifolia is an oleanene triterpenoidal saponin with a trisaccharide chain attached at C-3 of the aglycone and two angeloyl groups acylated at C-21 and C-22. The chemical structure of Y2 is shown below. *See also* Figure 26.

Y2

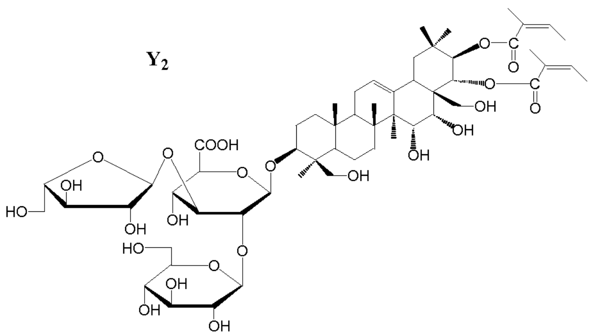

The formula of Y2 is $C_{57}H_{88}O_{24}$, and the chemical name of Compound Y2 is: 3-O-[β-D-glucopyranosyl-(1→2)]-α-L-arabinofuranosyl(1→3)-β-D-glucuronopyranosyl-21,22-O-biangeloyl-3β, 15α, 16α, 21β, 22α, 24β, 28-heptahydroxyolean-12-ene.

## Experiment 8. Purification of the Inhibition Components Y8-Y10 in the Xanthoceras Sorbifolia Extract

### (A) Fractionation of Xanthoceras Sorbifolia extracts components with FPLC

**Methods**

The methods for this experiment are similar to the methods decribed in Experiment 4 Section (A) and (B).

**Results**

The elution profile shows 4-5 broad fractions. See Figure 7. These fractions were analyzed with HPLC. FPLC fractions 63, 64 and 65 are further separated on 45% isocratic analysis, 4-5 major components were separated. See Figure 10. These fractions were assigned designations Y8, Y9 and Y10. These fractions were collected. Re-chromatography of the compound Y8, Y9 and Y10 showed a single peak in HPLC with a C18 reverse phase column. See Figure 11.

### (B) Inhibition analysis with MTT assay.

Inhibition analysis of purified compounds was determined with the MTT assay. Results indicate that compound Y8, Y9 and Y10 has activity against ovarian cancer cells (OCAR-3) with IC50 values of 3, 4 and 1.5 ug/ml, respectively. See Figure 4.

## Experiment 9. Determination of the Chemical Structure of Compound Y8 of Xanthoceras Sorbifolia Extract

**Methods**

The method for NMR and MS analysis for compound Y8 is similar to the method described in Experiment 5.

**Results**

The spectral profiles of the H-NMR, C13-NMR and 2D NMR (HMQC) of compound Y8 are presented in Figures 34-36. Table 9.1 summarizes the 1 D and 2D NMR chemical shift data and the assignment of functional groups derived from these data. Based on these data and analysis, the compound Y8 isolated from extract of Xanthoceras sorbifolia is an oleanene triterpenoidal saponin with a trisaccharide chain attached at C-3 of the aglycone and two angeloyl groups acylated at C-21 and C-22. The formula of compound Y8 is $C_{57}H_{88}O_{23}$, and the chemical name of Y8 is: 3-O-[*β*-glucopyranosyl (1→2)]-*α*-arabinofuranosyl (1→3)-*β*-glucuronopyranosyl-21, 22-*O*-diangeloyl-3*β*, 16*α*, 21*β*, 22*α*, 24*β*, 28-hexahydroxyolean-12-ene. The chemical structure of compound Y8 is presented in the following : *See also* Figure 33.

**Y-8**

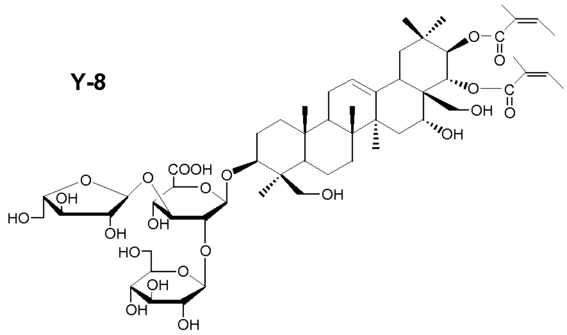

**Experiment 10. Determination of the Chemical Structure of Compound Y9 of Xanthoceras Sorbifolia Extract**

**Methods**

The method for NMR and MS analysis for compound Y9 is similar to the method described in Experiment 5.

**Results**

The spectral profiles of the H-NMR and 2D NMR, i.e., HMQC and HMBC, of Y9 are shown in Figures 38-40. Based on these data and analysis, compound Y9 isolated from extract of Xanthoceras sorbifolia is a bisdesmosidic polyhydroxyoleanene triterpenoidal saponin with a trisaccharide chain at C-3 of the backbone and a monosaccharide moiety at C-21 where two angeloyl groups were acylated at C-3 and C-4 position. The formula of compound Y9 is $C_{65}H_{100}O_{27}$ and the chemical name of Y9 is: 3-O-[*β*-galactopyranosyl (1→2)]-*α*-arabinofuranosyl (1→3)-*β*-glucuronopyranosyl-21-O-(3,4-diangeloyl)-*α*-rhamnopyranosyl-28-*O*-acetyl-3*β*, 16*α*, 21*β*, 22*α*, 28-pentahydroxyolean-12-ene. The chemical structure of Compound Y9 is presented in the following: *See also* Figure 37.

**$Y_9$**

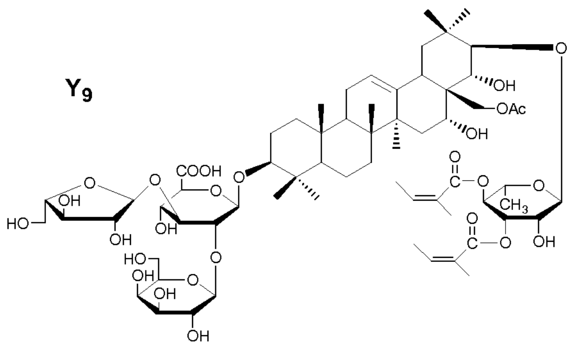

**Experiment 11. Determination of the Chemical Structure of Compound Y10 of Xanthoceras Sorbifolia Extract**

**Methods**

The method for NMR and MS analysis for compound Y10 is similar to the method described in Experiment 5.

**Results**

The profile of the H-NMR, C13-NMR and 2D NMR (HMQC) are shown in Figures 42-44 Table 11.1 summarizes the 1 D and 2D NMR chemical shift data and the assignment of functional groups derived from these data. Based on these data and analysis, compound Y10 isolated from extract of Xanthoceras sorbifolia is an oleanene triterpenoidal saponin with a trisaccharide chain attached at C-3 of the aglycone and two angeloyl groups acylated at C-21 and C-22. The formula of compound Y10 is $C_{57}H_{88}O_{22}$, and the chemical name of Y10 is: 3-O-[*β*-galactopyranosyl (1→2)]-*α*-arabinofuranosyl (1→3)-*β*-glucuronopyranosyl-21, 22-*O*-diangeloyl-3*β*, 16*α*, 21*β*, 22*α*, 28-pentahydroxyolean-12-ene. The chemical structure of Compound Y10 is presented in the following , *See also* Figure 41.

Y-10

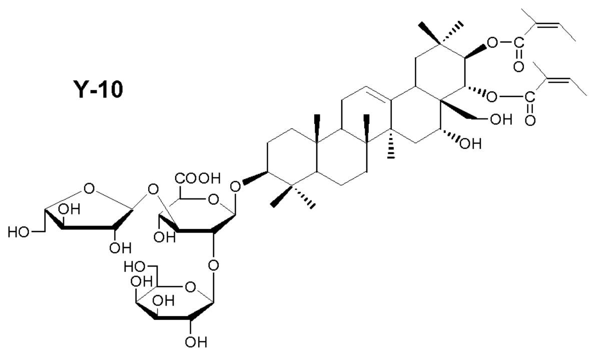

### Experiment 12. Determination of the Chemical Structure of Compound X of Xanthoceras Sorbifolia Extract

#### Methods

The method for NMR and MS analysis for compound X is similar to the method described in Experiment 5.

The active compound X isolated from extract of Xanthoceras Sorbifolia is an oleanene triterpenoidal saponin with a trisaccharide chain attached at C-3 of the aglycone and one angeloyl group acylated at C-22. The formula of X is $C_{59}H_{92}O_{22}$, and the chemical name of Compound X is: 3-O-{[$\beta$-D-galactopyranosyl (1$\rightarrow$2)]-[$\alpha$-L-arabinofuranosyl (1$\rightarrow$3)]-$\beta$-glucuronopyranoside butyl ester}-21-O-acetyl-22-O-angeloyl-3$\beta$,16$\alpha$,21$\beta$,22$\alpha$,28-pentahydroxyolean-12-ene. The chemical structure of compound X is presented in the following :

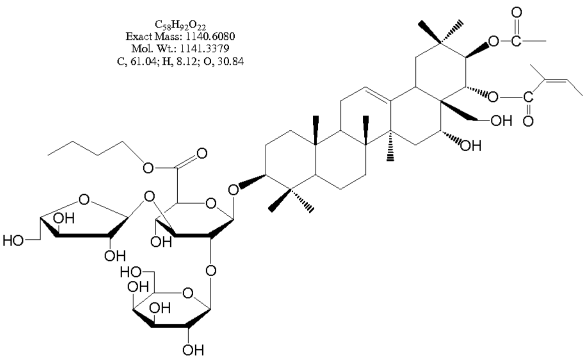


### Experiment 13. Determination the haemolytic activities of Compound Y of Xanthoceras Sorbifolia

#### Methods:

- Human whole blood was obtained from Houston Gulf Coast Blood Center
- Red blood cells were isolated by the following method: Human blood (in EDTA) was diluted 1:1 with PBS, underlay with 4 ml of Histopaque-1077 (SIGMA) and was centrifuged at 400g for 30 min.
- Red blood cells (RBC) were collected and washed three times with PBS.
- 10% suspension of RBC were prepared with PBS before use.
- 50 ul of RBC suspension was added to 2 ml of Saponins with different concentration.
- The suspension was mixed by vortexing and sit at room temperature for 60 minutes.
- The suspension was centrifuged at 3000 rpm for 5 min. Absorbance of the supernatant was measured at 540 nm.

#### Results:

In this experiment, hemolytic activity of human red blood cells by Xanifolia-Y (#63Y), Escin and SIGMA saponin standard were compared. Y contains two angeloyl groups, Escin has one angeloyl group and SIGMA saponin standard is a mixture of saponins from Quillaia bark. The results show that #63Y (compound Y) has higher hemolytic activity (IC50=1 ug/ml) than Escin or SIGMA saponin standard (IC50=5 ug/ml). See Figure 48A.

**Experiment 14. Determination the hemolytic and MTT activities of Compound Y after removal of the angeloly or the sugar moiety by alkaline or acid hydrolysis, respectively**

**Methods:**

(A) Alkaline Hydrolysis of Xanifolia-Y. 20 mg of Xanifolia-Y was dissolved in 0.5 ml of 1 M NaOH. The solution was incubated in 80C water bath for 4 hours. It was cooled to room temperature before neutralized with 0.5 ml 1 N HCl (adjust pH to about 3). The mixture was extracted with 2 ml 1-butanol 3 times. The butanol fractions were collected and lyophilized. The hydrolyzed saponin with further purified with HPLC in a C-18 column eluted with 25% acetonitrile.
(B) Acid Hydrolysis of Xanifolia-Y. 15 mg Xanifolia-Y was dissolved in 1 ml of Methanol. 1 ml of 2N HCl was then added. The mixture was refluxed in 80C water bath for 5 hours. The solution was then neutralized by adding 2 ml of 1 N NaOH (to final pH 3-4). The aglycone was then extracted with ethylacetate 3 ml x 3. The extracts were collected and pooled. Further isolation of aglycone (sugar-removed Xanifolia-Y) was achieved by HPLC with isocratic elution of 80% acetonitrile.

**Results:**

The angeloly groups or the sugar moiety of the compound Y, were removed by alkaline or acid hydrolysis, respectively. The hemolytic activities of the hydrolysed products were then analyzed. Resultsof these studies indicate that removing sugars reduce hemolytic activity, but removing angeloyl groups destroy the hemolytic activity. It also suggested that sugars are helpful but not essential for hemolytic activity. See Figure 48B. The experiment results show that compound-Y lost MTT activities if the angeloyl groups were removed. However, the MTT activities were very weak when the sugar moiety of the compound was removed. See Figure 48C, 48D. Results of comparison of hemoyltic activities between Compound Y, Escin and Saponin standard from SIGMA are shown in Figure 48A and 48B. Results of Comparison of hemolytic activities between Compound Y, compound Y without sugar moiety or angeloly groups are shown in Figure 48B. Chemical structures of compound Y without sugar moiety or angeloly groups are shown in Figure 49A and 49B respectively.

**TABLES OF EXPERIMENTAL RESULTS:**

**Table 5.1. 13C and 1 H NMR Data for Compound Y (in Pyridine-d5)[a]**

| Position | C | H | Key HMBC correlations |
|---|---|---|---|
| 1 | 38.7 | 0.83, 1.40 | C-3, C-5, C-9 |
| 2 | 26.4 | 1.81,2.14 | - |
| 3 | 89.6 | 3.25, 1H, dd, 12.0/4.0 Hz | C-23, C-24, GlcA C-1' |
| 4 | 39.4 | - | - |
| 5 | 55.3 | 0.78 | - |
| 6 | 18.5 | 1.55, 1.59 | C-8, C-10 |
| 7 | 36.5 | 2.00, 2.10 | C-5, C-9 |
| 8 | 41.2 | - | - |
| 9 | 47.0 | 3.06 | C-7, C-8, C-12, C-14, C-26 |
| 10 | 37.2 | - | - |
| 11 | 23.7 | 1.74, 1.89 | - |
| 12 | 125.2 | 5.49, 1H, br s | C-9, C-11, C-14, C-18 |
| 13 | 143.4 | - | - |
| 14 | 47.5 | - | - |

(continued)

| Position | C | H | Key HMBC correlations |
|---|---|---|---|
| 15 | 67.3 | 4.21 | C-8, C-27 |
| 16 | 73.6 | 4.45 | C-14, C-15, C-18 |
| 17 | 48.3 | - | - |
| 18 | 40.8 | 3.07 | C-12, C-13, C-14, C-16, C-19, C-20, C-28, |
| 19 | 46.8 | 1.41, 1.69 | - |
| 20 | 36.2 | - | - |
| 21 | 79.3 | 6.71, 1H, d, 10 Hz | C-20, C-22, C-29, C-30, 21-O-Ang C-1"" |
| 22 | 73.5 | 6.32, 1H, d, 10 Hz | C-16, C-17, C-21, C-28, 22-O-Ang C-1"" |
| 23 | 27.7 | 1.26, 3H, s | C-3, C-4, C-5, C-24 |
| 24 | 16.5 | 1.16, 3H, s | C-3, C-4, C-5, C-23 |
| 25 | 16.0 | 0.81, 3H, s | C-1, C-5, C-9, C-10 |
| 26 | 17.3 | 0.99, 3H, s | C-7, C-8, C-9, C-14 |
| 27 | 21.0 | 1.85, 3H, s | C-8, C-13, C-14, C-15 |
| 28 | 62.9 | 3.50, 1H, d, 11.0 Hz, 3.76, 1H, d, 11.0 Hz, | C-16, C-17, C-18, C-22 |
| 29 | 29.2 | 1.09, 3H, s | C-19, C-20, C-21, C-30 |
| 30 | 20.0 | 1.32, 3H, s | C-19, C-20, C-21, C-29 |
| GlcA | | | |
| 1’ | 104.9 | 4.89, 1H, d, 7.8 Hz | C-3 |
| 2’ | 79.1 | 4.38 | GlcA C-1’, C-3’, Gal C-1" |
| 3’ | 86.1 | 4.20 | GlcA C-2’, C-4’, Ara C-1’" |
| 4’ | 71.5 | 4.42 | GlcA C-3’, C-5’, C-6’ |
| 5’ | 78.0 | 4.52 | GlcA C-4’, C-6’ |
| 6’ | 171.9 | - | - |
| Gal | | | |
| 1" | 104.6 | 5.32, 1H, d, 7.7 Hz | GlcA C-2’ |
| 2" | 73.6 | 4.42 | Gal C-1 ", C-3" |
| 3" | 74.9 | 4.10 | Gal C-2" |
| 4" | 69.5 | 4.56 | Gal C-2", C-3" |
| 5" | 76.4 | 3.94 | Gal C-4", C-6" |
| 6" | 61.6 | 4.43, 4.52 | Gal C-4", C-5" |
| Ara-f | | | |
| 1 "’ | 110.6 | 6.03. 1H, br s | GlcA C-3’, Ara C-2"’, C-4"’ |
| 2"’ | 83.4 | 4.94 | Ara C-3"’ |
| 3"’ | 78.3 | 4.78 | Ara C-2"’ |
| 4"’ | 85.2 | 4.82 | Ara C-5"’ |
| 5’" | 62.2 | 4.12, 4.28 | Ara C-3"’ |
| 21-O-Ang | | | |
| 1"" | 167.7 | - | - |

(continued)

| Position | C | H | Key HMBC correlations |
|---|---|---|---|
| 2"" | 129.6 | - | - |
| 3"" | 137.2 | 5.96, 1H, dq, 7.0/1.5 Hz | Ang C-1 "", C-4"", C-5"" |
| 4"" | 15.5 | 2.10, 3H, dq, 7.0/1.5 Hz | Ang C-2"", C-3"" |
| 5"" | 20.8 | 2.00, 3H, s | Ang C-1"", C-2"", C-3"" |
| 22-O-Ang | | | |
| 1"" | 167.9 | - | - |
| 2"" | 129.8 | - | - |
| 3"" | 136.3 | 5.78, 1H, dq, 7.0/1.5 Hz | Ang C-1"", C-4"", C-5"" |
| 4"" | 15.5 | 1.93, 3H, dq, 7.0/1.5 Hz | Ang C-2"", C-3"" |
| 5"" | 20.5 | 1.74, 3H, s | Ang C-1"", C-2"", C-3"" |
| [a] The data were assigned based on HMQC and HMBC correlations. | | | |

**Table 7.1: 13C and 1 H NMR data for Y2 (in Pyridine-d5)[a]**

| Position | C | H |
|---|---|---|
| 1 | 38.4 | 0.83, 1.36 |
| 2 | 26.4 | 1.89, 2.25 |
| 3 | 91.3 | 3.39, 1H, m |
| 4 | 43.4 | - |
| 5 | 56.7 | 0.87, 1H, d, 12.0 Hz |
| 6 | 18.6 | 1.31, 1.57 |
| 7 | 36.3 | 1.97,2.12 |
| 8 | 40.7 | - |
| 9 | 46.7 | 1.63 |
| 10 | 36.6 | - |
| 11 | 23.9 | 1.69, 1.89 |
| 12 | 125.1 | 5.48, 1H, br s |
| 13 | 143.4 | - |
| 14 | 47.5 | - |
| 15 | 67.1 | 4.18, 1H, d, 4.1 Hz |
| 16 | 73.2 | 4.43 |
| 17 | 48.1 | - |
| 18 | 41.4 | 3.06 |
| 19 | 46.6 | 1.40, 3.08 |
| 20 | 36.1 | - |
| 21 | 78.3 | 6.69, 1H, d, 10.2 Hz |
| 22 | 73.1 | 6.30, 1H, d, 10.2 Hz |
| 23 | 22.0 | 1.29, 3H, s |

(continued)

| Position | C | H |
|---|---|---|
| 24 | 62.9 | 3.28, 1H, d, 11.2 Hz; 4.32 |
| 25 | 15.6 | 0.64, 3H, s |
| 26 | 17.1 | 0.94, 3H, s |
| 27 | 20.8 | 1.84, 3H, s |
| 28 | 63.1 | 3.48, 3.72 (each, 1H, d, 10.6 Hz) |
| 29 | 29.3 | 1.09, 3H, s |
| 30 | 20.0 | 1.32, 3H, s |
| 3-O-GlcA | | |
| 1 | 104.5 | 4.87, 1H, d, 7.2 Hz |
| 2 | 78.6 | 4.31 |
| 3 | 86.5 | 4.23 |
| 4 | 71.6 | 4.45 |
| 5 | 77.4 | 4.53 |
| 6 | 171.9 | |
| Glc | | |
| 1 | 103.7 | 5.48, 1H, d, 7.8 Hz |
| 2 | 75.3 | 4.02 |
| 3 | 78.0 | 4.31 |
| 4 | 69.3 | 4.52 |
| 5 | 78.2 | 3.62 |
| 6 | 61.5 | 4.33, 4.50 |
| Ara | | |
| 1 | 110.1 | 6.05, 1H, br s |
| 2 | 83.5 | 4.97 |
| 3 | 77.8 | 4.74 |
| 4 | 85.0 | 4.84 |
| 5 | 62.2 | 4.18, 4.33 |
| 21-O-ang | | |
| 1 | 167.5 | - |
| 2 | 128.7 | - |
| 3 | 137.2 | 5.95, 1H, dd, 14.4/7.2 Hz |
| 4 | 16.7 | 2.08, 3H, d, 7.2 Hz |
| 5 | 20.6 | 2.00, 3H, s |
| 22-O-ang | | |
| 1 | 167.9 | - |
| 2 | 128.9 | - |
| 3 | 136.3 | 5.76, 1H, dd, 14.4/7.2 Hz |
| 4 | 15.6 | 1.95, 3H, dd, 7.2 Hz |

(continued)

| Position | C | H |
|---|---|---|
| 5 | 20.4 | 1.74, 3H, s |
| [a] The data were assigned based on COSY, HMQC and HMBC correlations. | | |

**Table 9.1. $^{13}$C and $^{1}$H NMR Data for $Y_8$ (in** pyridine-$d_5$)

| Position | $^{13}$C | $^{1}$H |
|---|---|---|
| 1 | 38.2 | 0.74, 1.30 |
| 2 | 26.3 | 1.85, 2.26 (1H, m) |
| 3 | 91.1 | 3.30 (1H, m) |
| 4 | 43.4 | - |
| 5 | 55.9 | 0.82 |
| 6 | 18.2 | 1.22, 1.48 |
| 7 | 32.9 | 1.24, 1.49 |
| 8 | 39.8 | - |
| 9 | 46.5 | 1.67 |
| 10 | 36.2 | - |
| 11 | 23.8 | 1.70, 1.83 |
| 12 | 123.3 | 5.39 (1 H, br s) |
| 13 | 142.5 | - |
| 14 | 41.4 | - |
| 15 | 34.6 | 1.64, 1.83 |
| 16 | 68.4 | 4.53 |
| 17 | 47.8 | - |
| 18 | 39.7 | 3.09 |
| 19 | 47.0 | 1.39, 3.11 |
| 20 | 36.2 | - |
| 21 | 78.5 | 6.68 (1H, d, *J* = 10.2 Hz) |
| 22 | 73.4 | 6.30 (1H, d, *J* = 10.2 Hz) |
| 23 | 22.1 | 1.32 (3H, s) |
| 24 | 63.2 | 3.28, 4.31 (each, 1H, d, *J* = 10.8 Hz) |
| 25 | 15.4 | 0.62 (3H, s) |
| 26 | 16.4 | 0.78 (3H, s) |
| 27 | 27.3 | 1.82 (3H, s) |
| 28 | 63.3 | 3.39, 3.62 (each, 1H, d, *J* = 10.8 Hz) |
| 29 | 29.3 | 1.08 (3H, s) |
| 30 | 20.0 | 1.32 (3H, s) |
| 3-*O*-Glc A-*p* | | |
| 1 | 104.5 | 4.93 (1H, d, *J* = 7.2 Hz) |
| 2 | 78.0 | 4.23 |
| 3 | 86.2 | 4.25 |
| 4 | 71.6 | 4.44 |
| 5 | 77.3 | 4.53 |
| 6 | 171.9 | - |
| Glc-*p* | | |
| 1 | 103.7 | 5.48 (1H, d, *J* = 7.2 Hz) |
| 2 | 75.3 | 4.04 |
| 3 | 77.8 | 4.27 |
| 4 | 69.3 | 4.48 |

(continued)

| Position | $^{13}C$ | $^{1}H$ |
|---|---|---|
| 5 | 78.2 | 3.61 |
| 6 | 61.1 | 4.38, 4.48 |
| Ara-*f* | | |
| 1 | 111.1 | 6.04 (1H, br s) |
| 2 | 83.5 | 4.97 |
| 3 | 77.4 | 4.84 |
| 4 | 85.2 | 4.86 |
| 5 | 62.1 | 4.12, 4.37 |
| 21-O-Ang | | |
| 1 | 167.5 | - |
| 2 | 128.9 | - |
| 3 | 137.0 | 5.93 (1H, q, $J$ = 7.2 Hz) |
| 4 | 15.7 | 2.07 (3H, d, $J$ = 7.2 Hz) |
| 5 | 20.8 | 2.00 (3H, s) |
| 22-O-Ang | | |
| 1 | 167.9 | - |
| 2 | 128.9 | - |
| 3 | 136.2 | 5.87 (1H, q, $J$ = 7.2 Hz) |
| 4 | 15.6 | 2.03 (3H, d, $J$ = 7.2 Hz) |
| 5 | 20.6 | 1.88 (3H, s) |

[a-g] The data with the same labels in each column may be interchangeable.

**Table 11.1 $^{13}C$ and $^{1}H$ NMR Data for $Y_{10}$ (in pyridine-$d_5$)**

| Position | $^{13}C$ | $^{1}H$ |
|---|---|---|
| 1 | 38.5 | 0.87, 1.38 |
| 2 | 26.4 | 1.86, 2.12 (1H, m) |
| 3 | 89.7 | 3.24 (1H, dd, J = 12.0/4.2 Hz) |
| 4 | 39.8 | - |
| 5 | 55.6 | 0.75 |
| 6 | 18.2 | 1.29, 1.49 |
| 7 | 32.9 | 1.27, 1.54 |
| 8 | 39.8 | - |
| 9 | 46.7 | 1.68 |
| 10 | 36.5 | - |
| 11 | 23.6 | 1.70, 1.83 |
| 12 | 123.3 | 5.40 (1H, brs) |
| 13 | 142.5 | - |
| 14 | 41.4 | - |
| 15 | 34.8 | 1.60, 1.83 |
| 16 | 68.4 | 4.49 |
| 17 | 47.8 | - |
| 18 | 39.7 | 3.06 |
| 19 | 47.0 | 1.40,3.10 |
| 20 | 36.1 | - |
| 21 | 78.5 | 6.69 (1H, d, $J$ = 10.2 Hz) |
| 22 | 73.5 | 6.31 (1H, d, $J$ = 10.2 Hz) |
| 23 | 27.7 | 1.30 (3H, s) |
| 24 | 16.5 | 1.17 (3H, s) |

(continued)

| Position | $^{13}C$ | $^{1}H$ |
|---|---|---|
| 25 | 15.4 | 0.80 (3H, s) |
| 26 | 16.7 | 0.83 (3H, s) |
| 27 | 27.3 | 1.83 (3H, s) |
| 28 | 63.4 | 3.40, 3.64 (each, 1H, d, *J* = 10.8 Hz) |
| 29 | 29.3 | 1.09 (3H, s) |
| 30 | 20.1 | 1.33 (3H, s) |
| 3-O-Glc A-*p* | | |
| 1 | 104.9 | 4.91 (1H, d, *J* = 7.8 Hz) |
| 2 | 78.7 | 4.40 |
| 3 | 86.1 | 4.23 |
| 4 | 71.5 | 4.44 |
| 5 | 77.1 | 4.53 |
| 6 | 171.8 | - |
| Gla-*p* | | |
| 1 | 104.6 | 5.34 (1H, d, *J* = 7.8 Hz) |
| 2 | 73.4 | 4.50 |
| 3 | 74.9 | 4.11 |
| 4 | 69.6 | 4.58 |
| 5 | 76.4 | 3.98 |
| 6 | 61.6 | 4.47, 4.52 |
| Ara-*f* | | |
| 1 | 110.9 | 6.05 (1H, brs) |
| 2 | 83.4 | 4.95 |
| 3 | 77.5 | 4.78 |
| 4 | 85.2 | 4.83 |
| 5 | 62.1 | 4.16, 4.39 |
| 21-O-Ang | | |
| 1 | 167.5 | - |
| 2 | 128.8 | - |
| 3 | 137.9 | 5.92 (1H, q, 7.2 Hz) |
| 4 | 15.7 | 2.07 (3H, d, 7.2 Hz) |
| 5 | 20.8 | 2.00 (3H, s) |
| 22-O-Ang | | |
| 1 | 167.9 | - |
| 2 | 128.8 | - |
| 3 | 136.8 | 5.87 (1H, q, 7.2 Hz) |
| 4 | 15.6 | 2.03 (3H, d, 7.2 Hz) |
| 5 | 20.6 | 1.88 (3H, s) |

Although the present invention has been described in detail with particular reference to preferred embodiments thereof, it should be understood that the invention is capable of other different embodiments, and its details are capable of modifications in various obvious aspects. As is readily apparent to those skilled in the art, variations and modifications can be affected while remaining within the spirit and scope of the invention. Accordingly, the foregoing disclosure, description, and figures are for illustrative purpose only, and do not in any way limit the invention which is defined only by the claims.

## Claims

**1.** The compound for use in a method of treating ovarian cancer, having the following structure:

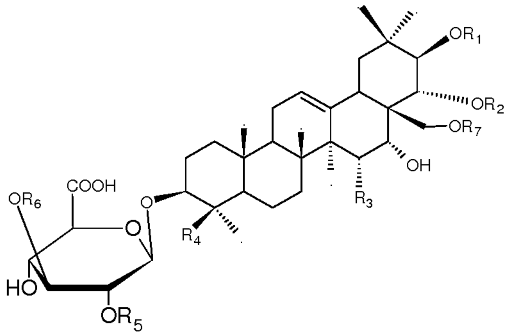

wherein R5 is a glucopyranosyl, R6 is a arabinofuranosyl, R1 is angeloyl, R2 is angeloyl, R3 is OH, R4=$CH_2OH$, R7=H, and said compound has the following structure (Y2):

Y2

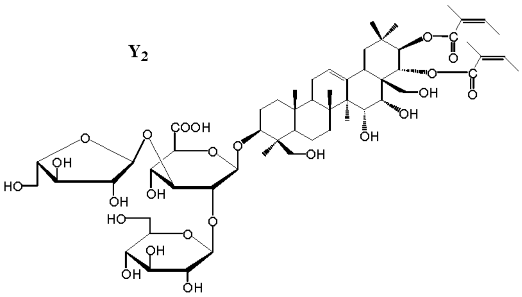

or is named 3-O-[β-D-glucopyranosyl-(1→2)]-α-L-arabinofuranosyl(1→3)-β-D-glucuronopyranosyl-21,22-O-diangeloyl-3β, 15α, 16α, 21β, 22α, 24β, 28-heptahydroxyolean-12-ene); or

wherein R5 is a glucopyranosyl, R6 is an arabinofuranosyl, R1 is angeloyl, R2 is angeloyl, R3 is H, R4=$CH_2OH$, and R7=H, and said compound has the following structure (Y8):

Y-8

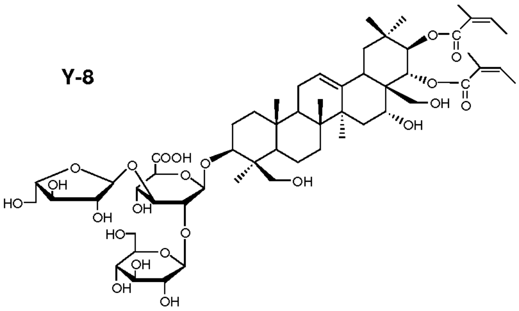

or is named 3-O-[*β*-glucopyranosyl (1→2)]-*α*-arabinofuranosyl (1→3)-*β*-glucuronopyranosyl-21, 22-*O*-diangeloyl-3*β* 16*α*, 21*β* *22α,* 24*β*, 28-hexahydroxyolean-12-ene, or

wherein R5 is a galactopyranosyl, R6 is a arabinofuranosyl, R1 is angeloyl, R2 is angeloyl, R3 is H, R4 is $CH_3$, R7=H, and said compound has the following structure (Y10)

Y-10

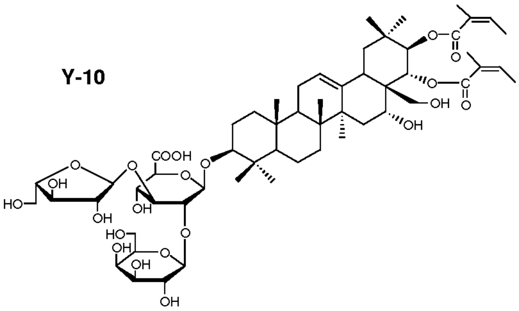

or is named (3-O-[$\beta$-galactopyranosyl (1→2)]-$\alpha$-arabinofuranosyl (1→3)-$\beta$-glucuronopyranosyl-21, 22-O-diangeloyl-3$\beta$ 16$\alpha$, 21$\beta$, 22$\alpha$, 28-pentahydroxyolean-12-ene.

**2.** The compound for use in a method of treating ovarian cancer according to claim 1, which is in the form of a salt.

**3.** The compound for use in a method of treating ovarian cancer according to claim 1, being isolated from natural sources.

**4.** The compound for use in a method of treating ovarian cancer according to claim 1, which is obtainable by the method comprising the steps of:

(a) extracting Xanthoceras sorbifolia or plant powder with organic solvents to obtain an organic extract; wherein the powder is prepared from the husks, branches, stems, leaves, kernels, roots, barks or seed shells of the herb or plant.
(b) collecting the organic extract;
(c) refluxing the organic extract to obtain a second extract;
(d) removing the organic solvent from the second extract;
(e) drying and sterilizing the second extract to obtain a crude extract powder;
(f) fractionating the crude extract powder into components using HPLC and FPLC chromatography with silica gel, C18 or other equivalent solid phase materials;
(g) monitoring absorption wavelength at 207nm or 254nm;
(h) identifying the bioactive components of the crude extract powder;
(i) purifying the bioactive components of the crude extract powder with FPLC to obtain a fraction of the bioactive components; and
(j) isolating the compound from the fraction of the bioactive component with preparative HPLC.

**Patentansprüche**

**1.** Verbindung zur Verwendung in einem Verfahren zur Behandlung von Eierstockkrebs mit der folgenden Struktur:

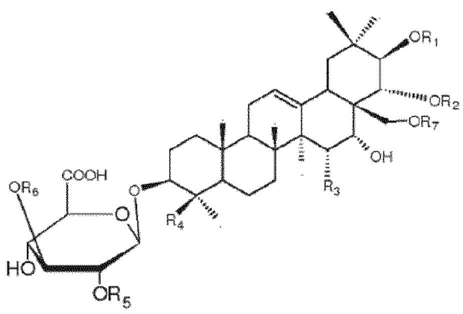

wobei R5 ein Glucopyranosyl ist, R6 ein Arabinofuranosyl ist, R1 Angeloyl ist, R2 Angeloyl ist, R3 OH ist, R4=$CH_2OH$, R7=H ist und die Verbindung die folgende Struktur (Y2) aufweist:

Y2

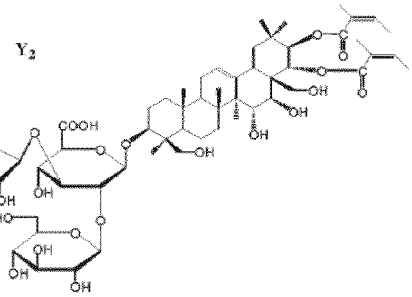

oder als 3-O-[β-D-Glucopyranosyl-(1→2)]-α-L-arabinofuranosyl(1→3)-β-D-glucuronopyranosyl-21,22-O-diangeloyl-3β, 15α, 16α, 21β, 22α, 24β, 28-heptahydroxyolean-12-en) bezeichnet wird; oder
wobei R5 ein Glucopyranosyl ist, R6 ein Arabinofuranosyl ist, R1 Angeloyl ist, R2 Angeloyl ist, R3 H ist, R4=$CH_2OH$

und R7=H ist und die Verbindung die folgende Struktur (Y8) aufweist:

Y-8

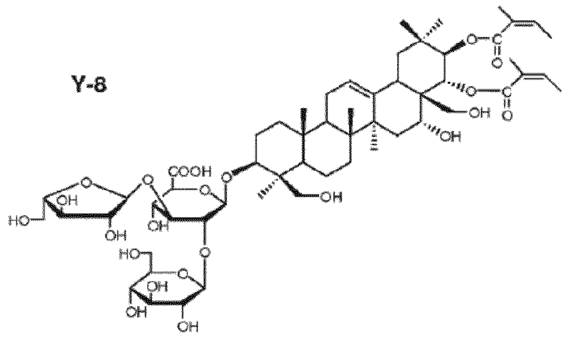

oder als 3-O-[β-Glucopyranosyl (1→2)]-α-arabinofuranosyl (1→3)-β-glucuronopyranosyl-21, 22-O-diangeloyl-3β, 16α, 21β, 22α, 24β, 28-hexahydroxyolean-12-en bezeichnet wird oder
wobei R5 ein Galactopyranosyl ist, R6 ein Arabinofuranosyl ist, R1 Angeloyl ist, R2 Angeloyl ist, R3 H ist, R4 $CH_3$ ist, R7=H ist und die Verbindung die folgende Struktur (Y10) aufweist:

Y-10

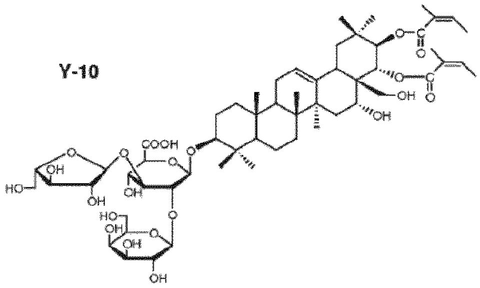

oder als (3-O-[β-Galactopyranosyl (1→2)]-α-arabinofuranosyl (1→3)-β-glucuronopyranosyl-21, 22-O-diangeloyl-3β, 16α, 21β, 22α, 28-pentahydroxyolean-12-en bezeichnet wird.

**2.** Verbindung zur Verwendung in einem Verfahren zur Behandlung von Eierstockkrebs nach Anspruch 1, die in Form eines Salzes vorliegt.

**3.** Verbindung zur Verwendung in einem Verfahren zur Behandlung von Eierstockkrebs nach Anspruch 1, die aus natürlichen Quellen isoliert wird.

**4.** Verbindung zur Verwendung in einem Verfahren zur Behandlung von Eierstockkrebs nach Anspruch 1, die durch das Verfahren erhältlich ist, das die folgenden Schritte umfasst:

(a) Extrahieren von Xanthoceras sorbifolia oder eines Pflanzenpulvers mit organischen Lösungsmitteln, um einen organischen Extrakt zu erhalten; wobei das Pulver aus den Hülsen, Zweigen, Stämmen, Blättern, Kernen, Wurzeln, Rinden oder Samenschalen des Krauts oder der Pflanze hergestellt wird;
(b) Sammeln des organischen Extrakts;
(c) Erhitzen des organischen Extrakts bis zum Rückfluss, um einen zweiten Extrakt zu erhalten;
(d) Entfernen des organischen Lösungsmittels aus dem zweiten Extrakt;
(e) Trocknen und Sterilisieren des zweiten Extrakts, um ein Rohextraktpulver zu erhalten;
(f) Fraktionieren des Rohextraktpulvers in Bestandteile unter Verwendung von HPLC- und FPLC-Chromatographie mit Silicagel, C18 oder anderen gleichwertigen Festphasenmaterialien;
(g) Überwachen einer Absorptionswellenlänge bei 207 nm oder 254 nm;
(h) Identifizieren der bioaktiven Bestandteile des Rohextraktpulvers;
(i) Reinigen der bioaktiven Bestandteile des Rohextraktpulvers mit FPLC, um eine Fraktion der bioaktiven Bestandteile zu erhalten; und
(j) Isolieren der Verbindung aus der Fraktion des bioaktiven Bestandteils mit präparativer HPLC.

## Revendications

**1.** Composé pour son utilisation dans un procédé de traitement du cancer ovarien, possédant la structure suivants :

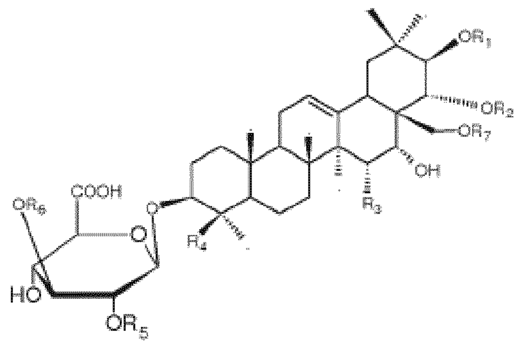

dans laquelle R5 est un glucopyranosyle, R6 est un arabinofuranosyle, R1 est un angéloyle, R2 est un angéloyle, R3 est OH, R4 = $CH_2OH$, R7 = H, et ledit composé possède la structure (Y2) suivants :

Y2

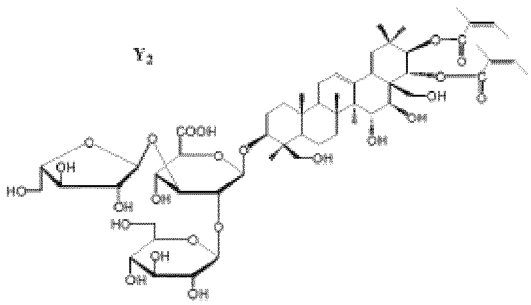

ou est appelé 3-O-[p-D-glucopyranosyl-(l-"2)-a-L-arabinofuranosyl(1→3)-β-D-glucuronopyranosyl-21,22-O-diangéloyl-3β,15α,16α,21β,22α,24β,28-heptahydroxyoléan-12-ène) ; ou
dans laquelle R5 est un glucopyranosyle, R6 est un arabinofuranosyle, R1 est un angéloyle, R2 est un angéloyle, R3 est H, R4 = $CH_2OH$ et R7 = H, et ledit composé possède la structure (Y8) suivants :

Y-8

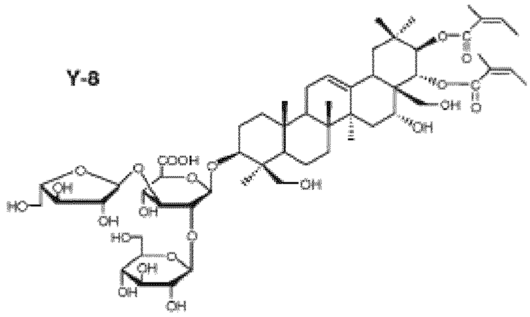

ou est appelé 3-O-[β-glucopyranosyl(1→2)-α-arabinofuranosyl (1→3)-β-glucuronopyranosyl-21,22-O-diangéloyl-3β,16α,21β,22α,24β,28-hexahydroxyoléan-12-ène) ; ou
dans laquelle R5 est un galactopyranosyle, R6 est un arabinofuranosyle, R1 est un angéloyle, R2 est un angéloyle, R3 est H, R4 est $CH_3$, R7 = H, et ledit composé possède la structure (Y10) suivants :

Y-10

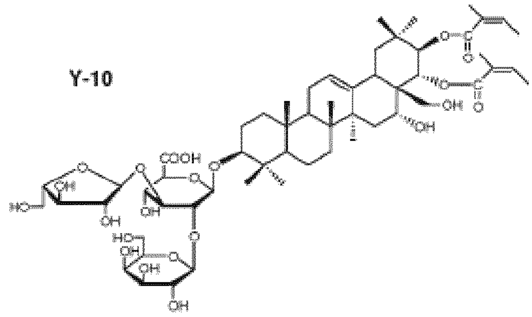

ou est appelé (3-O-[β-galactopyranosyl(1→2)-α-arabinofuranosyl(1→3)-β-glucuronopyranosyl-21,22-O-diangéloyl-3β,16α,21β,22α,28-pentahydroxyoléan-12-ène.

2. Composé pour son utilisation dans un procédé de traitement du cancer ovarien selon la revendication 1, qui est sous la forme d'un sel.

3. Composé pour son utilisation dans un procédé de traitement du cancer ovarien selon la revendication 1, qui est isolé à partir de sources naturelles.

4. Composé pour son utilisation dans un procédé de traitement du cancer ovarien selon la revendication 1, qui peut être obtenu par le procédé comprenant les étapes suivantes :

(a) l'extraction d'une poudre de Xanthoceras sorbifolia ou d'une plante avec des solvants organiques pour obtenir un extrait organique ; dans laquelle la poudre est préparée à partir des cosses, des branches, des tiges, des feuilles, des noyaux, des racines, des écorces ou des coques des graines de l'herbe ou de la plante,
(b) la collecte de l'extrait organique ;
(c) la mise à reflux de l'extrait organique pour obtenir un second extrait ;
(d) l'élimination du solvant organique du second extrait ;
(e) le séchage et la stérilisation du second extrait pour obtenir une poudre brute d'extrait ;
(f) le fractionnement de la poudre brute d'extrait en composants par chromatographie CLHP et FPLC avec du gel de silice, une phase en C18 ou d'autres matières de phase solide équivalentes ;
(g) la surveillance de la longueur d'onde d'absorption à 207 nm ou 254 nm ;
(h) l'identification des composants bioactifs de la poudre brute d'extrait ;
(i) la purification des composants bioactifs de la poudre brute d'extrait par FPLC pour obtenir une fraction des composants bioactifs ; et
(j) l'isolement du composé à partir de la fraction du composant bioactif par CLHP préparative.

Figure 1

Structure of six anticancer Saponins with biangeloyl groups (Y3 (Y), Y2, Y8, Y10, Y1 and Y9)

$Y_3$

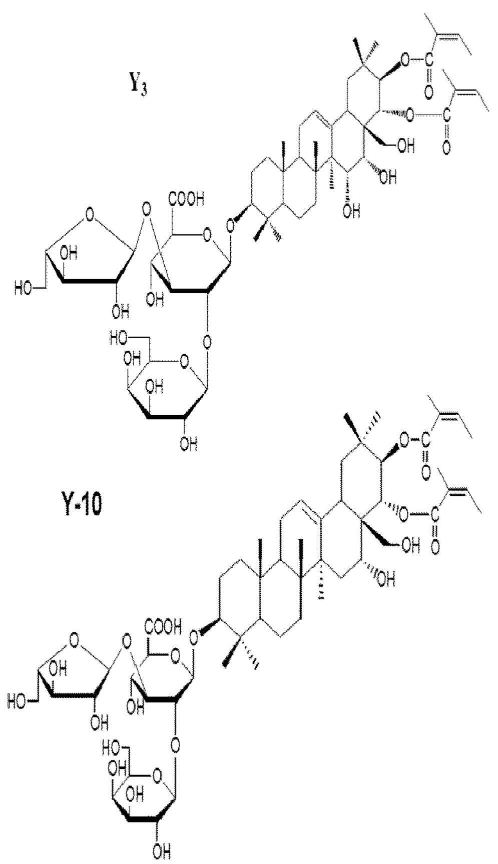

Y-10

$Y_2$

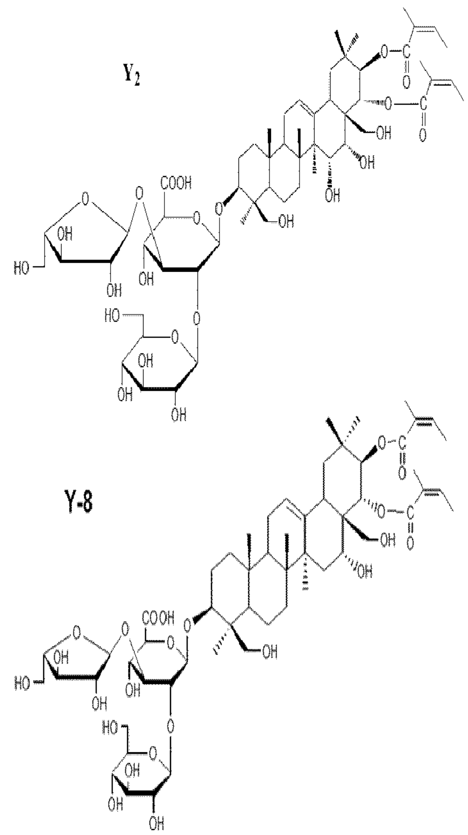

Y-8

$Y_1$

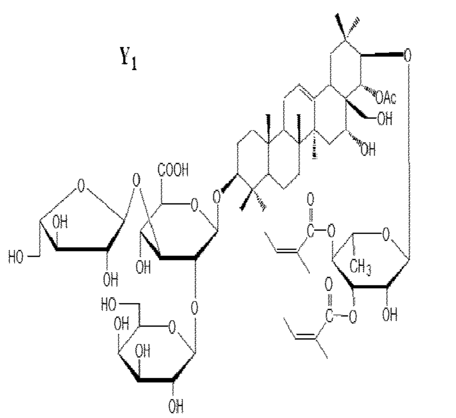

$Y_9$

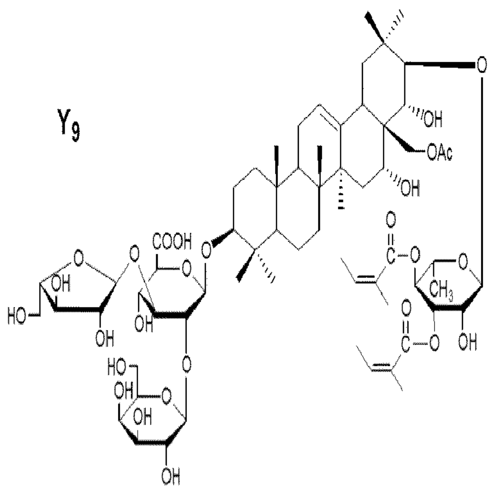

Figure 2
Anticancer activity of Compound Y

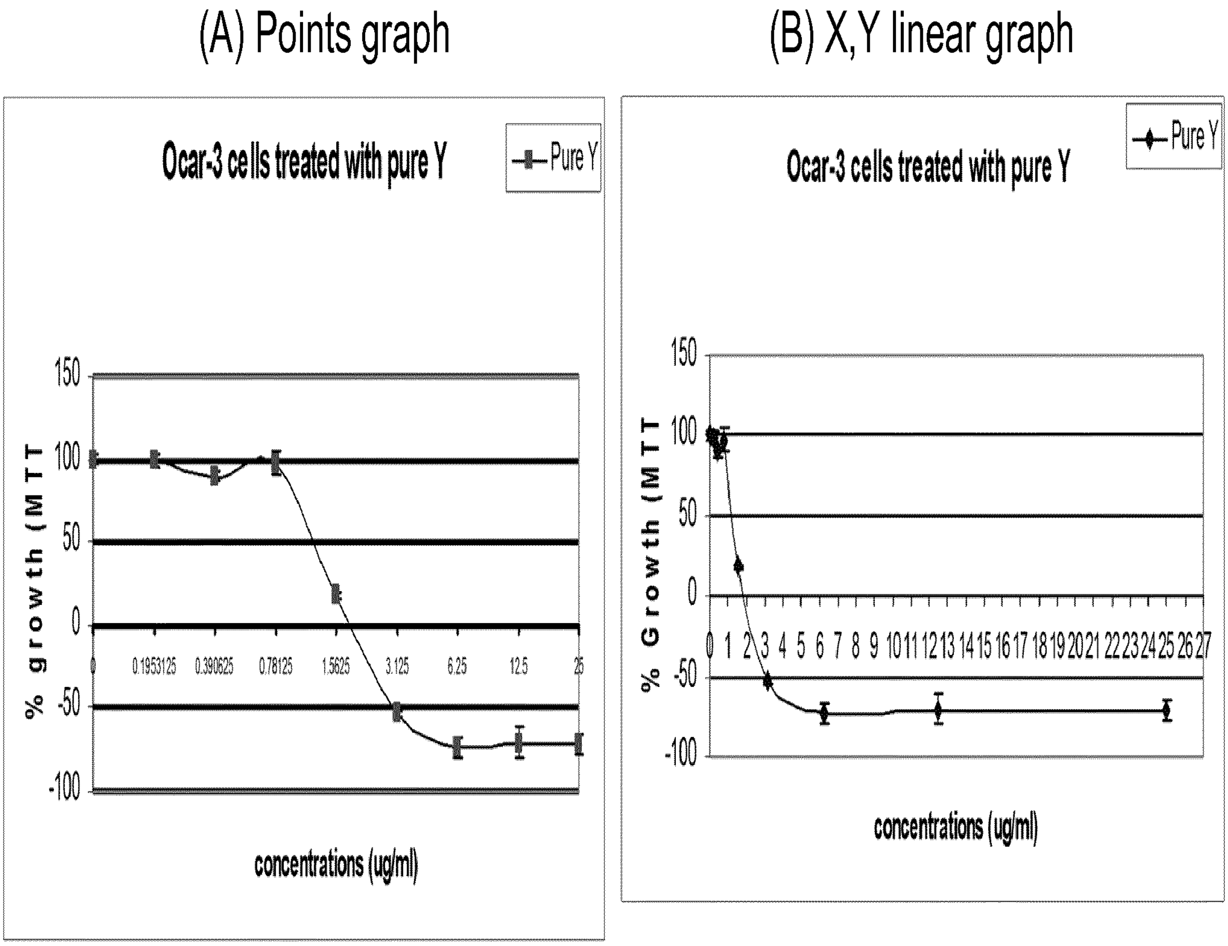

Figure 3
Y1 and Y2 activity on Ovarian caner cells

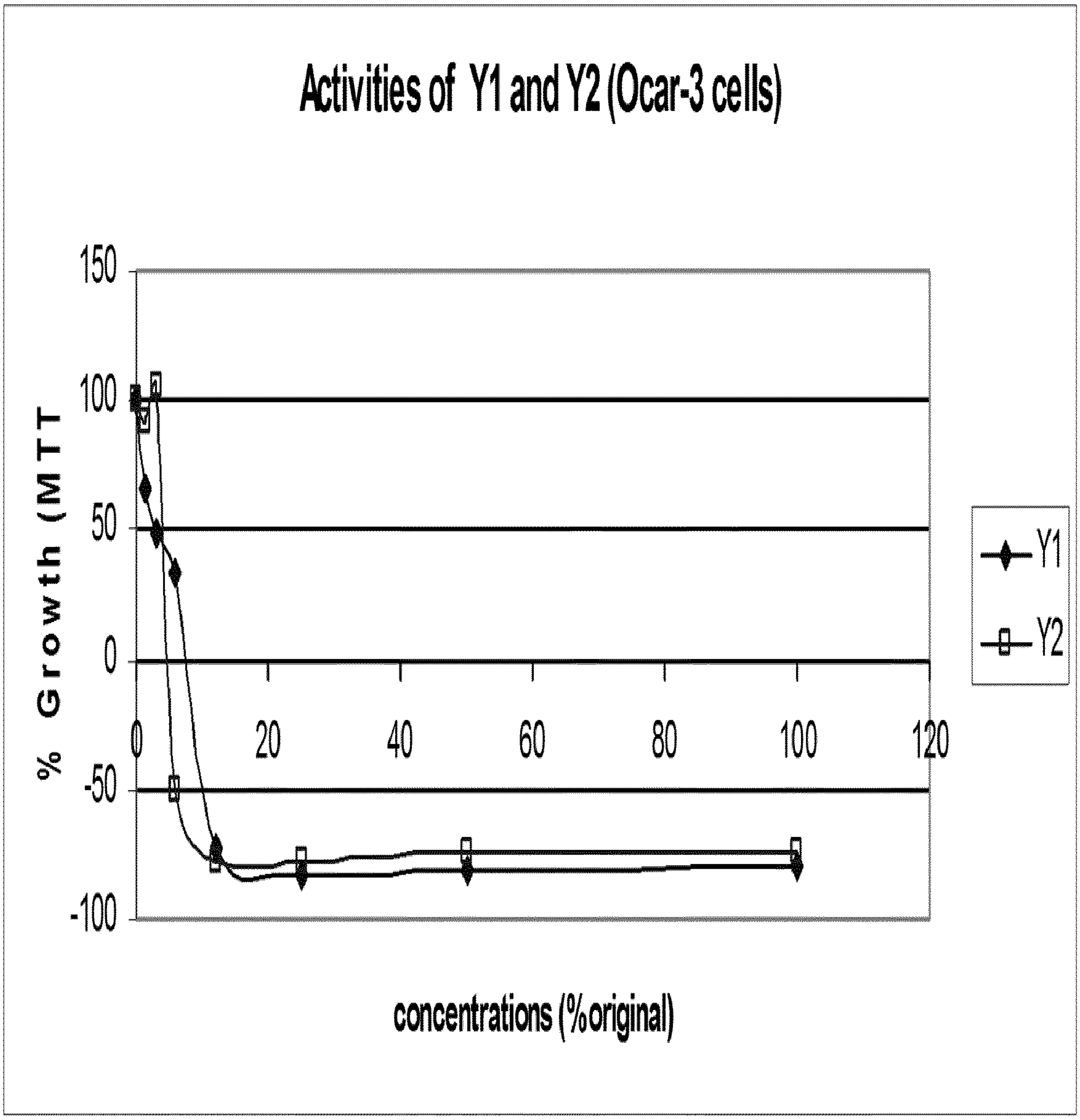

Figure 4
Anticancer activity of Compounds Y, Y8, Y9 and Y10.

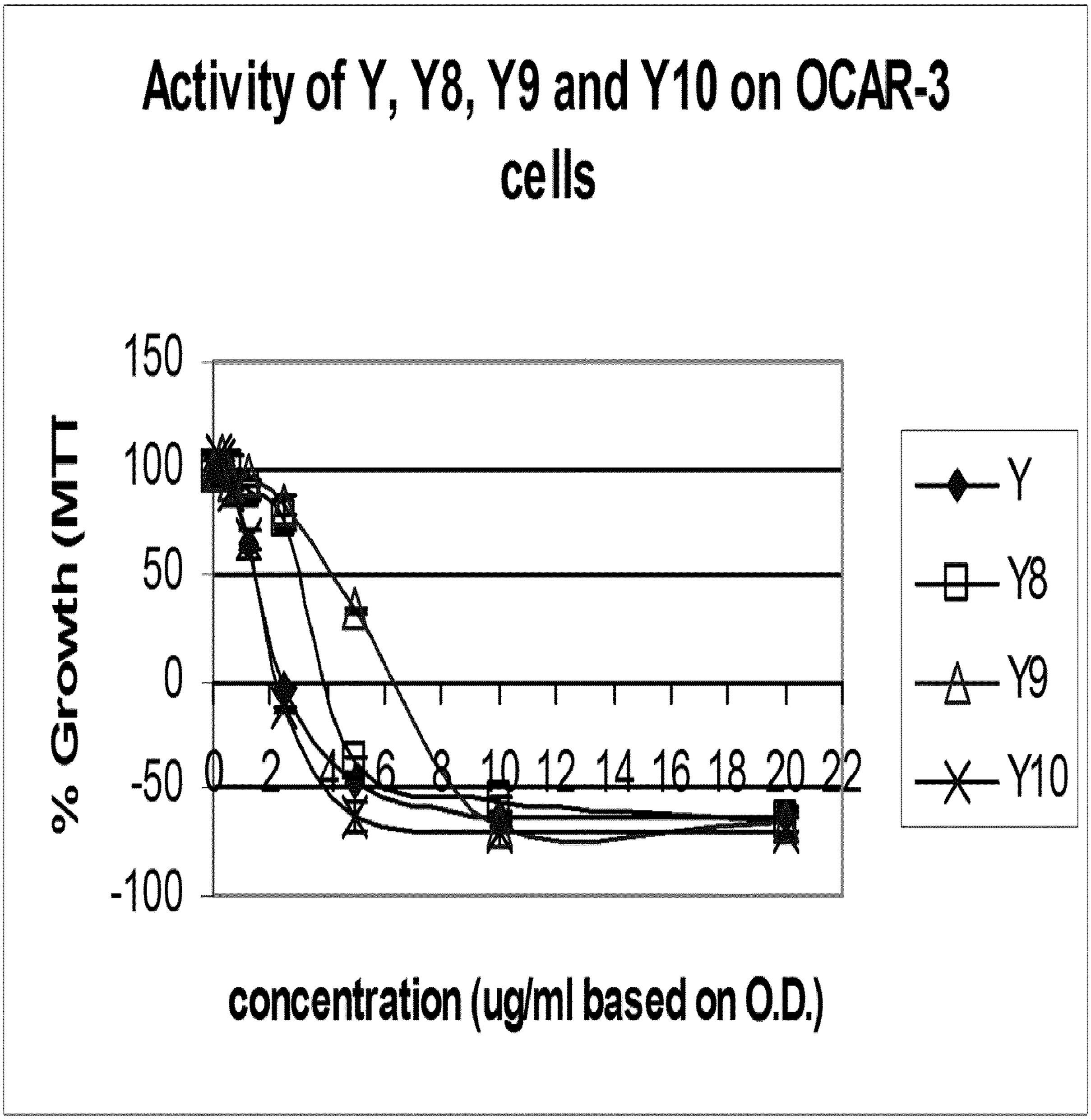

Figure 5
Consensus structure of compound Ys
(Y,Y2, Y8 and Y10)

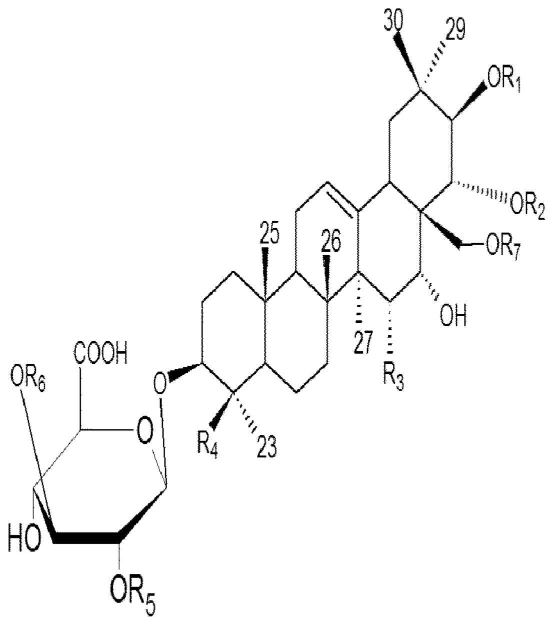

R1 = Angeloyl
R2 = Angeloyl
R3 = OH or H
R4 = CH3 or $CH_2OH$
R5 = D- glucose, D-galactose
R6 = L- arabinose
R7 = H,

Angeloyl = 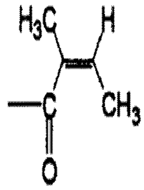

Figure 6

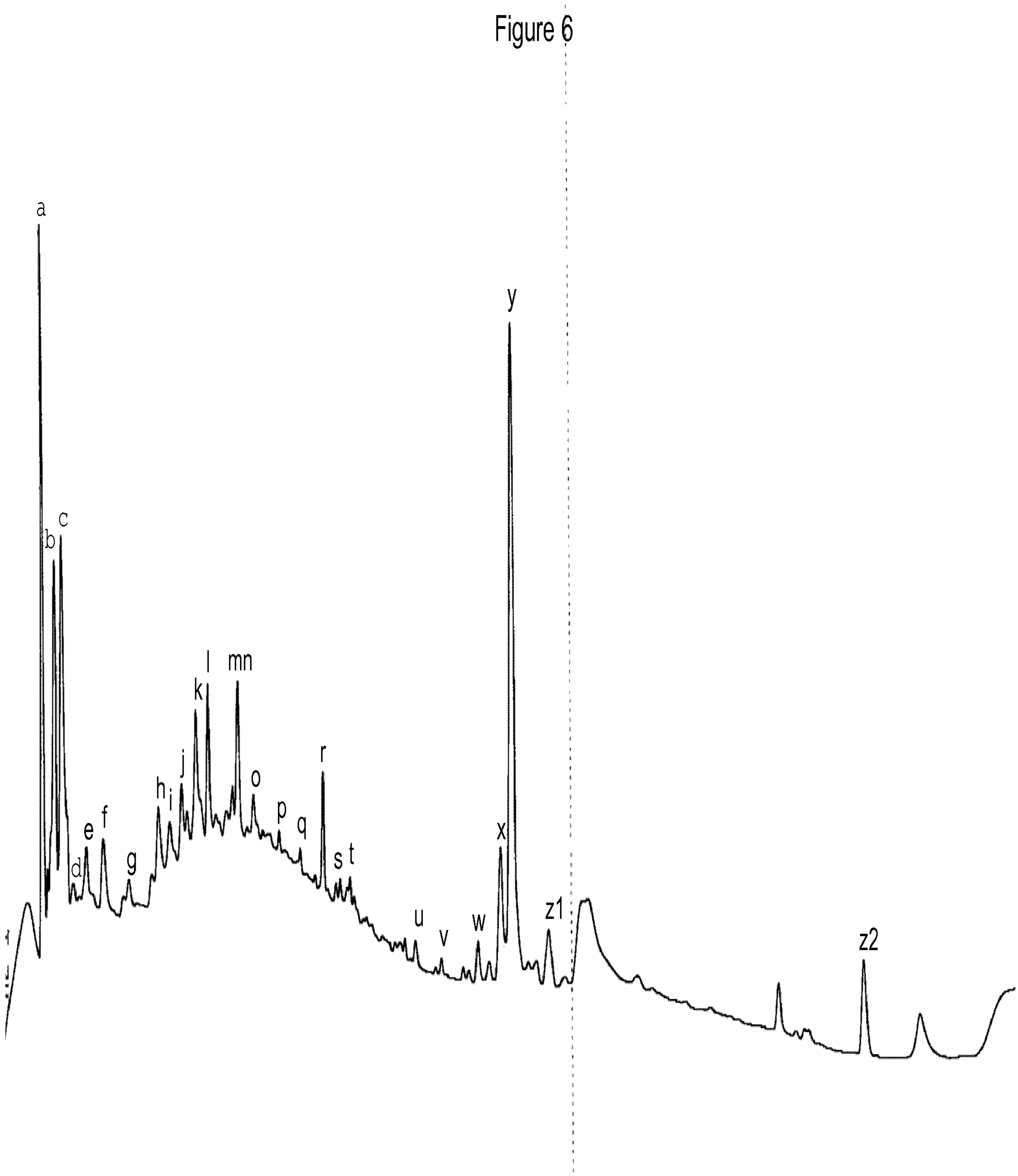

Figure 7
FPLC profile of Plant extract

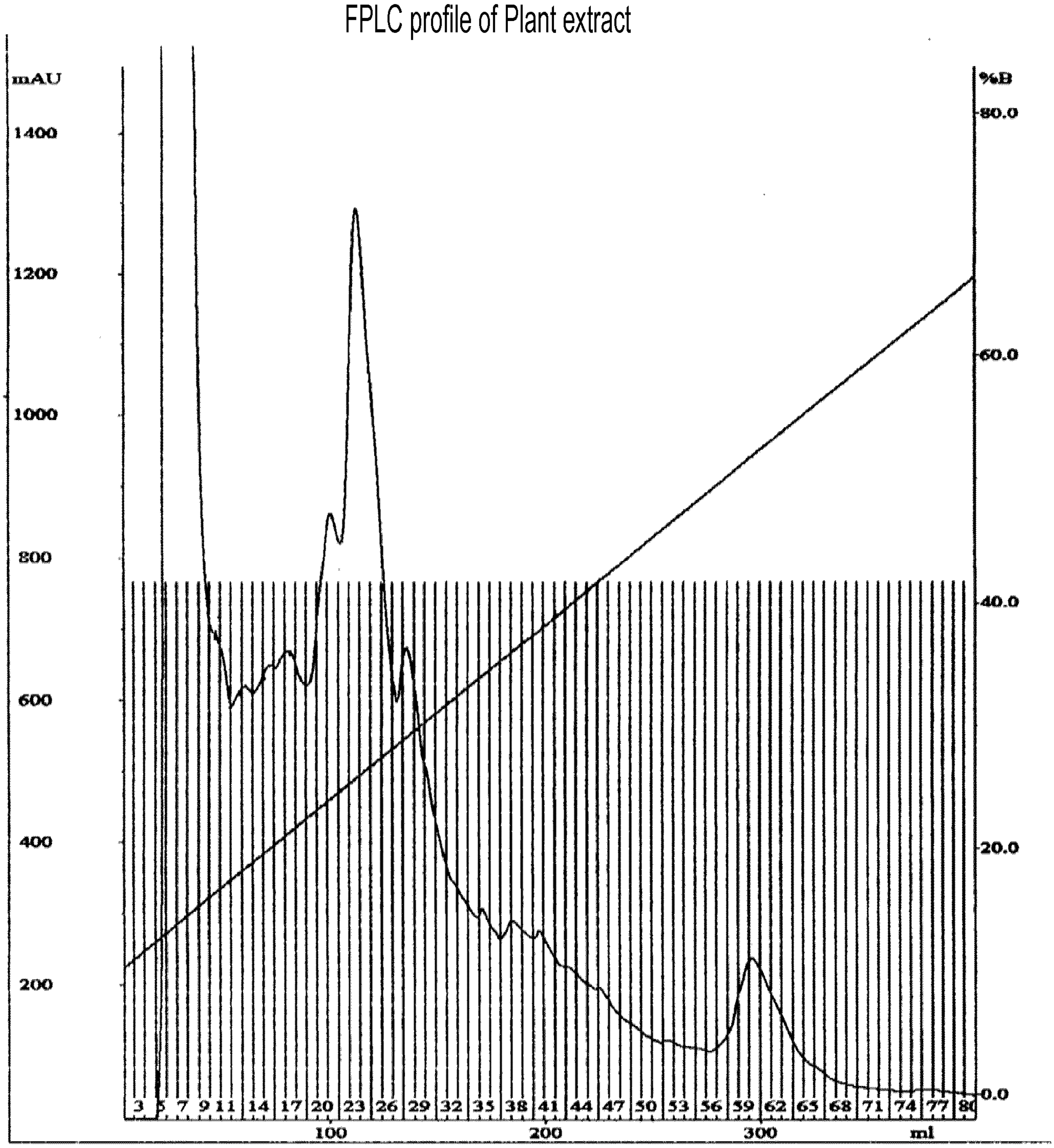

Figure 8
Cell growth activity of fractions of plant extract

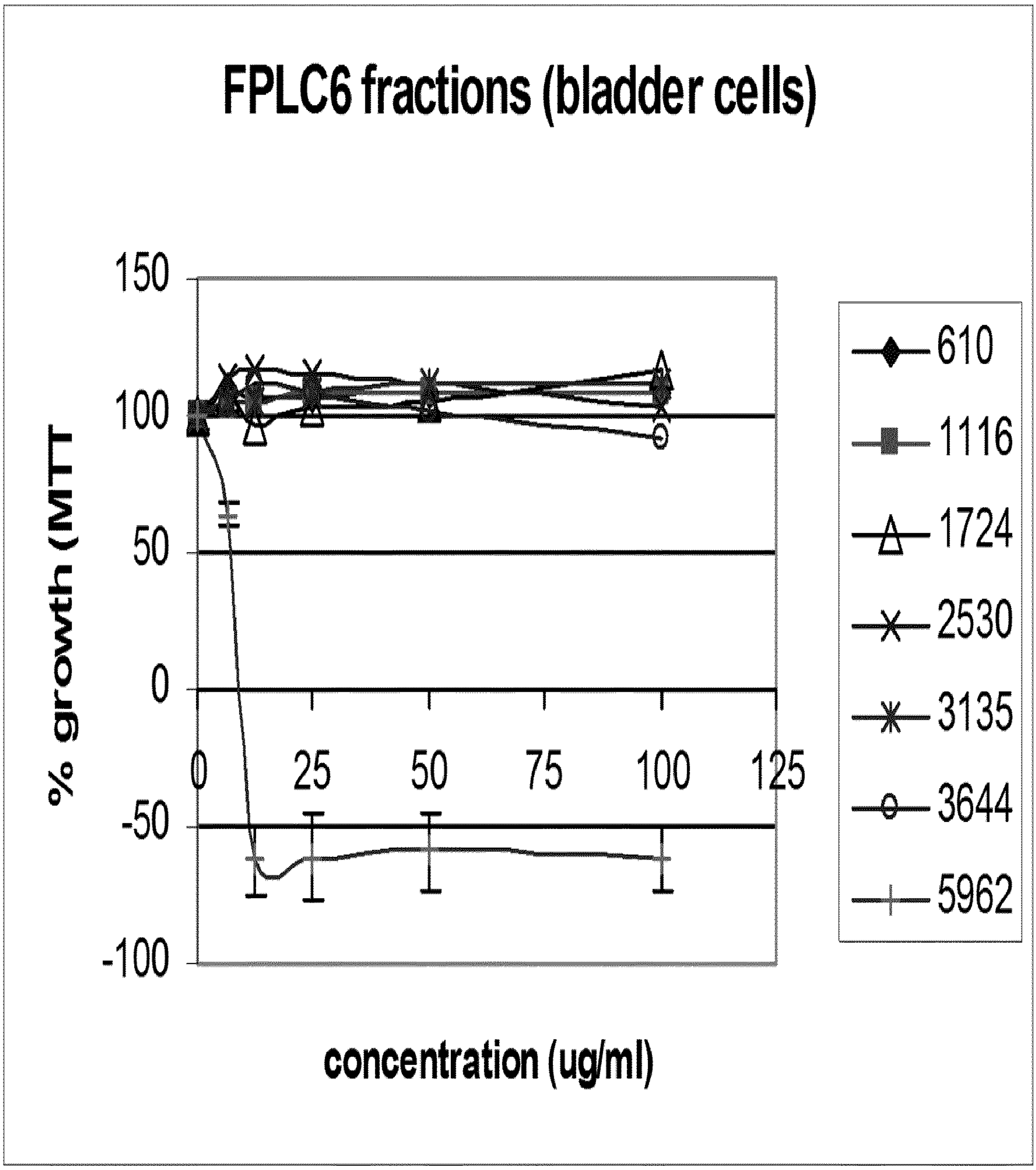

Figure 9
Purification of Factions Ys

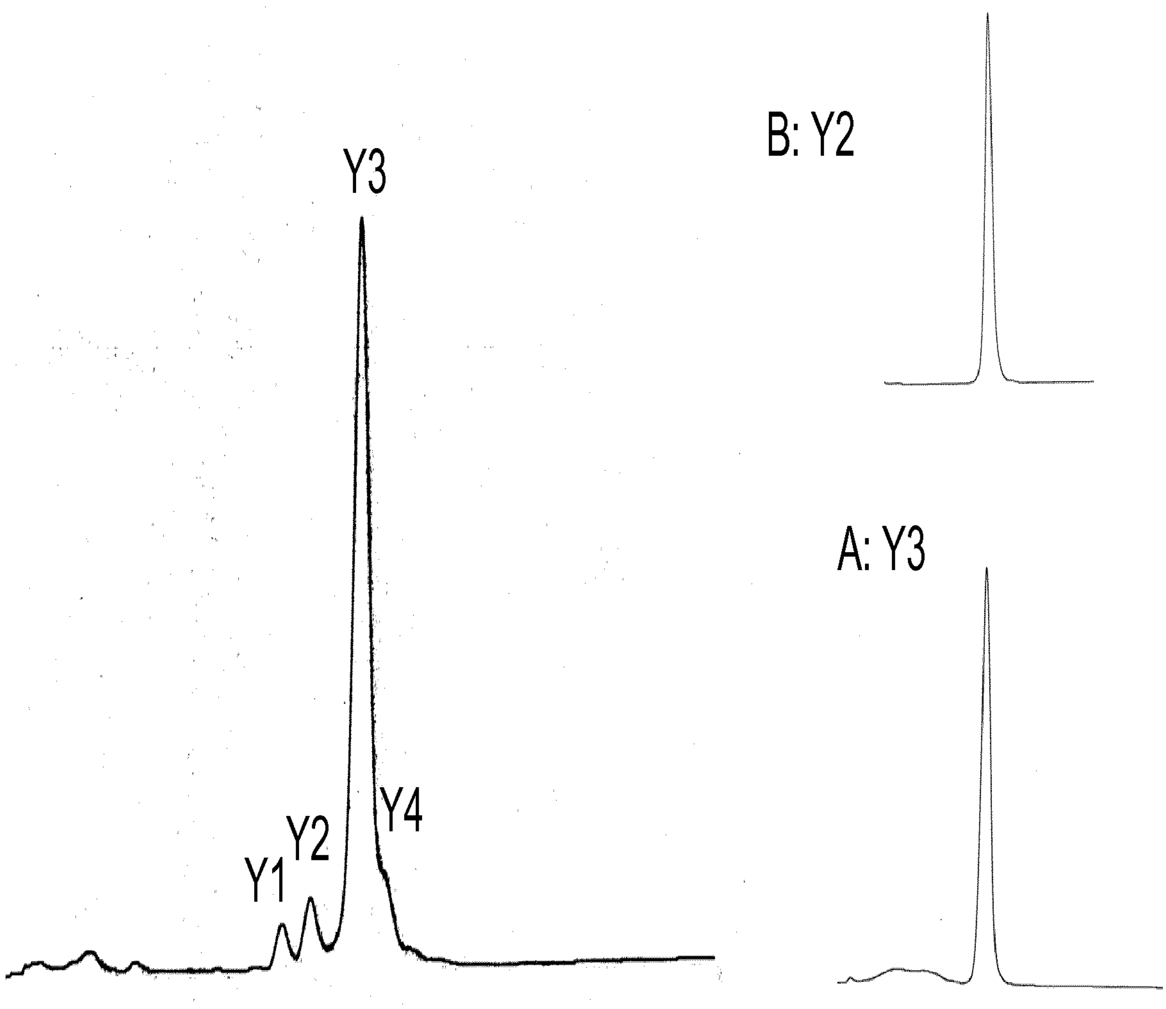

Figure 10
Purification of Y8-Y10 with HPLC (iso-45)

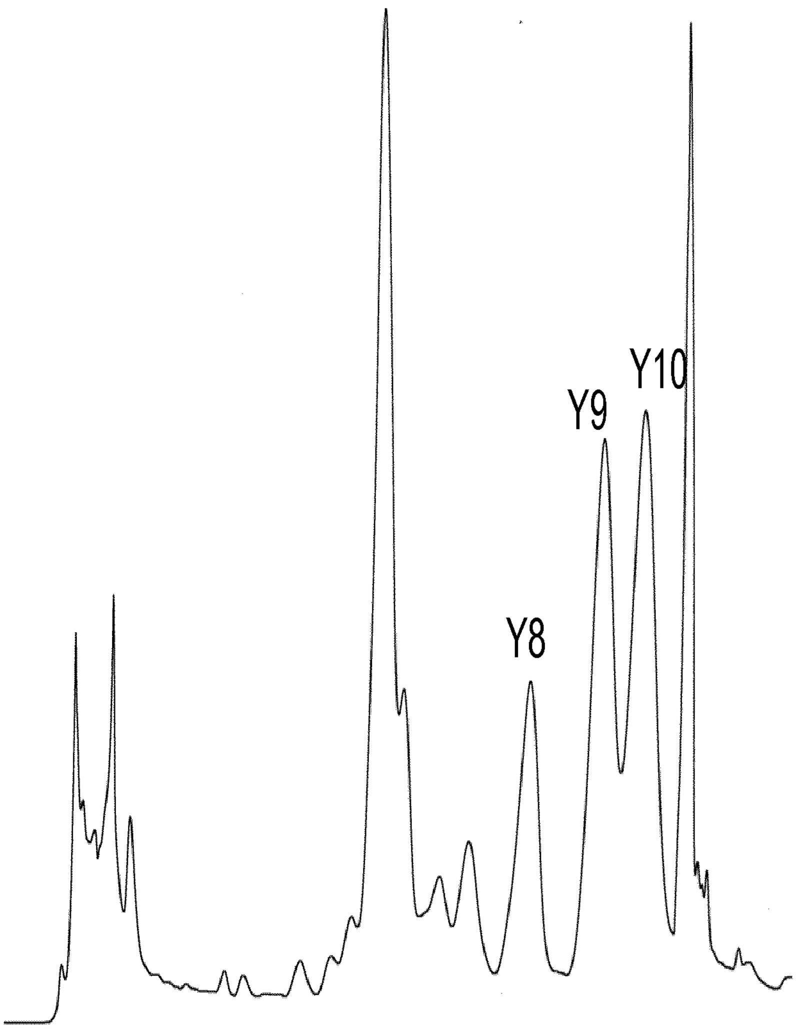

Figure 11
Purity of Y8, Y9 and Y10

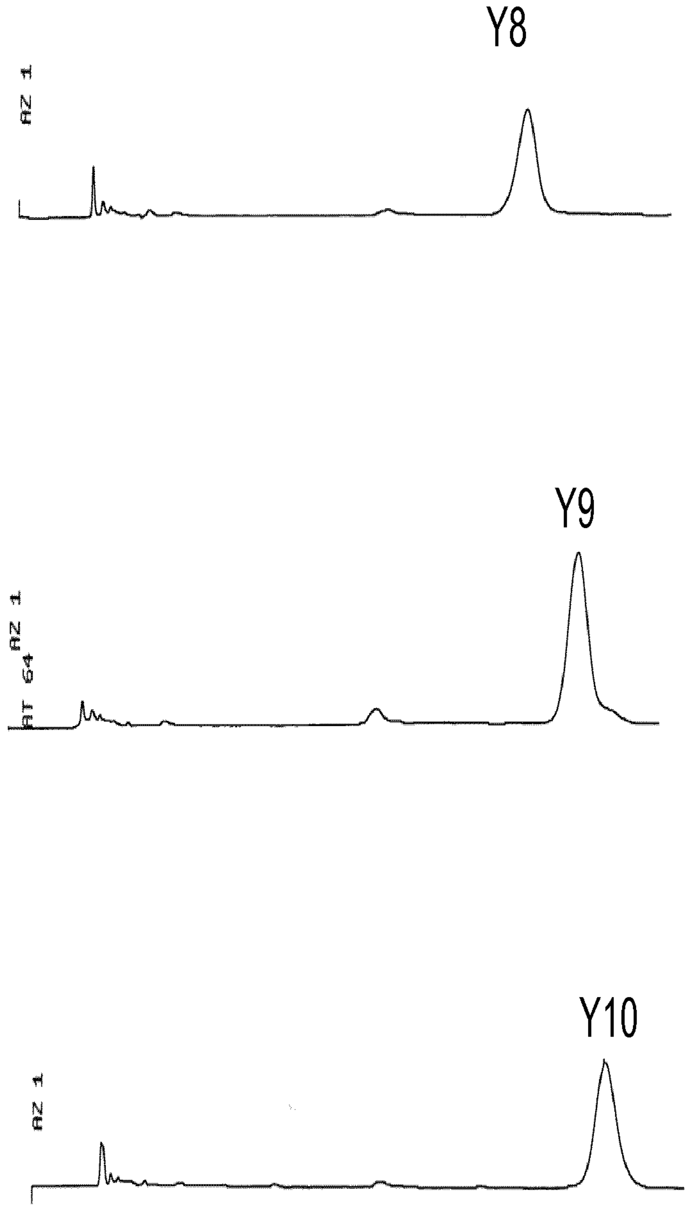

Figure 12
Inhibition of Ovarian cancer growth by plant extract

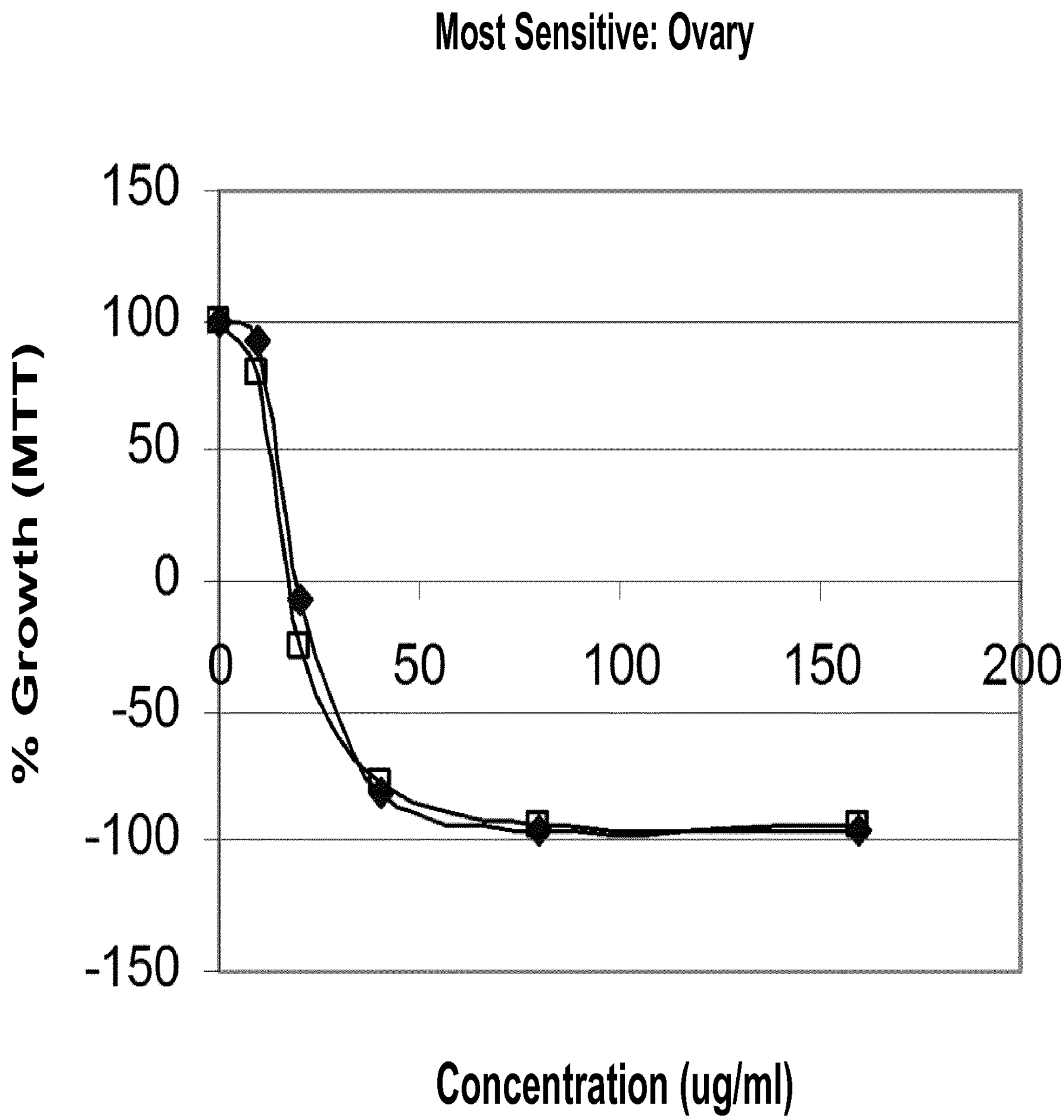

Figure 13
Comparison of Potency of Y in ovary and cervix cancer cells

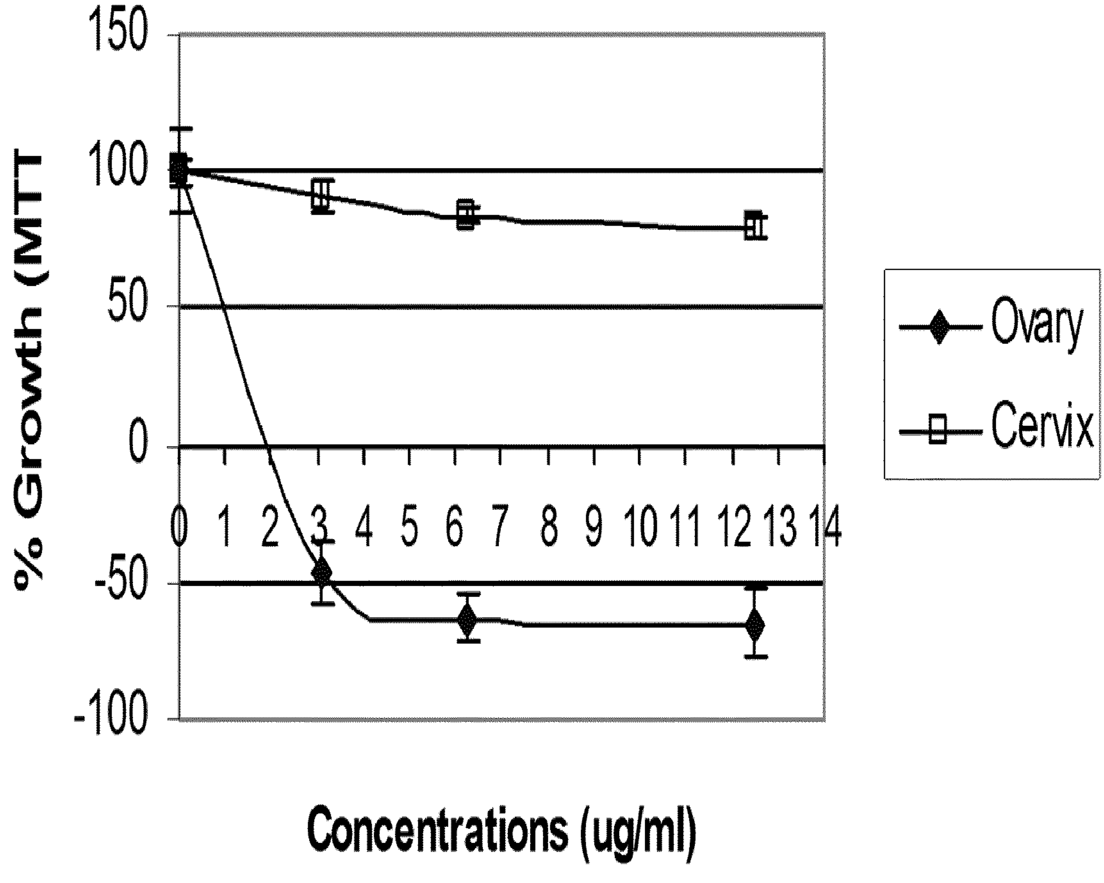

Figure 14A
Growth curve of cancer cells after treatment with extract

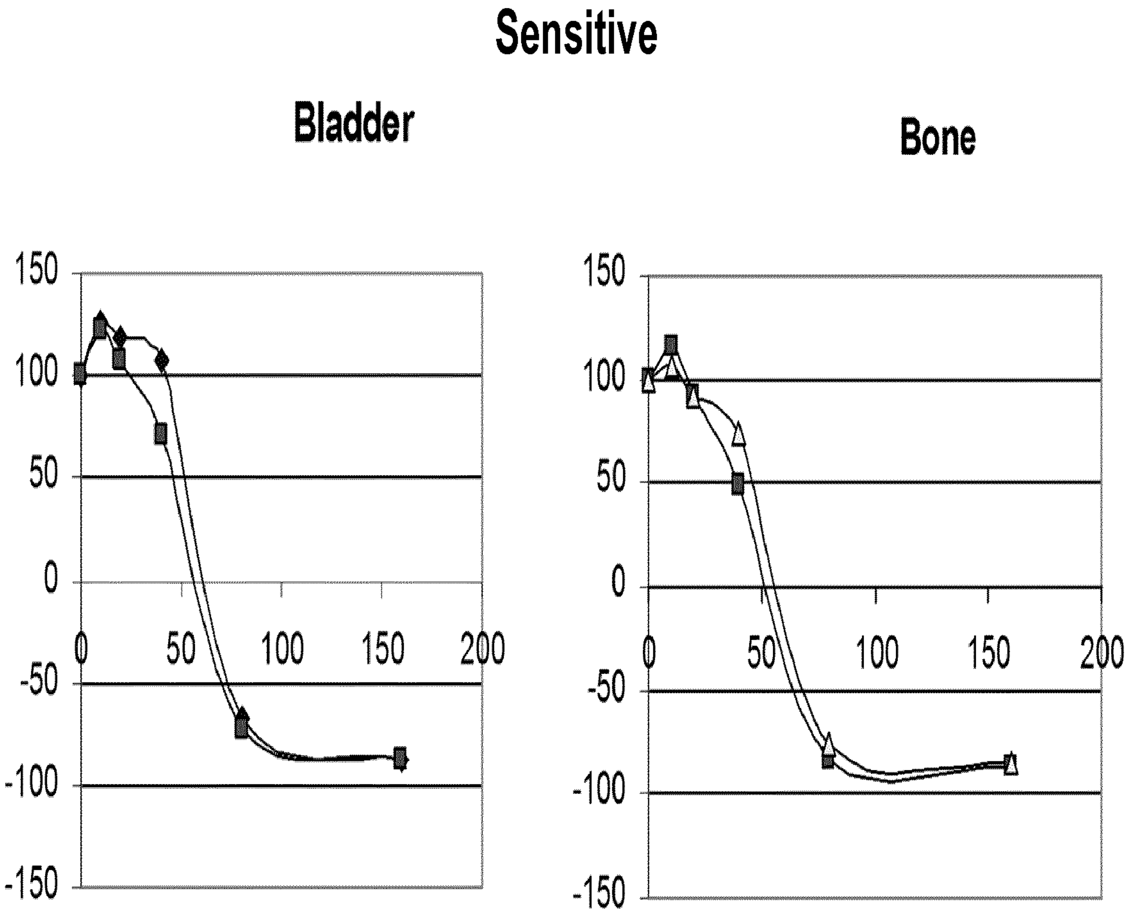

Figure 14B

Growth curve of cancer cells after treatment with extract

**Semi-sensitive**

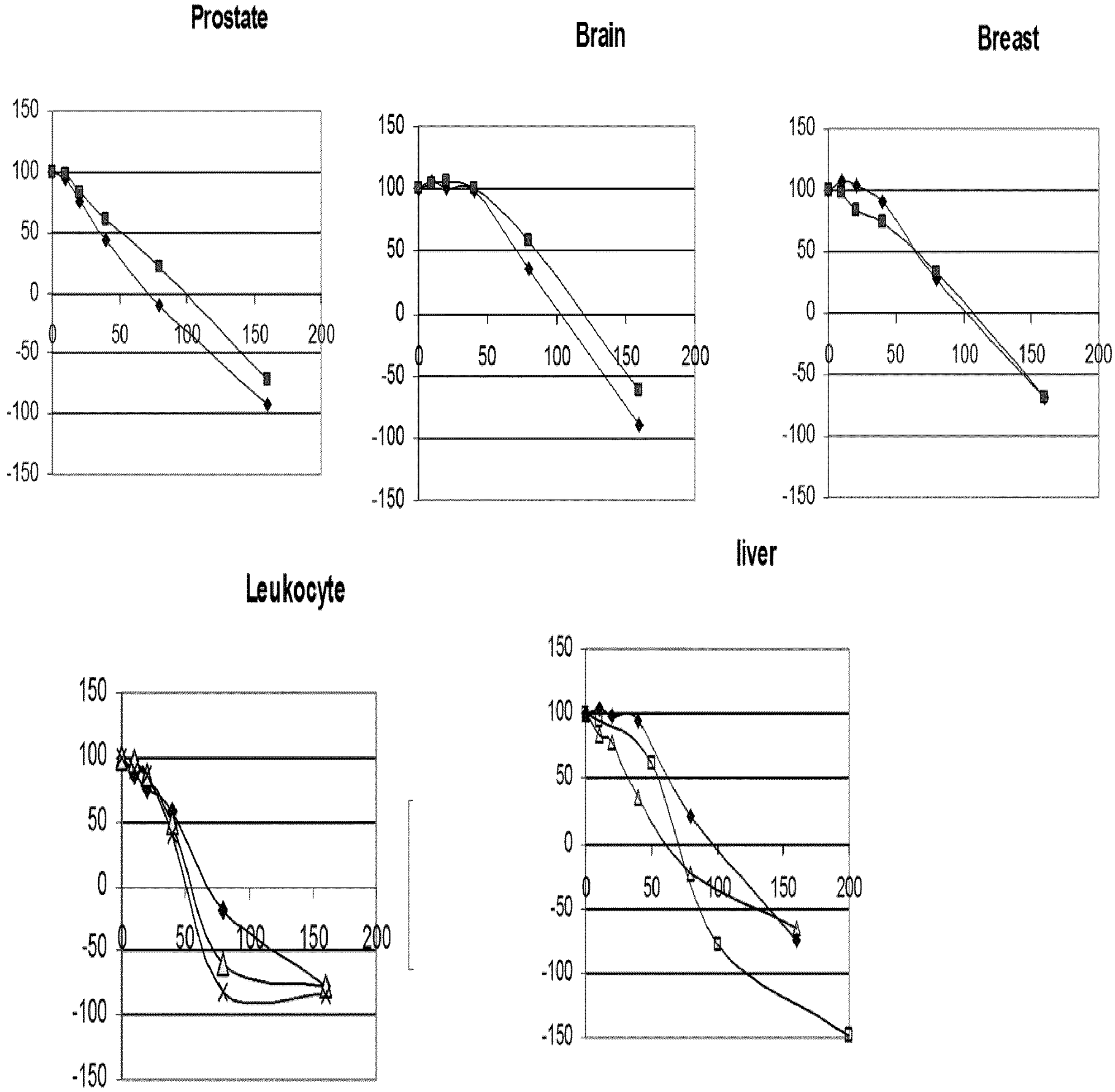

Figure 14C

Growth curve of cancer cells after treatment with extract

## Least sensitive

Colon

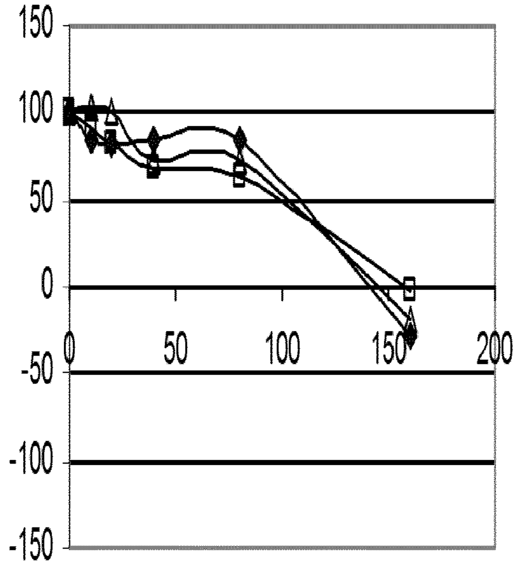


Cervix

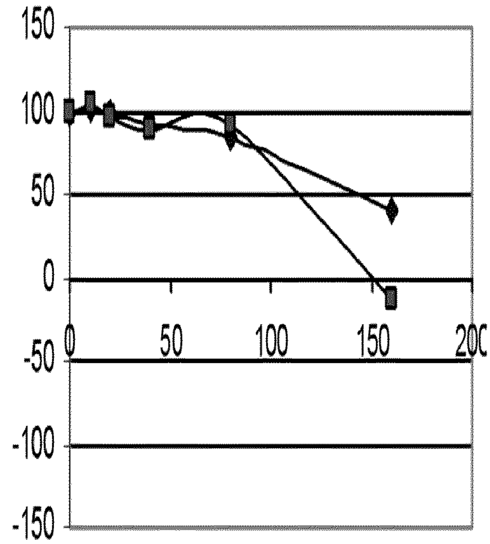


Lung

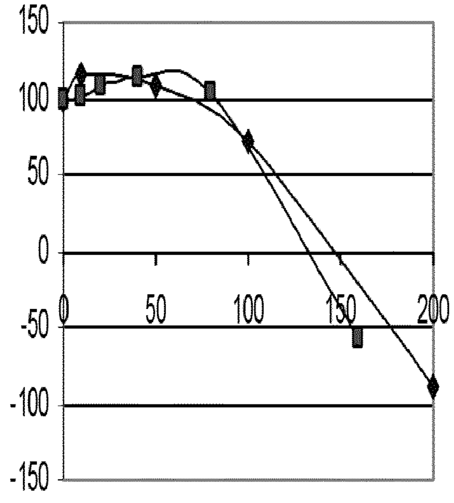

Figure 15
Chemical Structure of compound Y (Y3)

$Y_3$

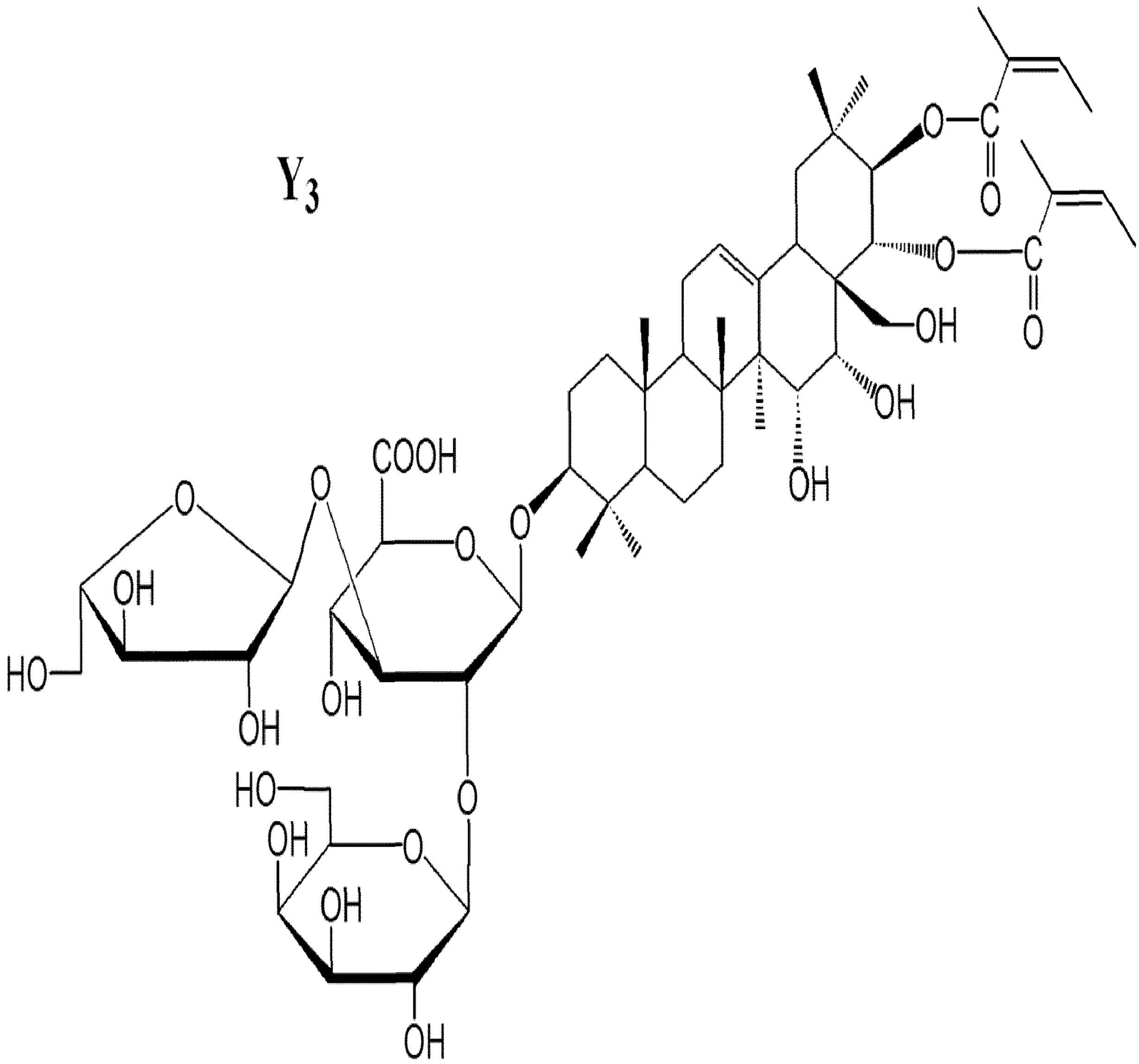

Figure 16
Proton NMR of Y

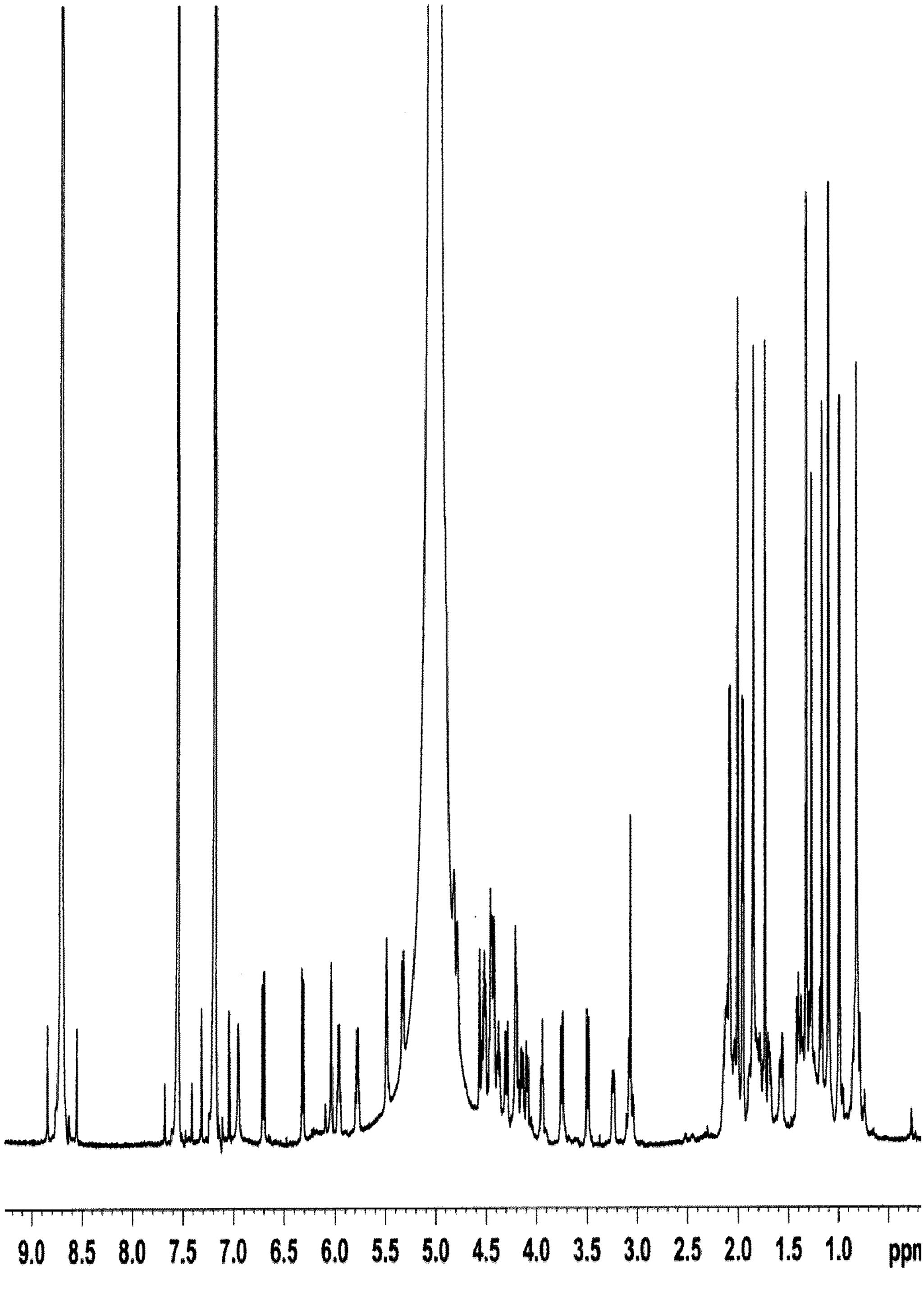

Figure 17
2D NMR (HMQC) of Y

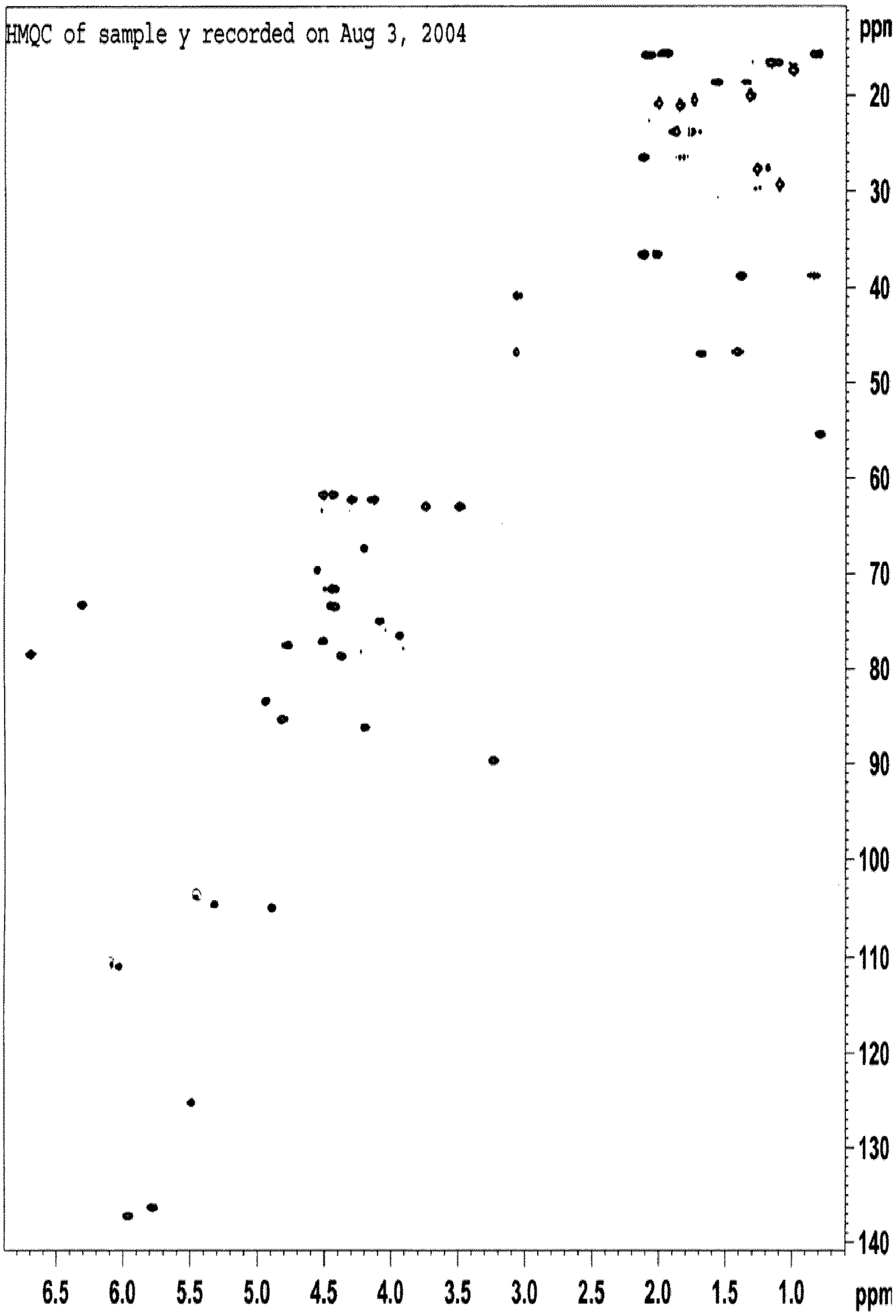

Figure 18
HMBC of Y

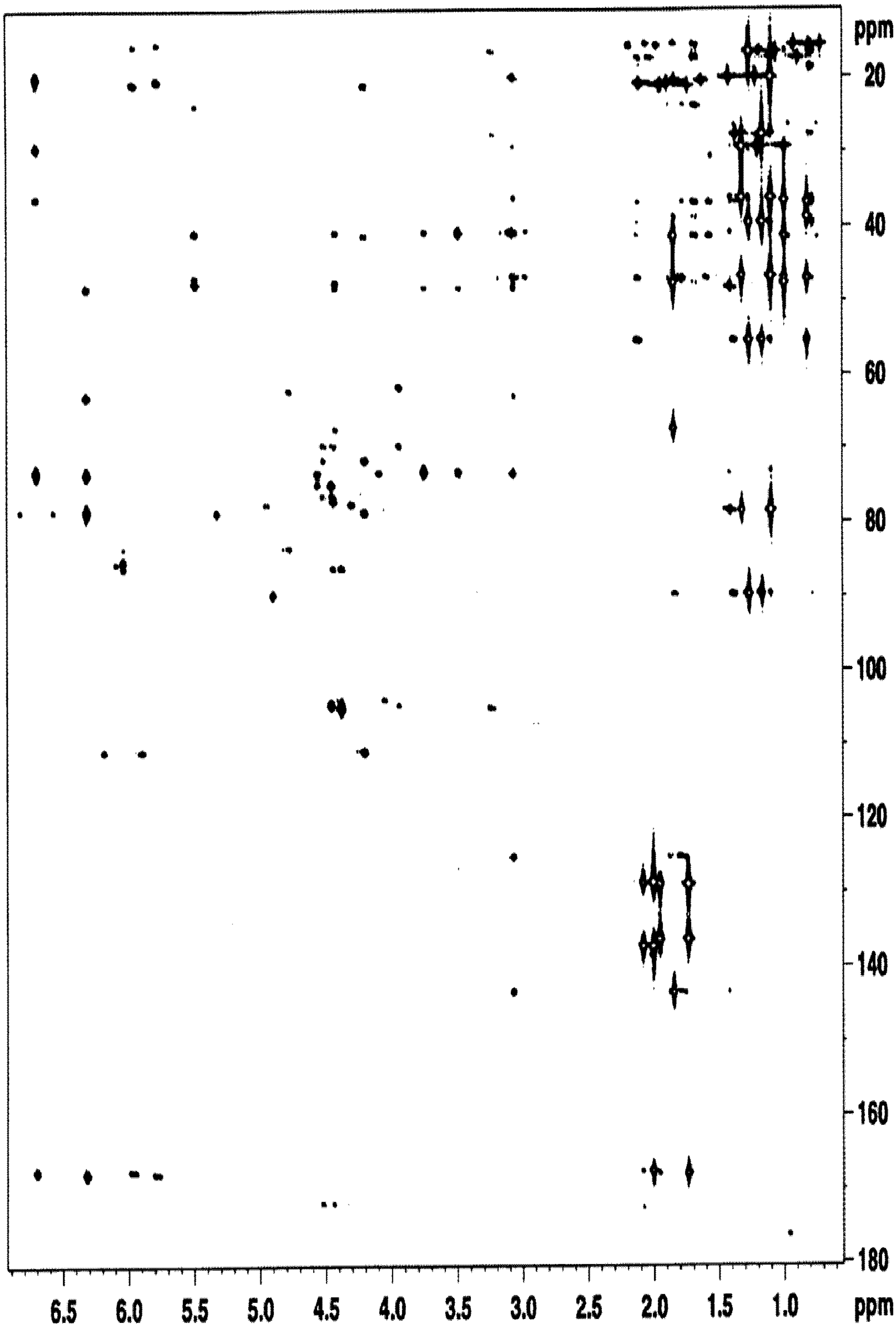

Figure 19
MS (MALDI-TOF) of Y
Y + Matrix (CHCA) + Angiotensin 1 "two point Calibration"

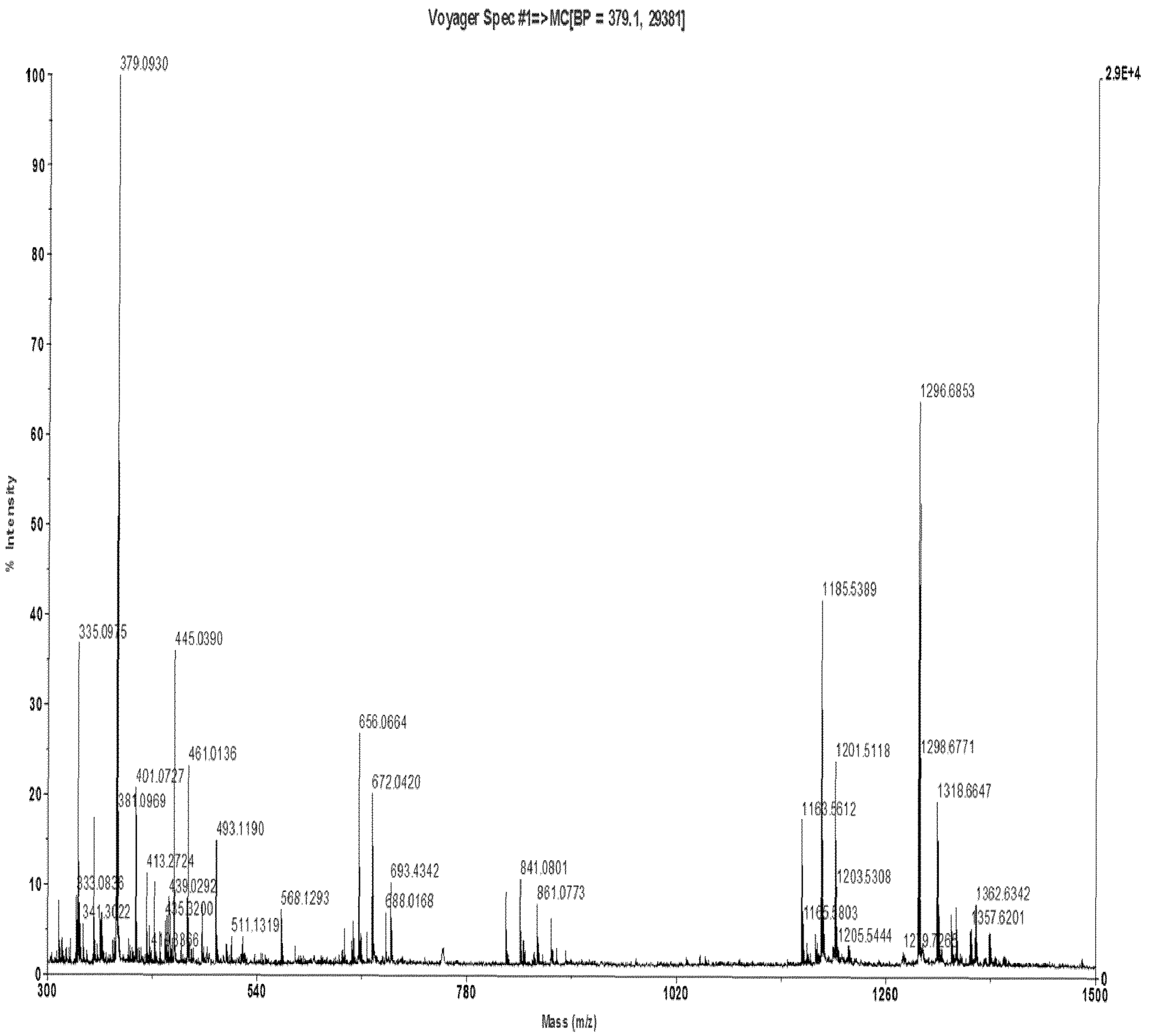

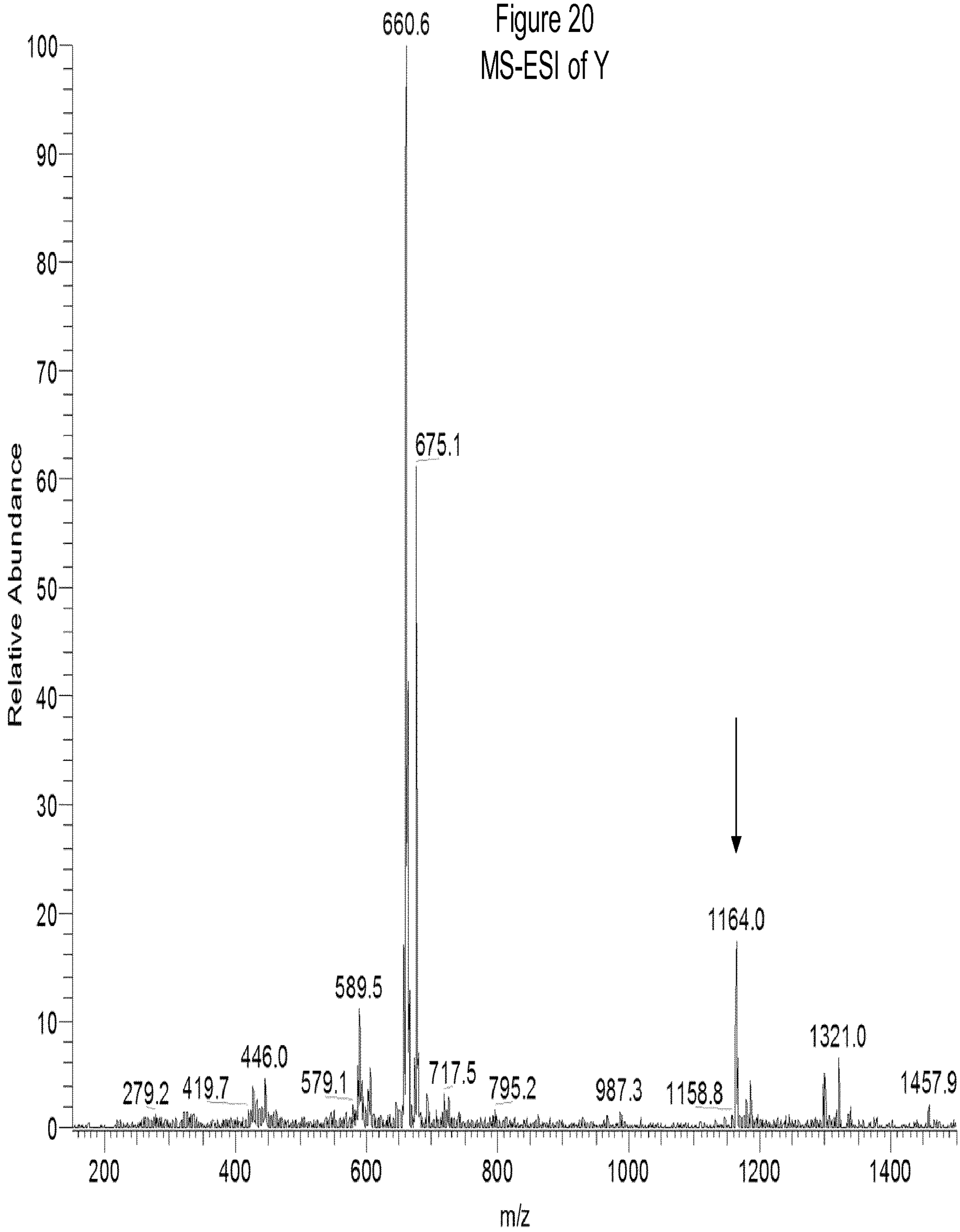
Figure 20
MS-ESI of Y
660.6
675.1
589.5
446.0
279.2
419.7
579.1
717.5
795.2
987.3
1158.8
1164.0
1321.0
1457.9
Relative Abundance
m/z
0
10
20
30
40
50
60
70
80
90
100
200
400
600
800
1000
1200
1400

Figure 21
Chemical Structure of Y1

Y1

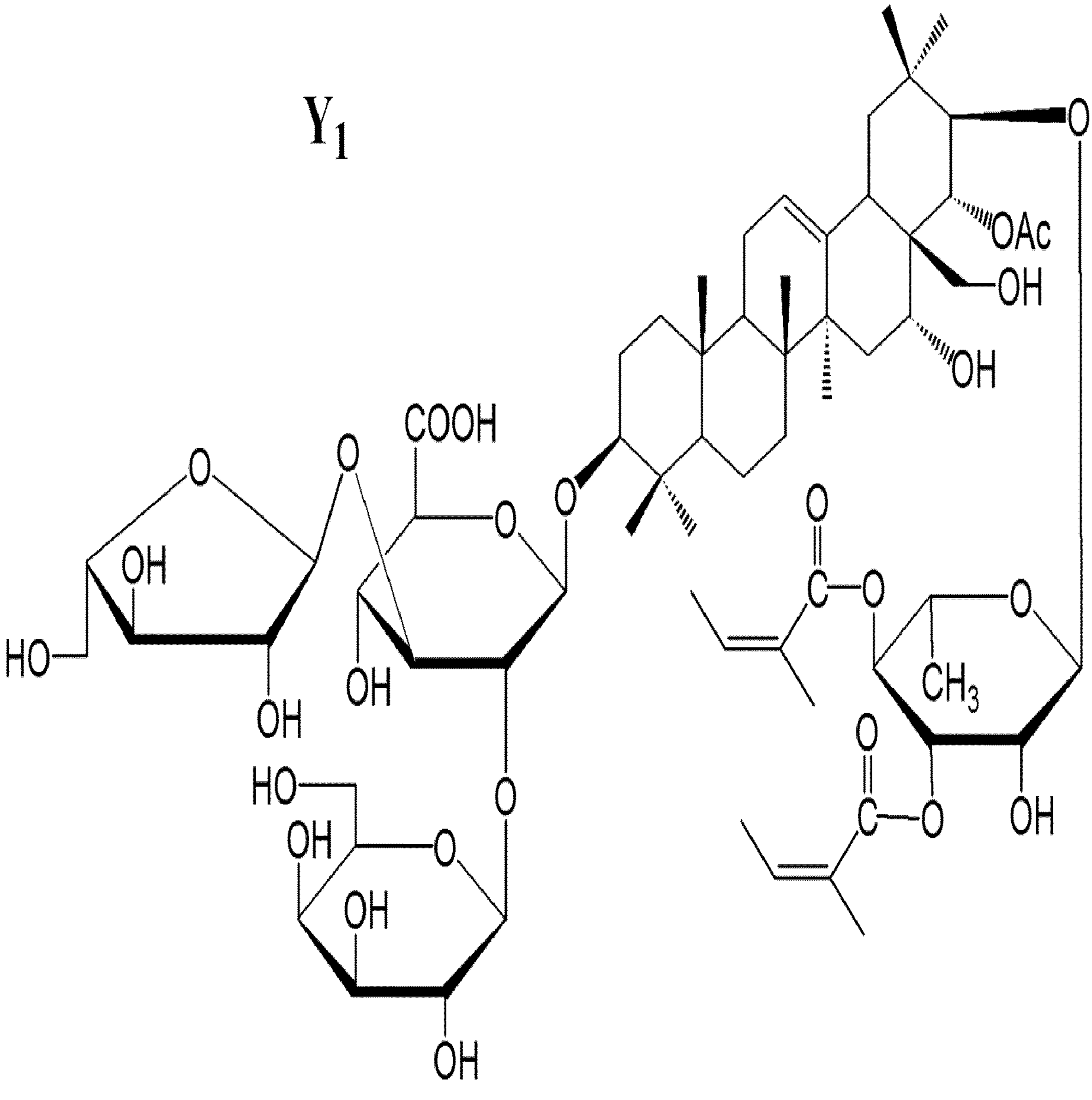

Figure 22
Proton NMR of Y1

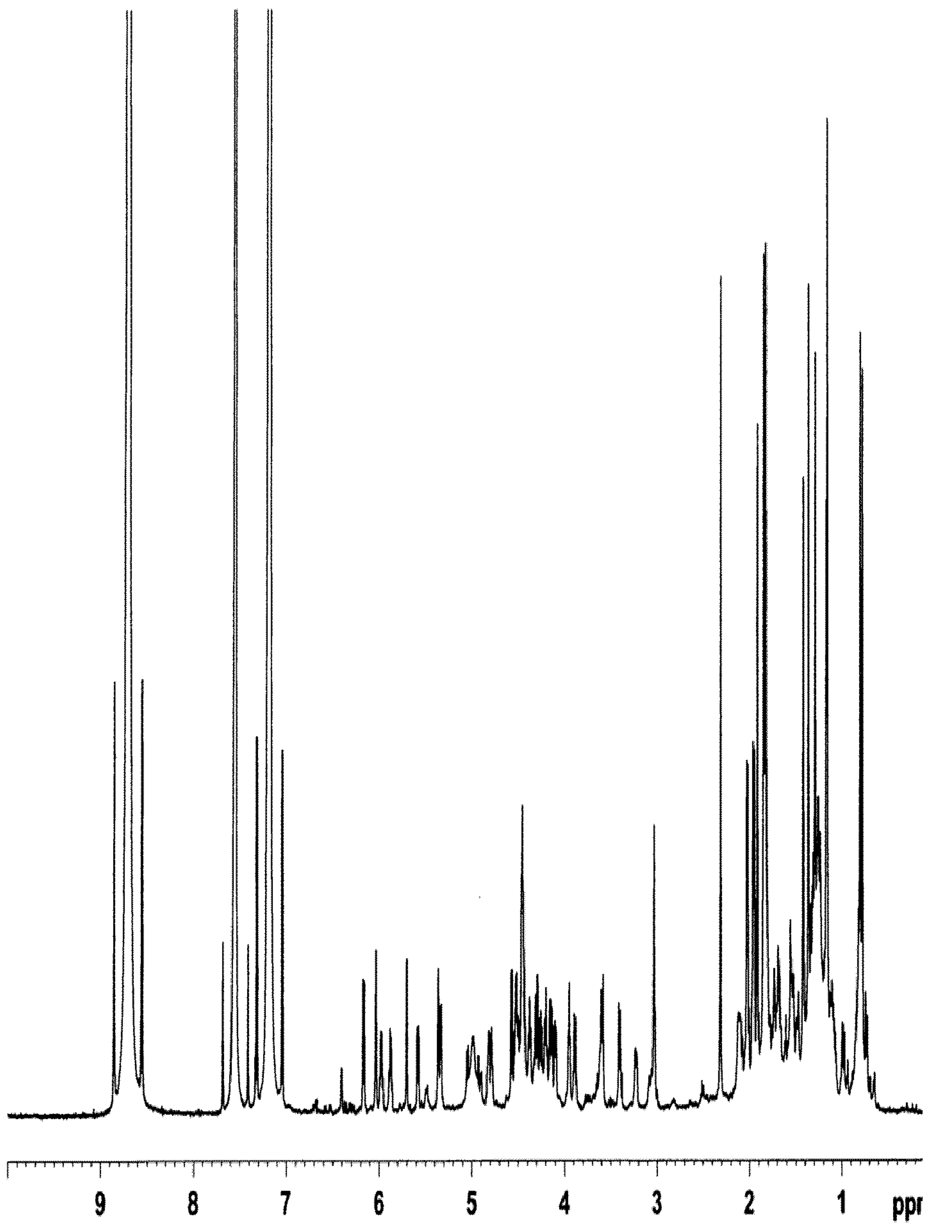

Figure 23
HMQC of Y1

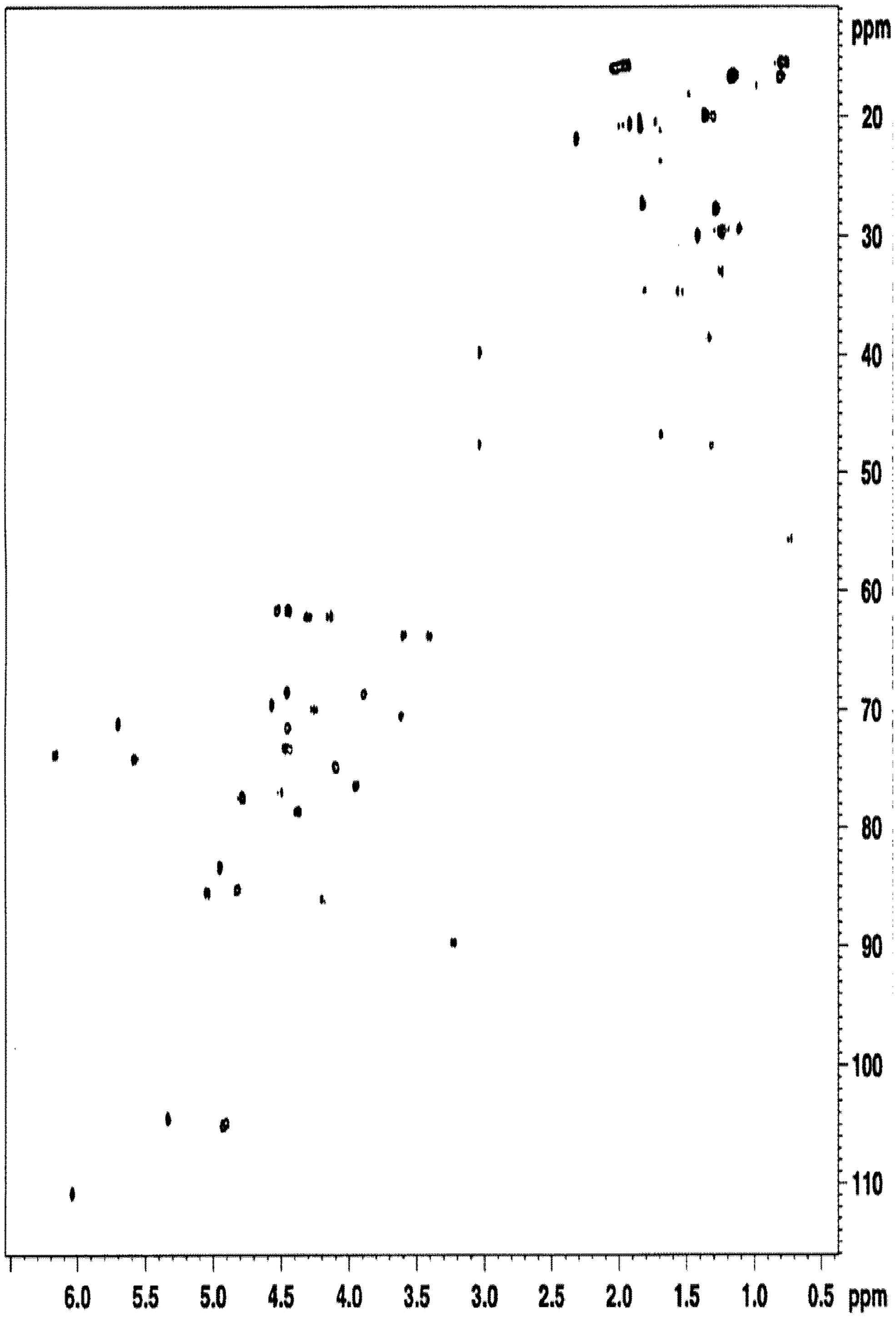

Figure 24
HMBC of Y1

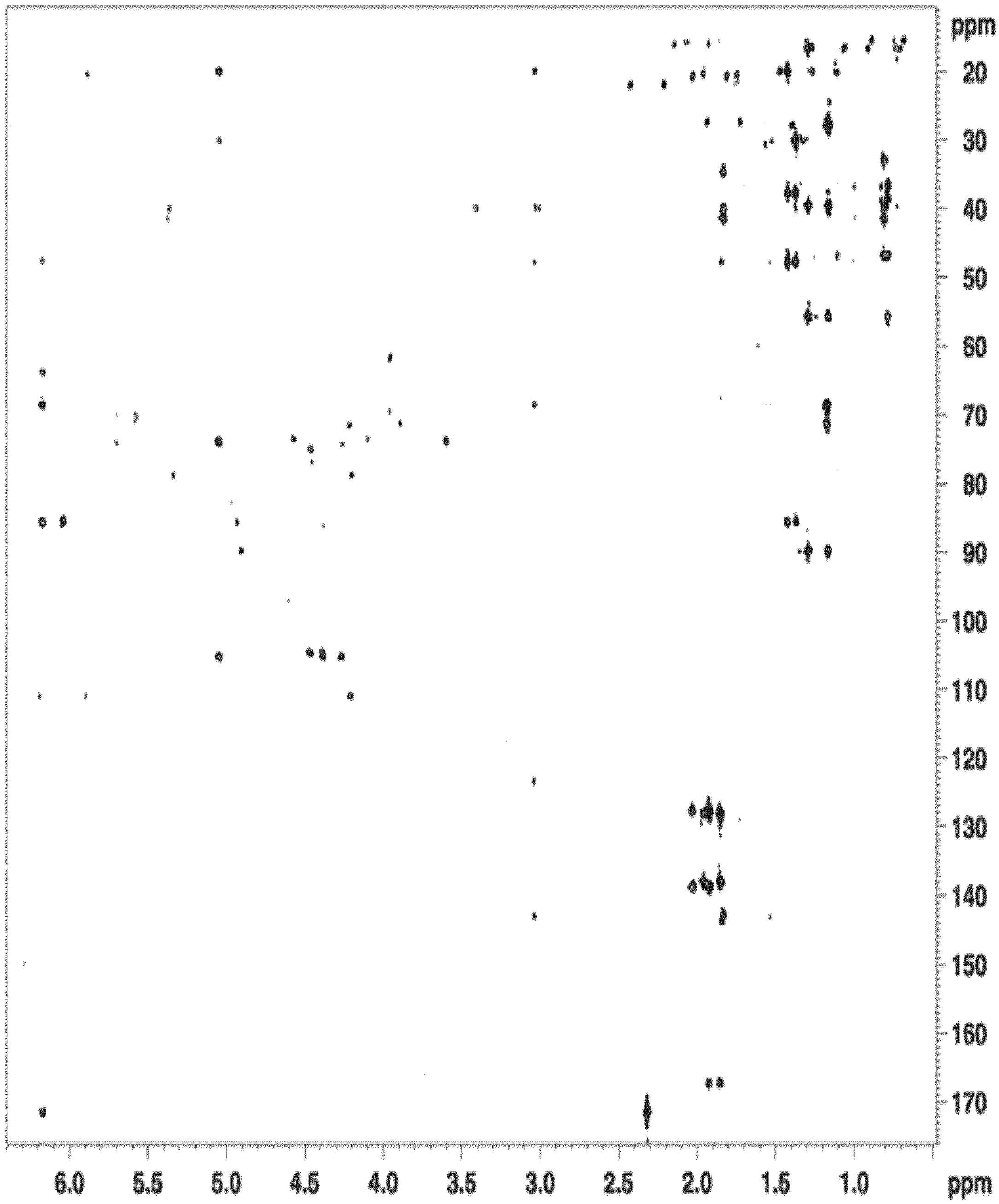

Figure 25
2D NMR (COSY) of Y1

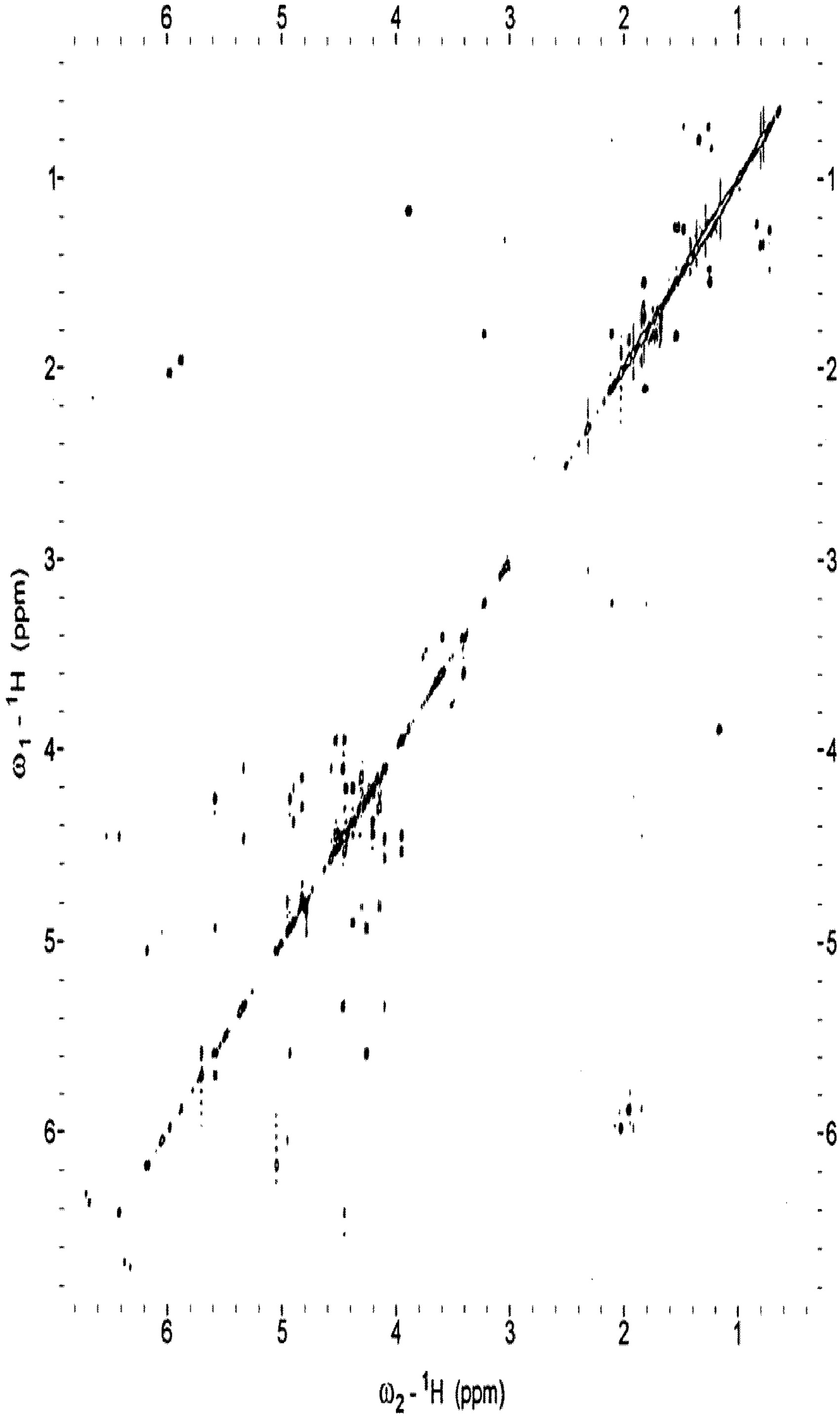

Figure 26.
Chemical Structure of Y2

$Y_2$

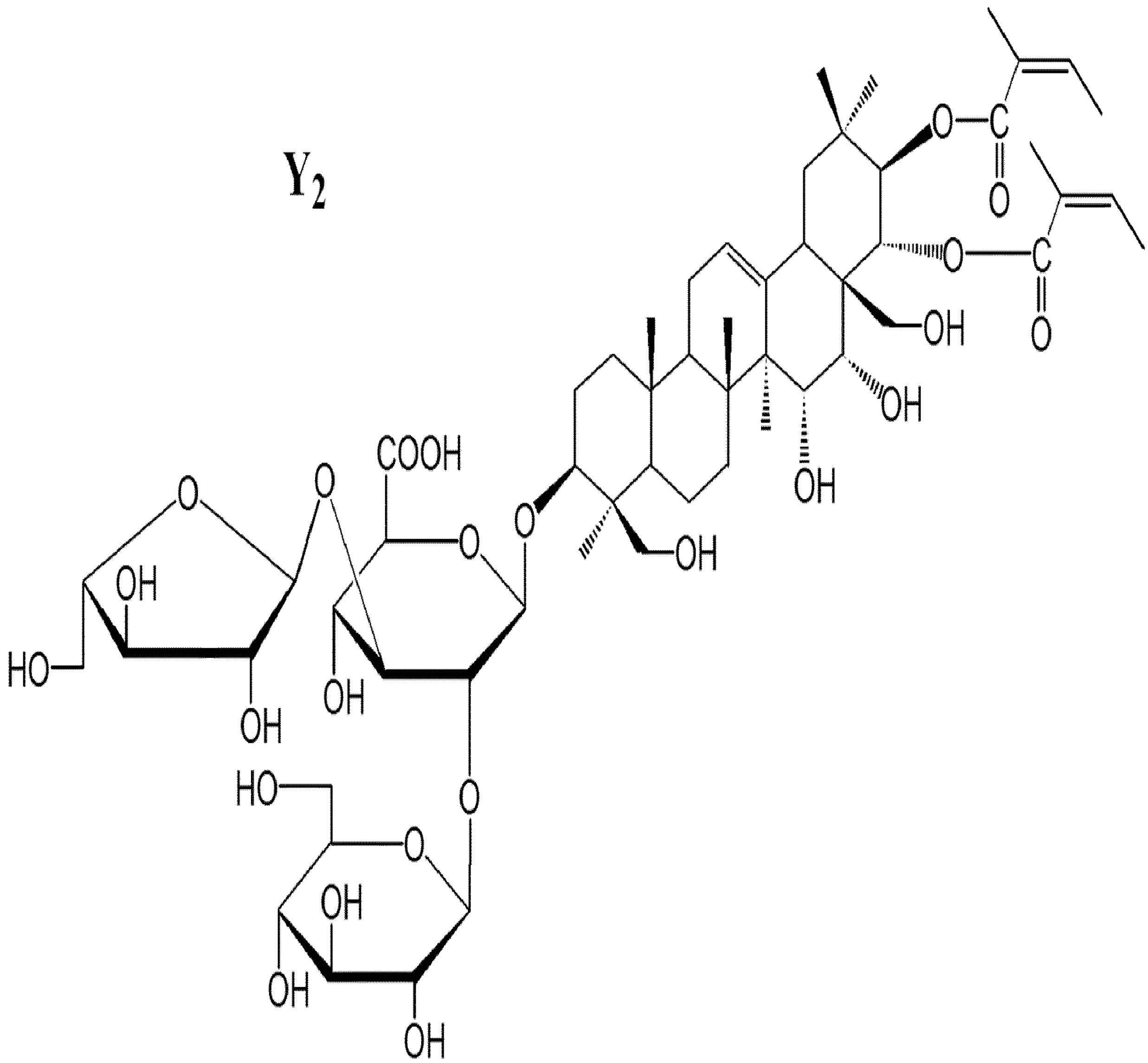

Figure 27
H-NMR Spectrum of Y2

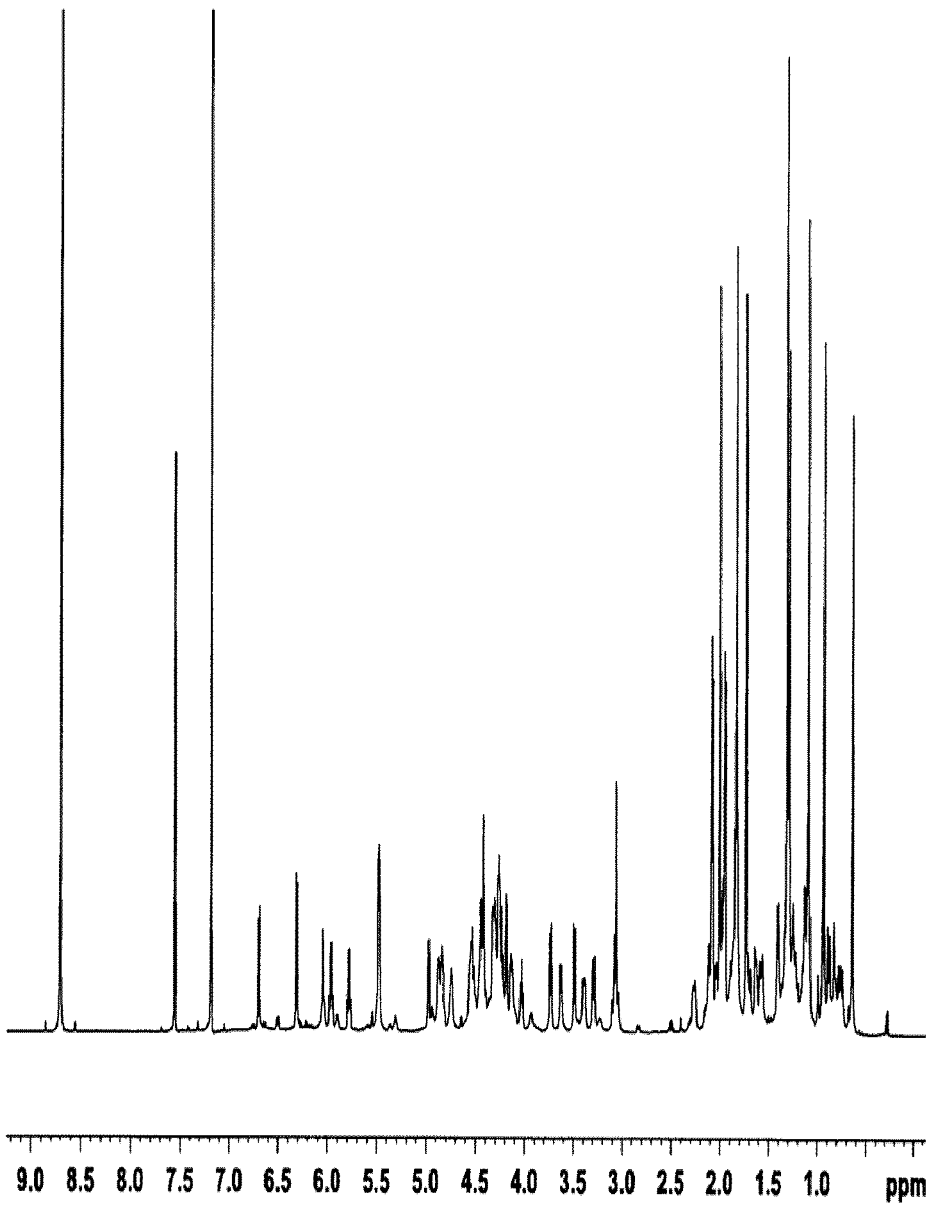

Figure 28
Y2 HMQC-level-1

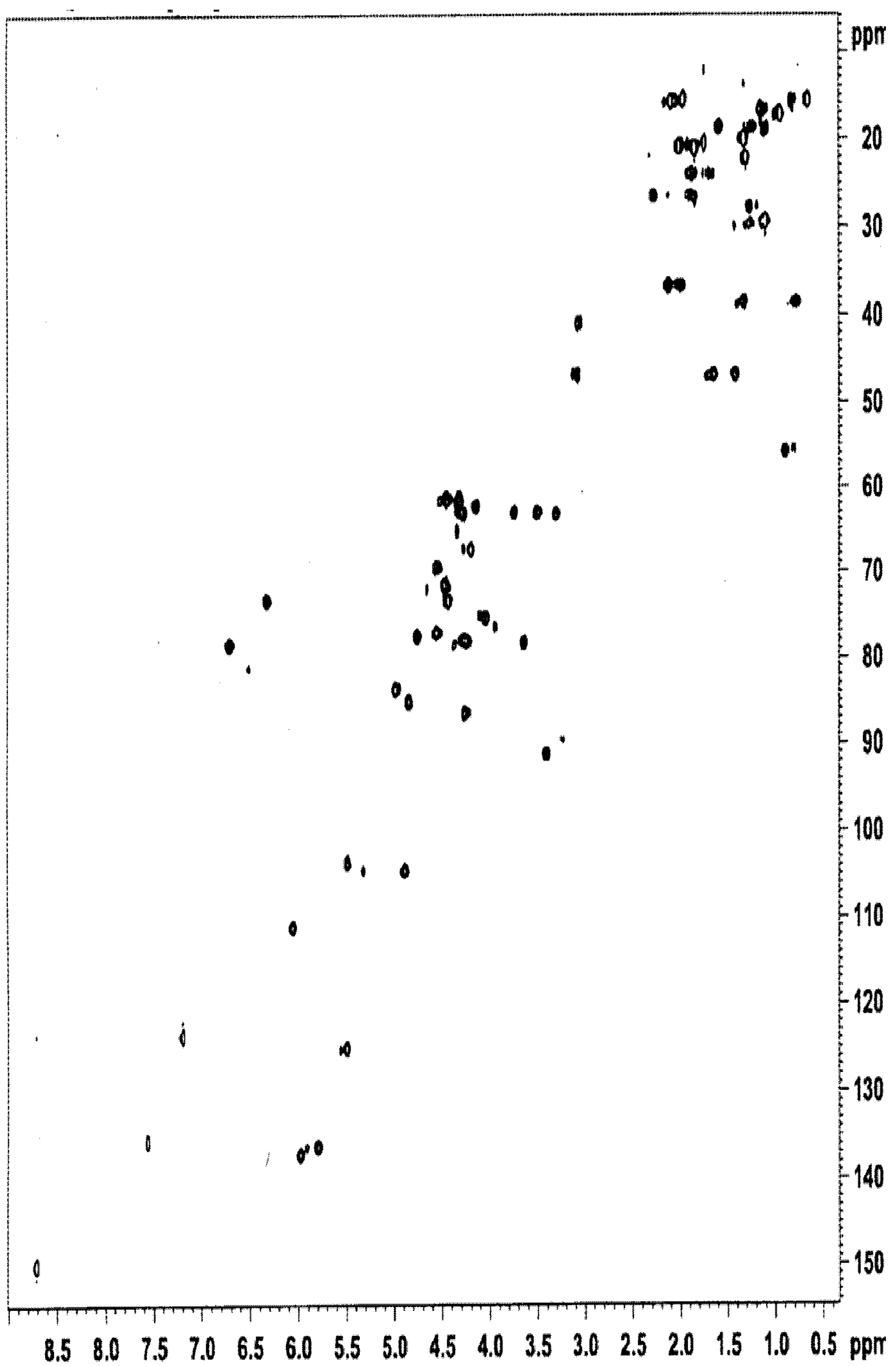

Figure 29

Y2 C13 H-NMR spectrum

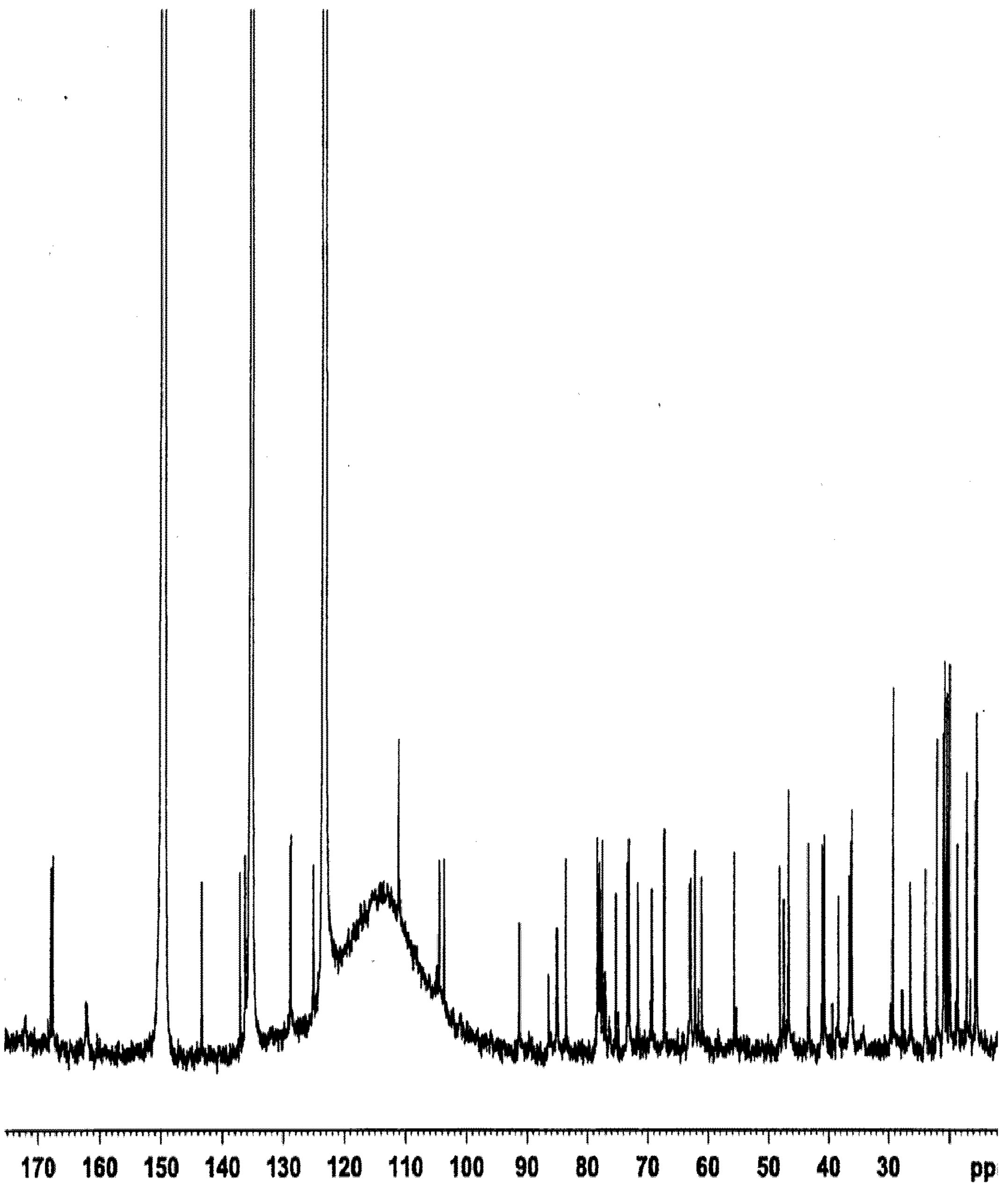

Figure 30
Y2-HMBC-level 1

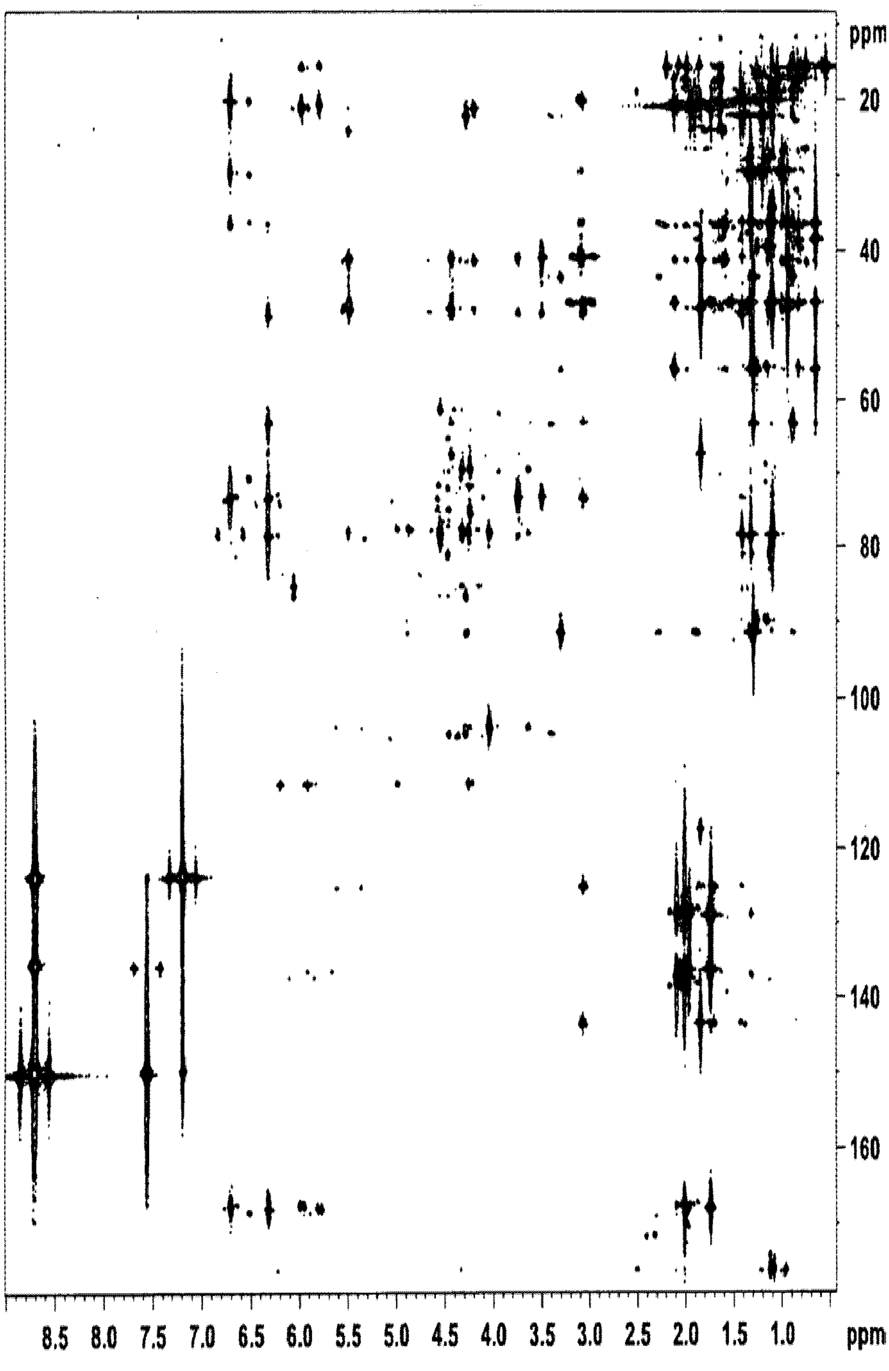

Figure 31
Y2-HOHAHA-level-1

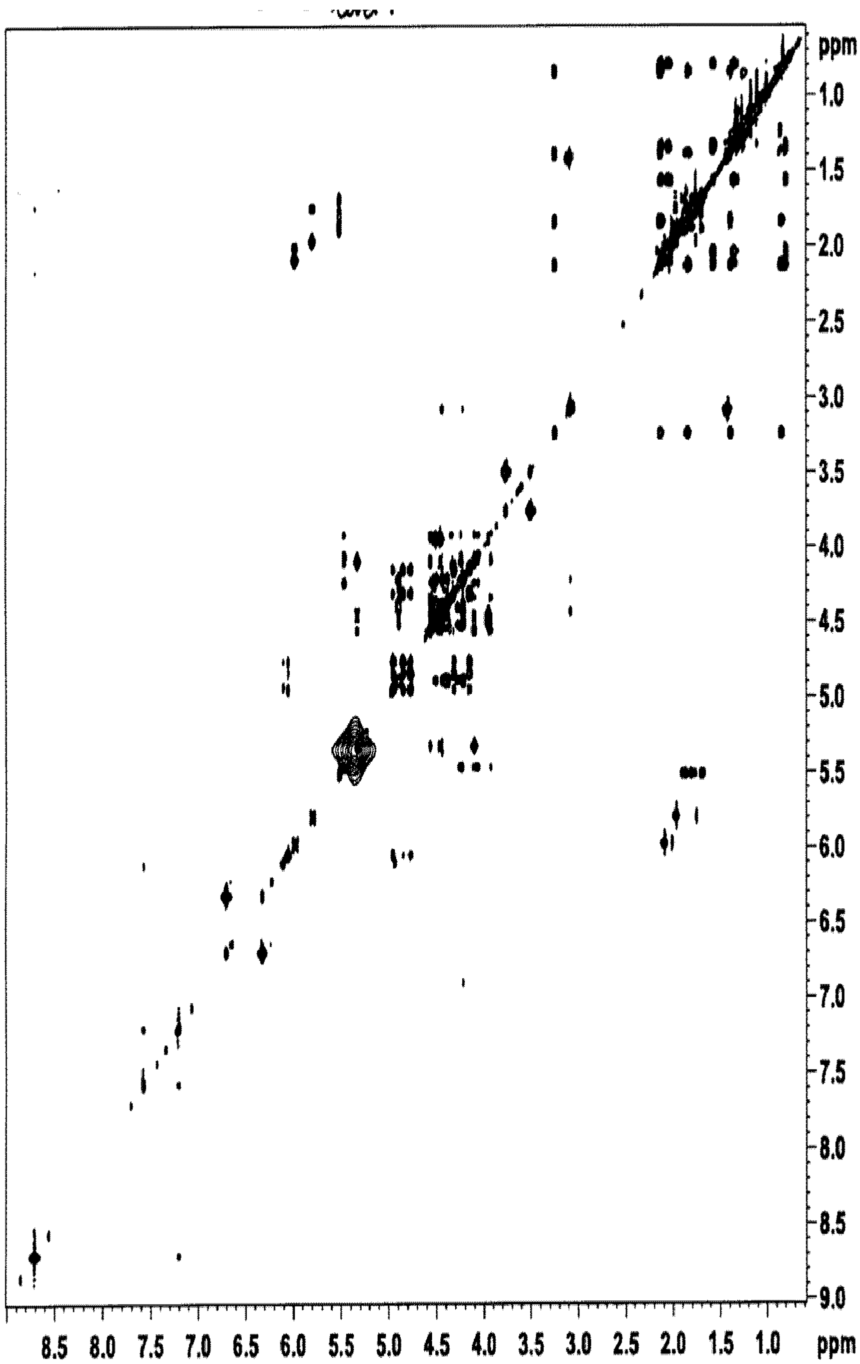

Figure 32
Mass spectrum of Y2 + Matrix + Standards

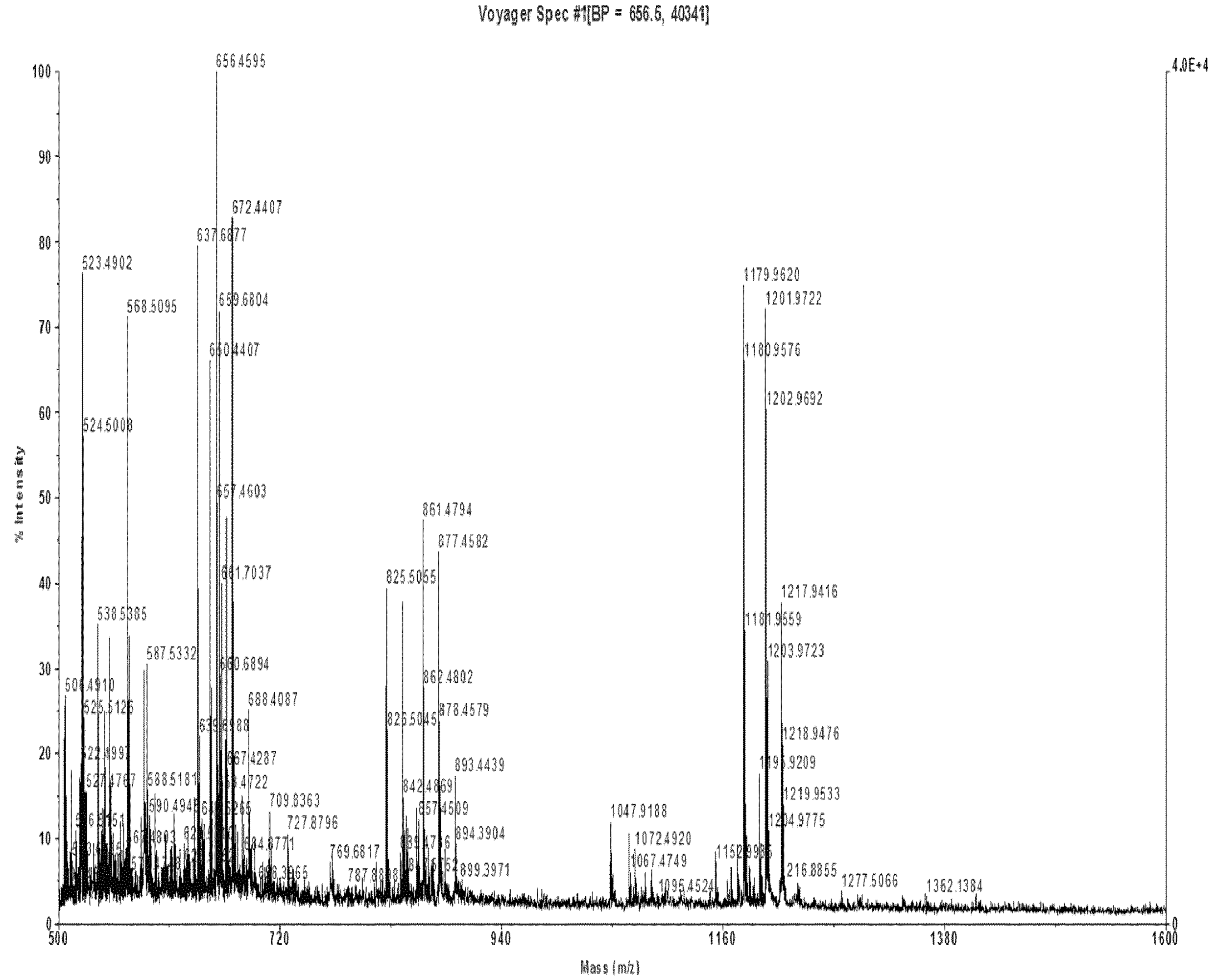

Figure 33
Structure of Y8

Y-8

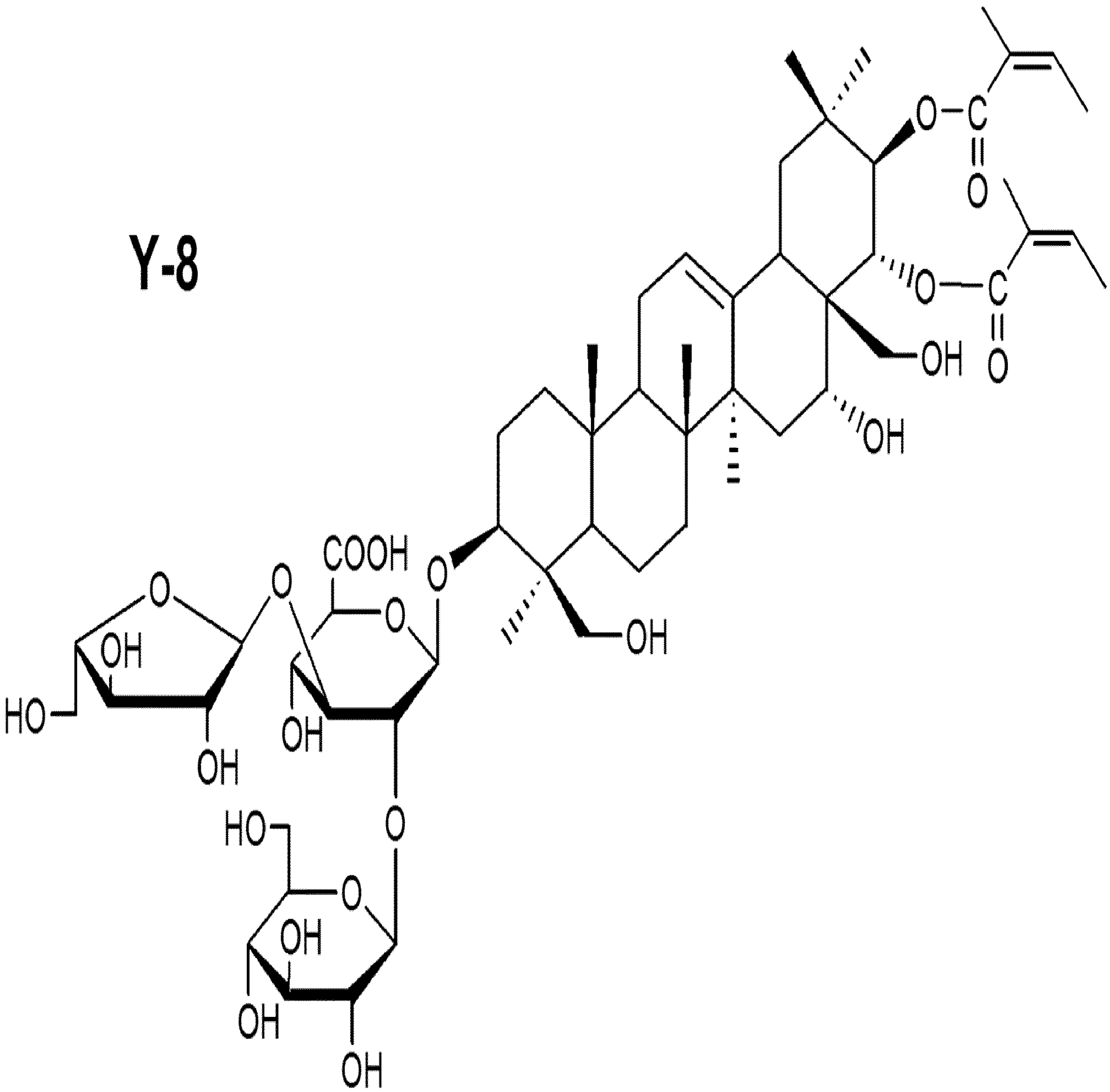

Figure 34
Y8-H-NMR-full

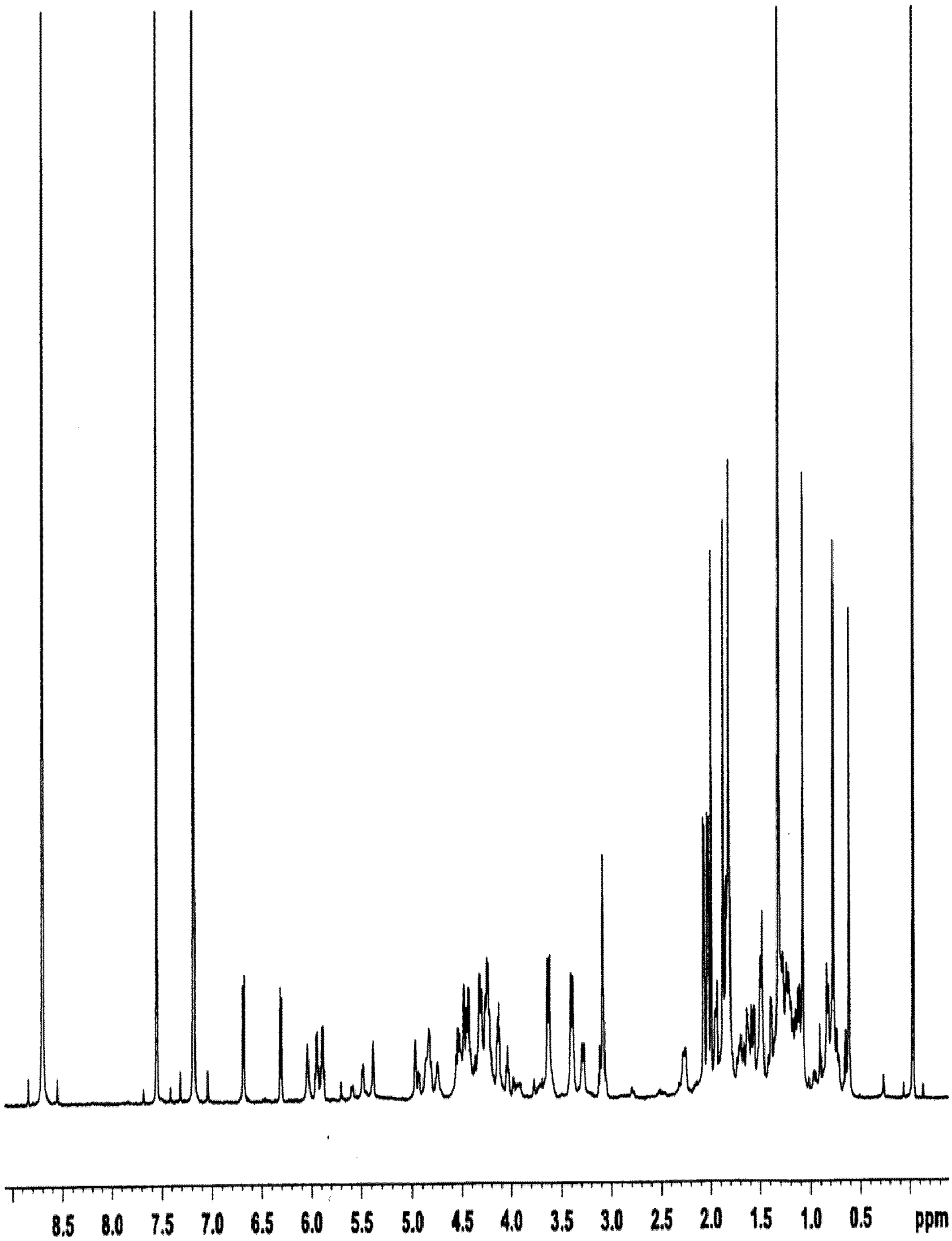

Figure 35
Y8-C13 NMR

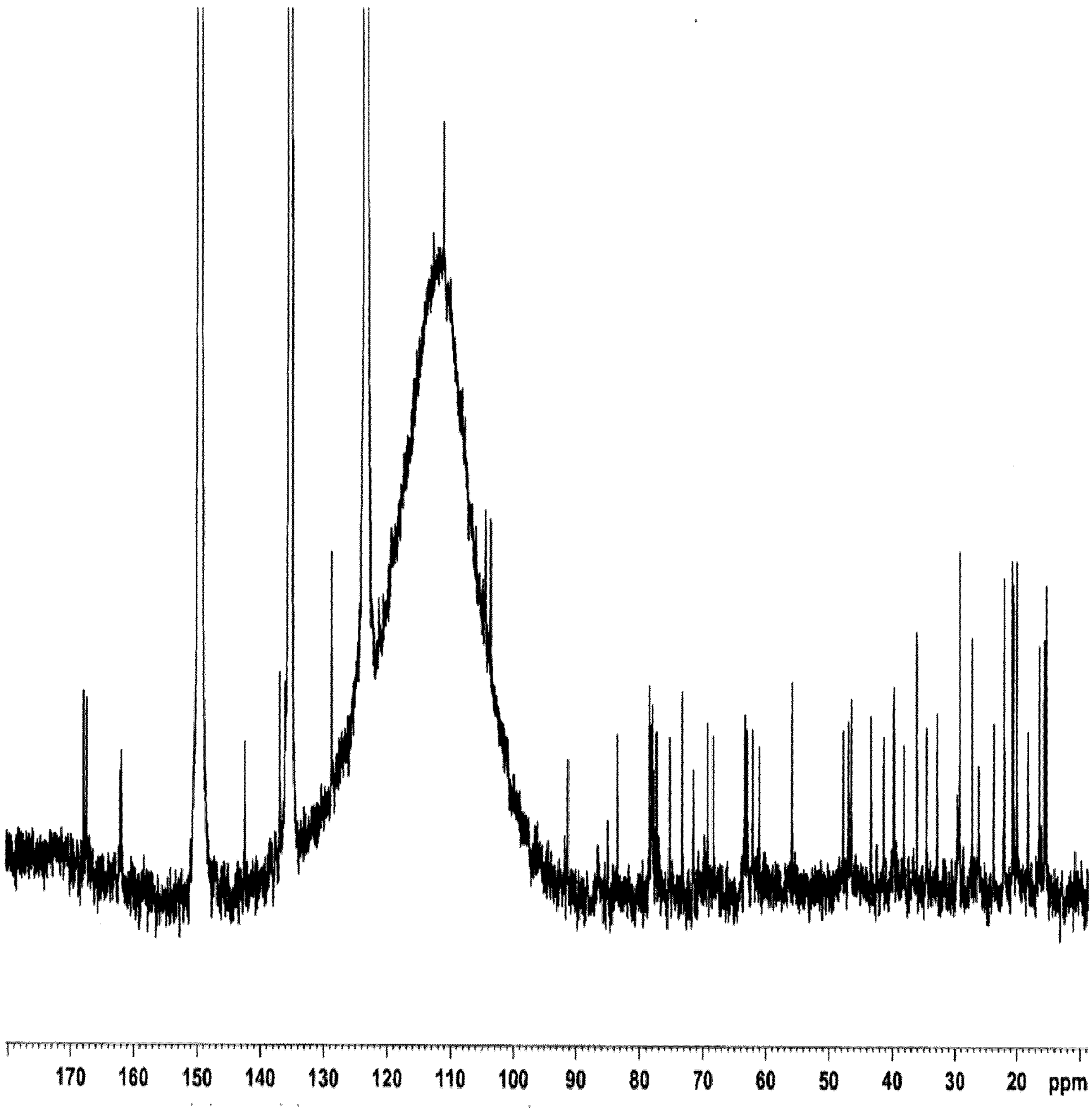

Figure 36
Y8-HMQC-level-1

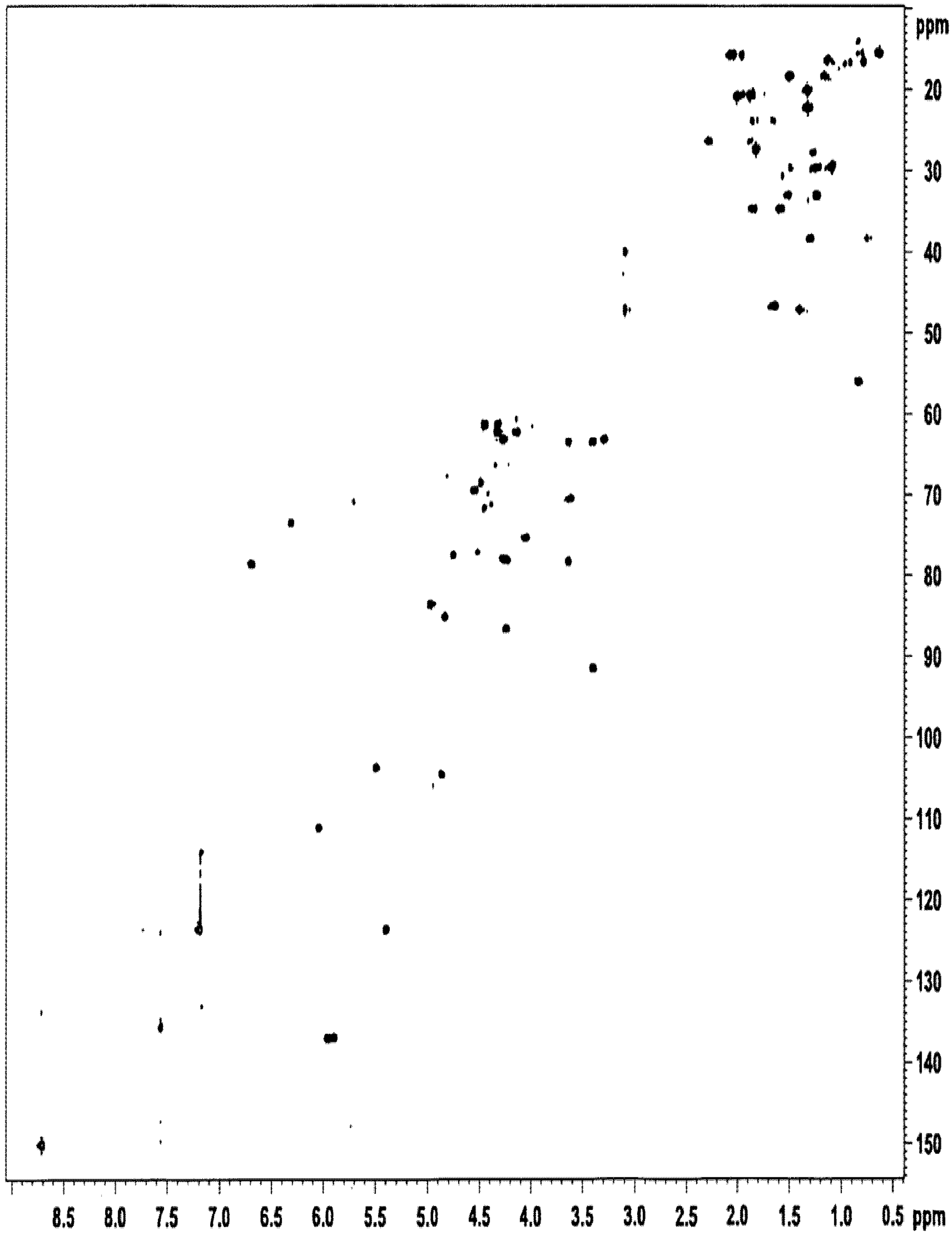

Figure 37
Structure of Y9

$Y_9$

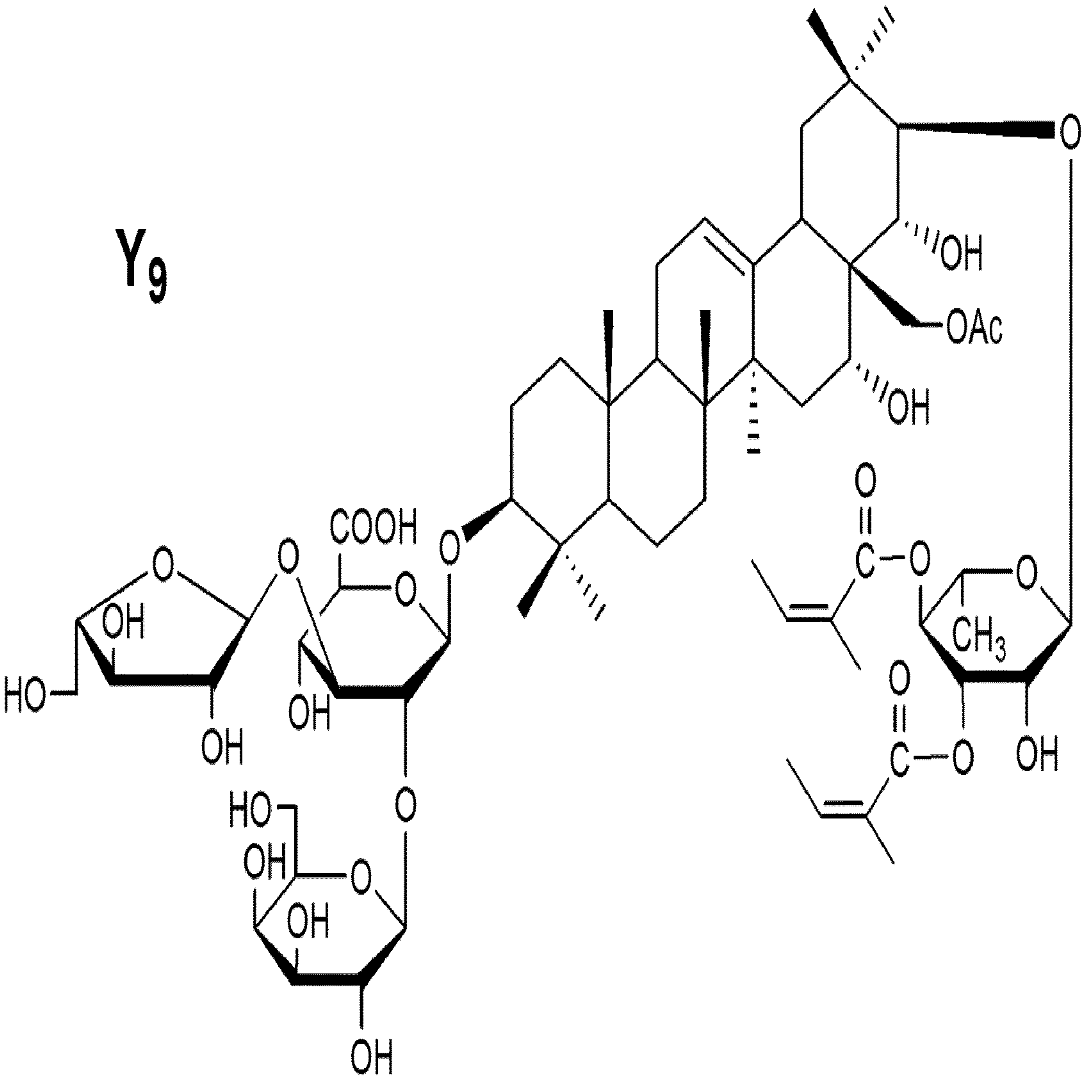

Figure 38

Y9-H-NMR

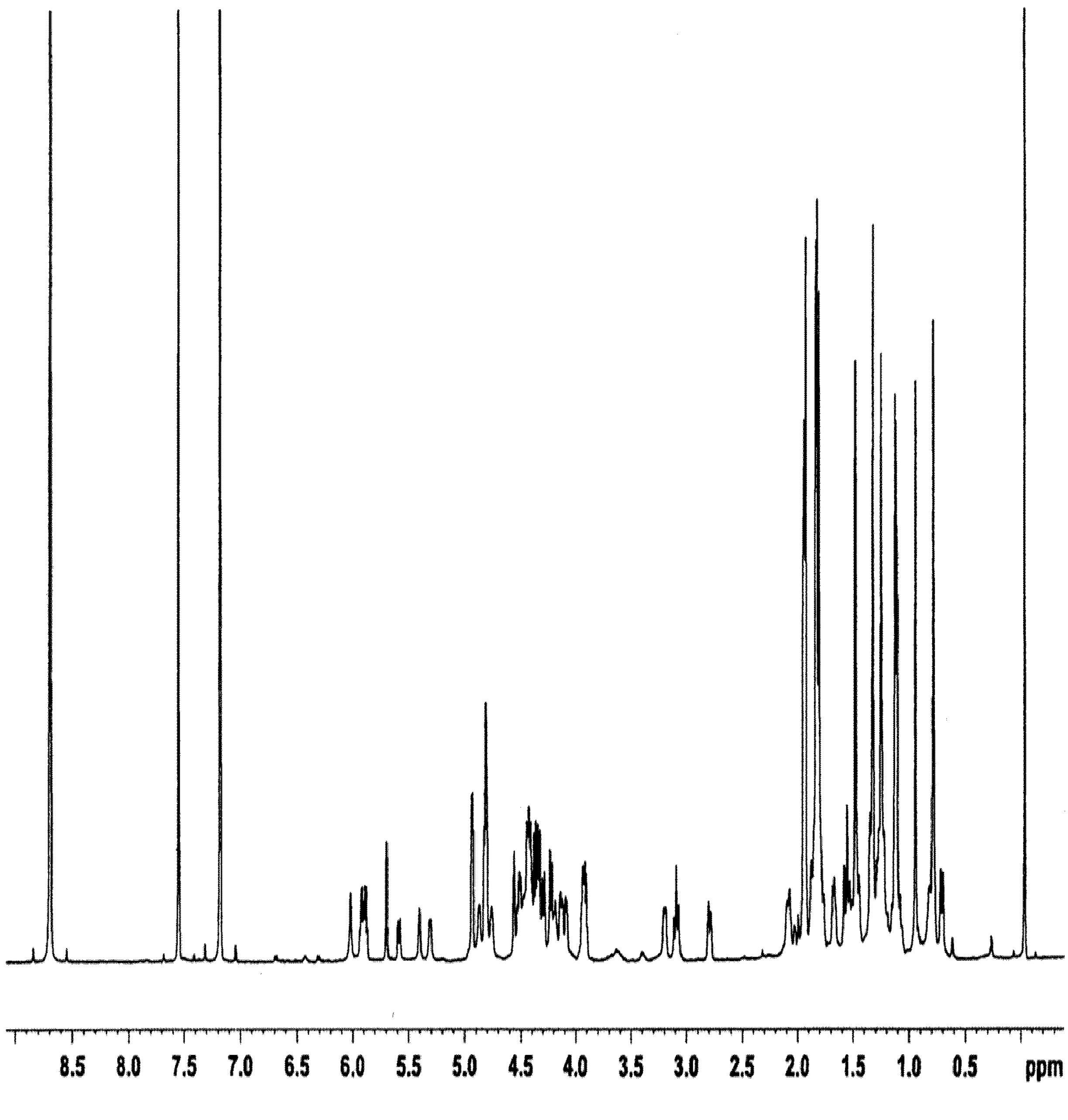

Figure 39
Y9-HMQC-level-1

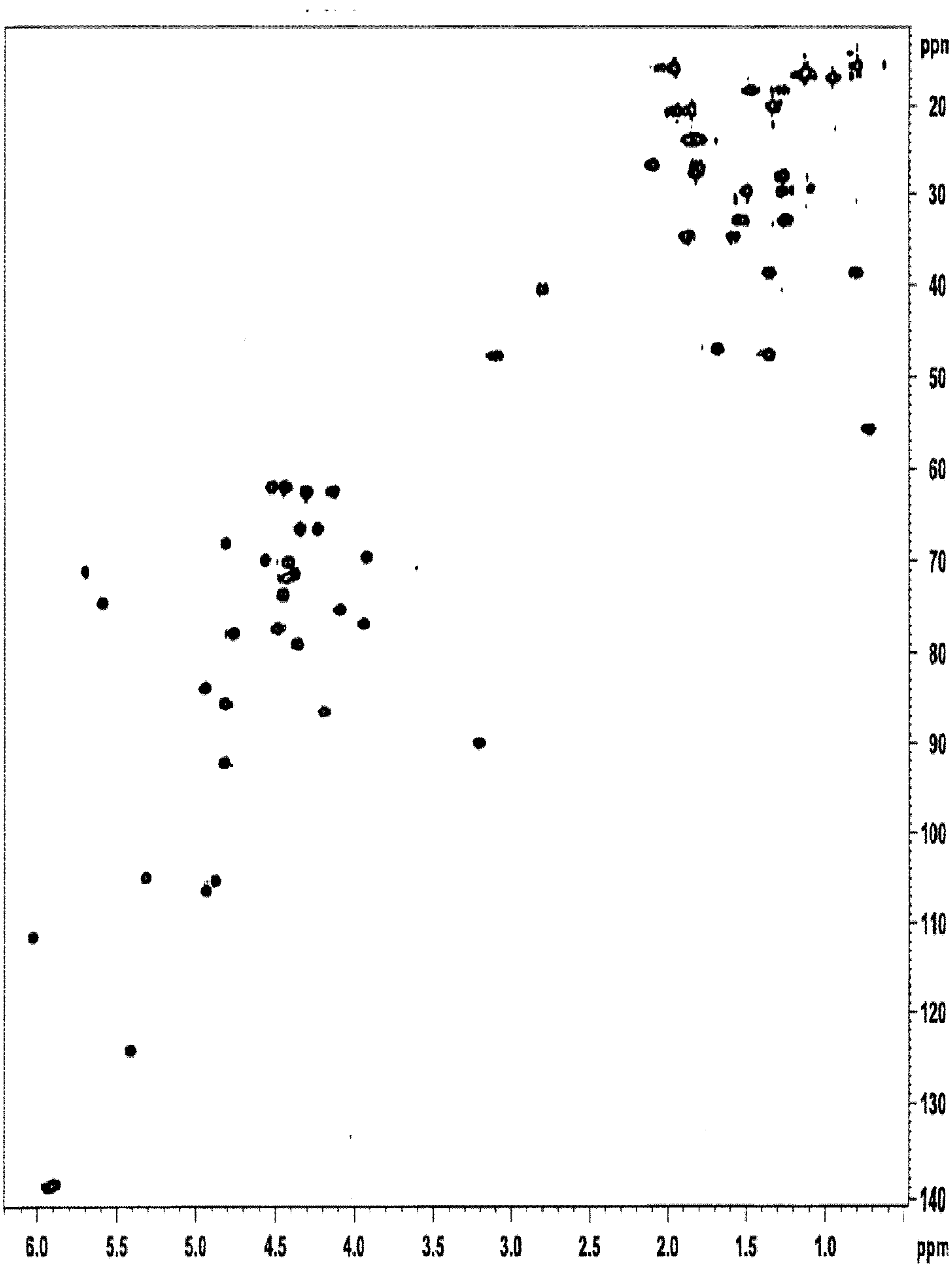

Figure 40
Y9 2D NMR (HMBC)

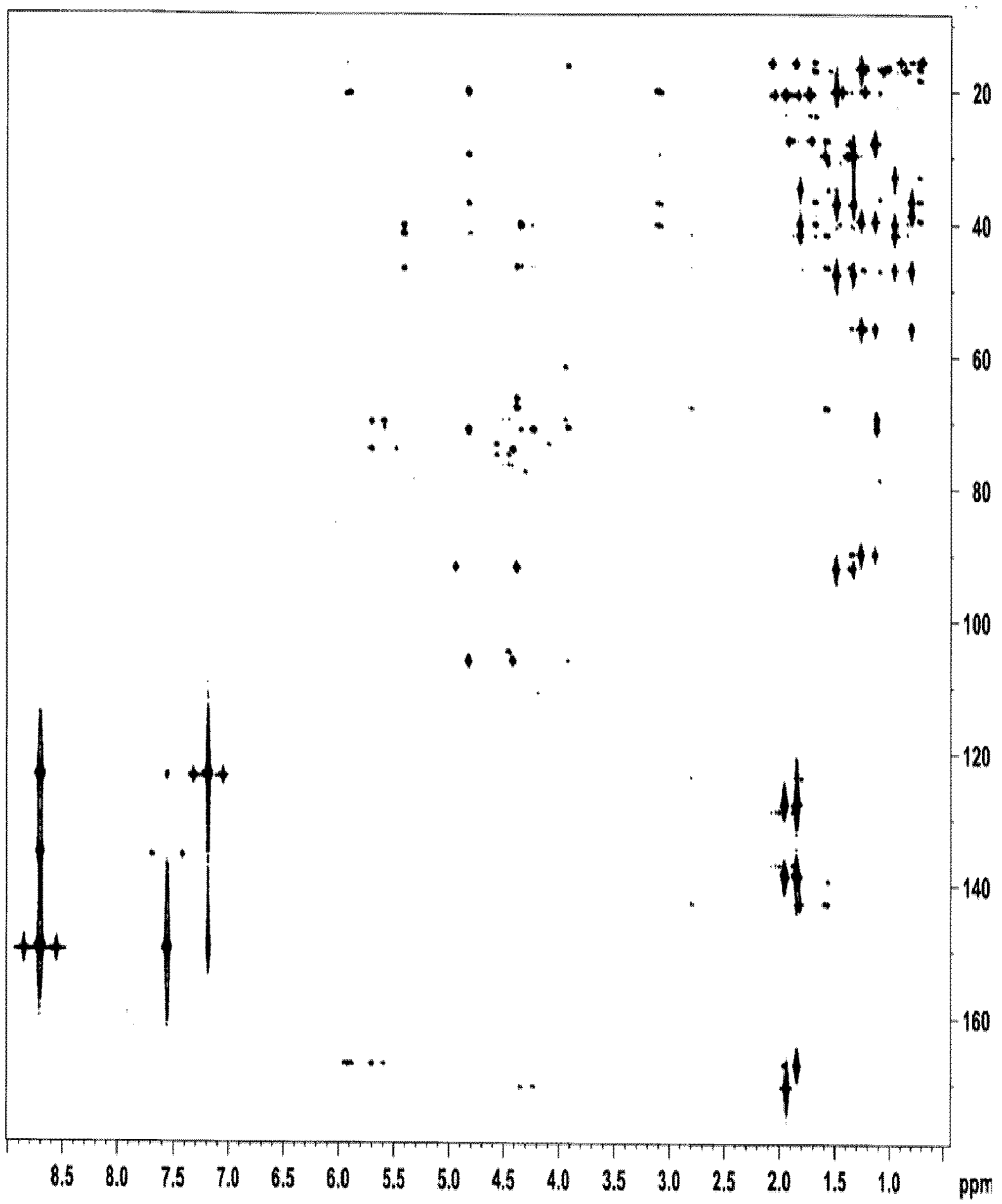

Figure 41
Structure of Y10

Y-10

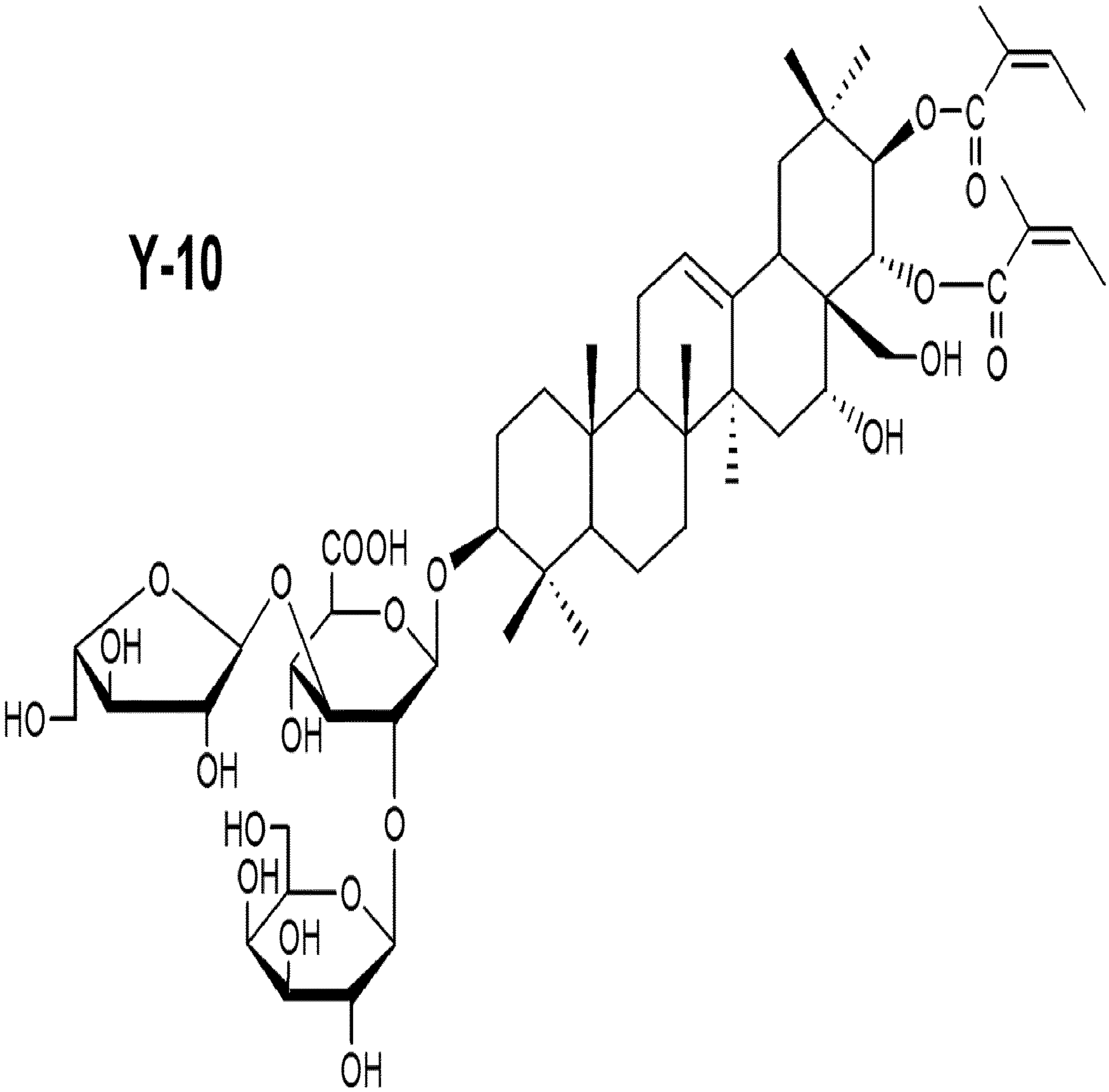

Figure 42
Y10-H-NMR

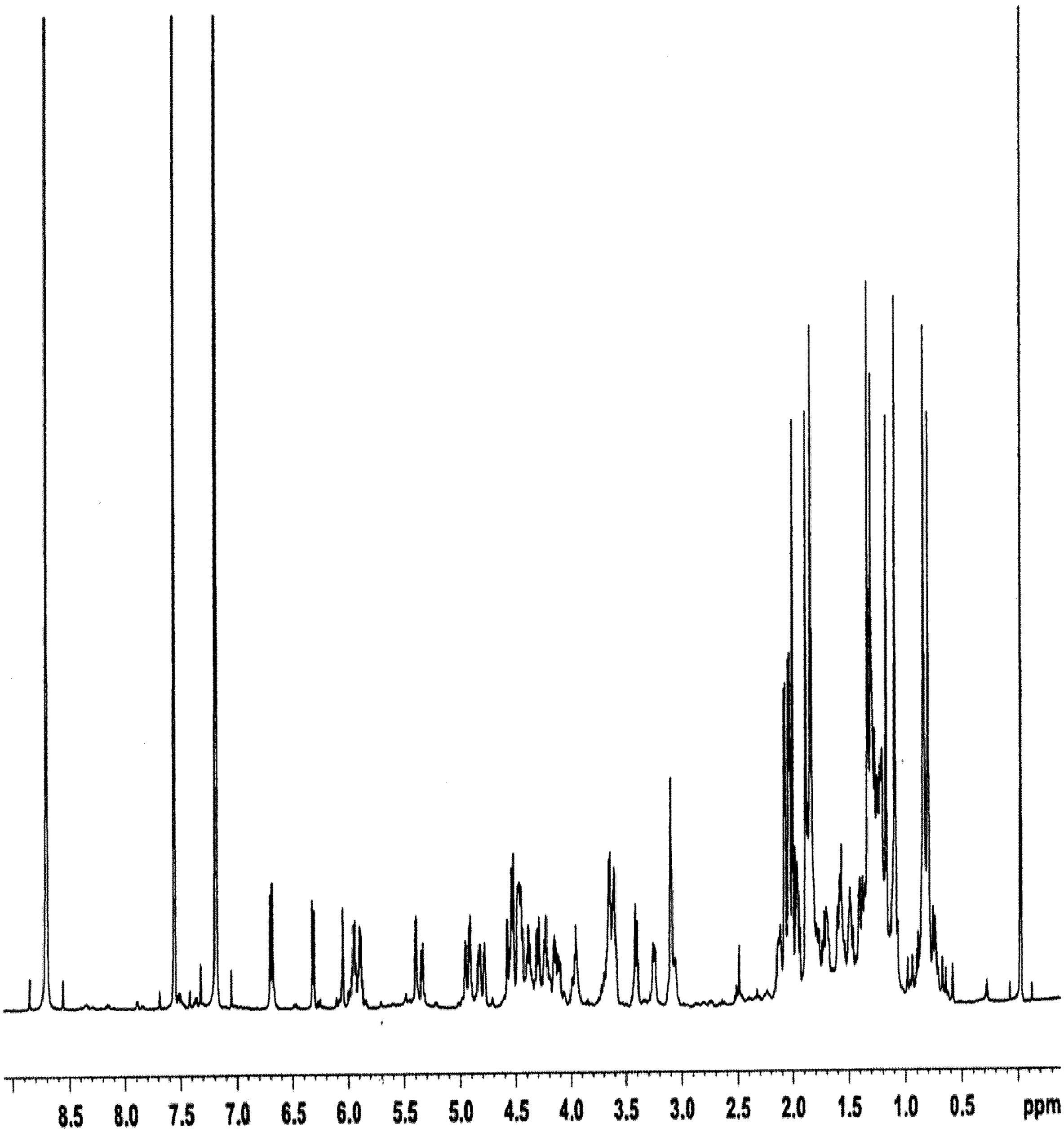

Figure 43
C13-NMR of Y10

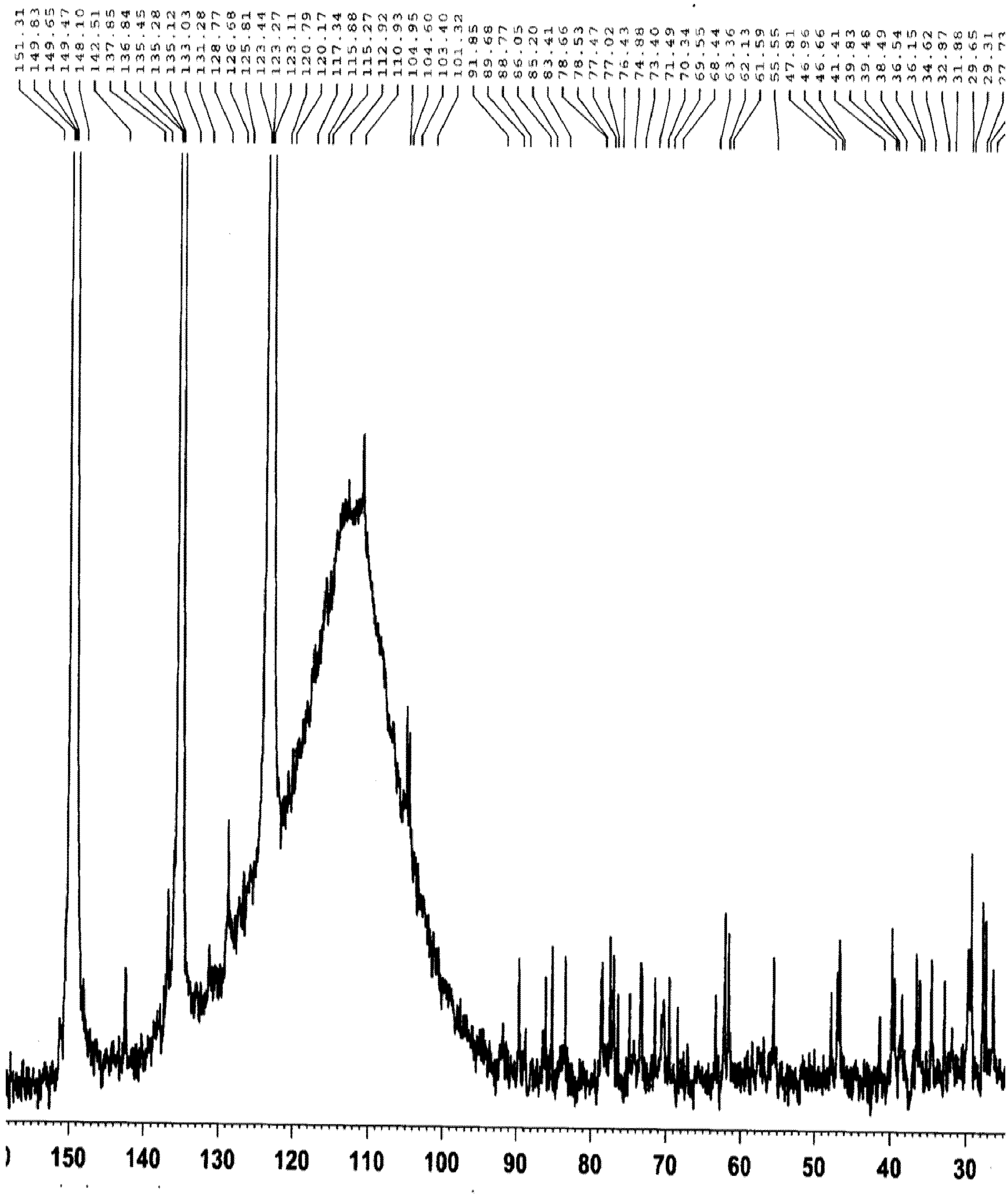

Figure 44
Y10-HMQC-level-1

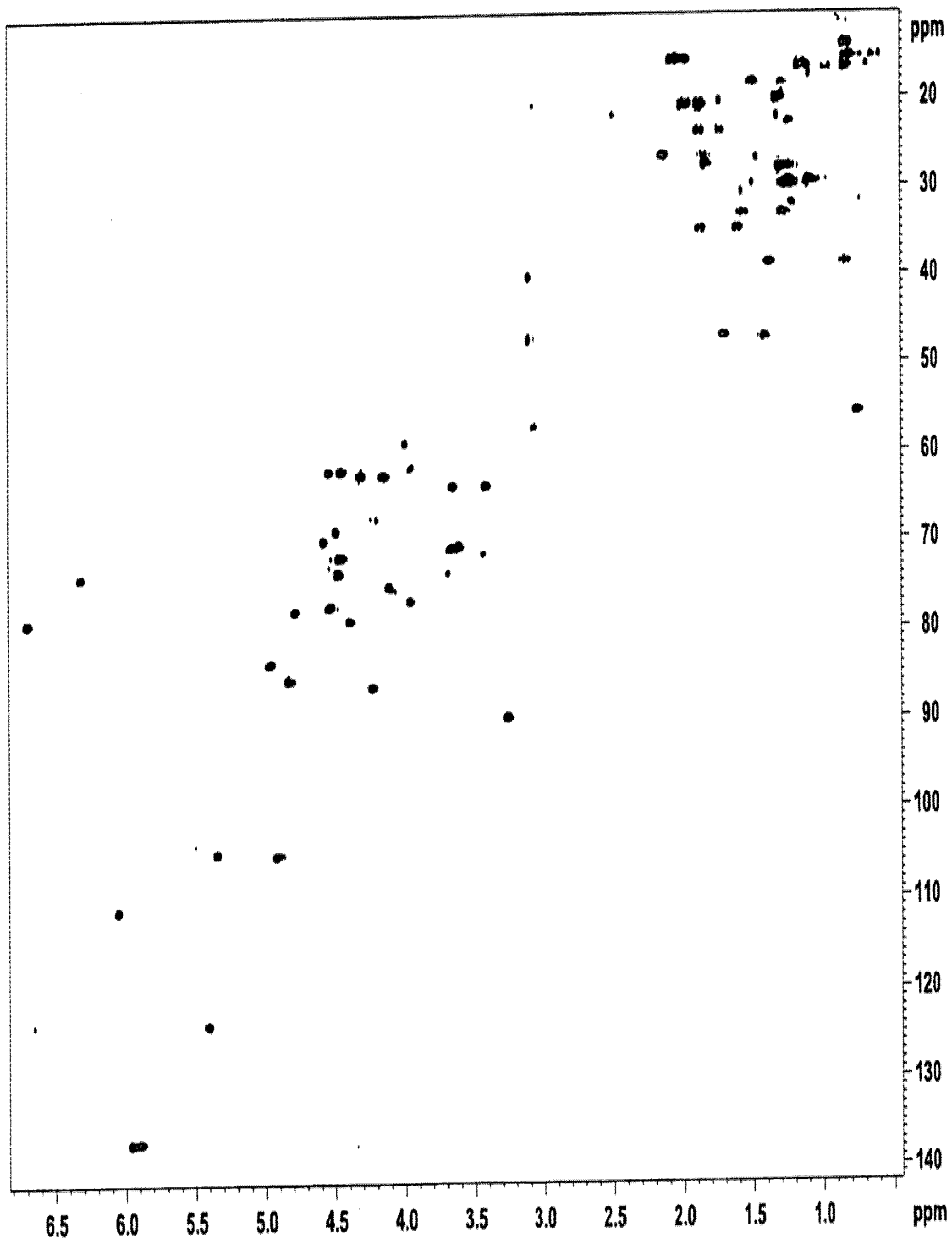

Figure 45

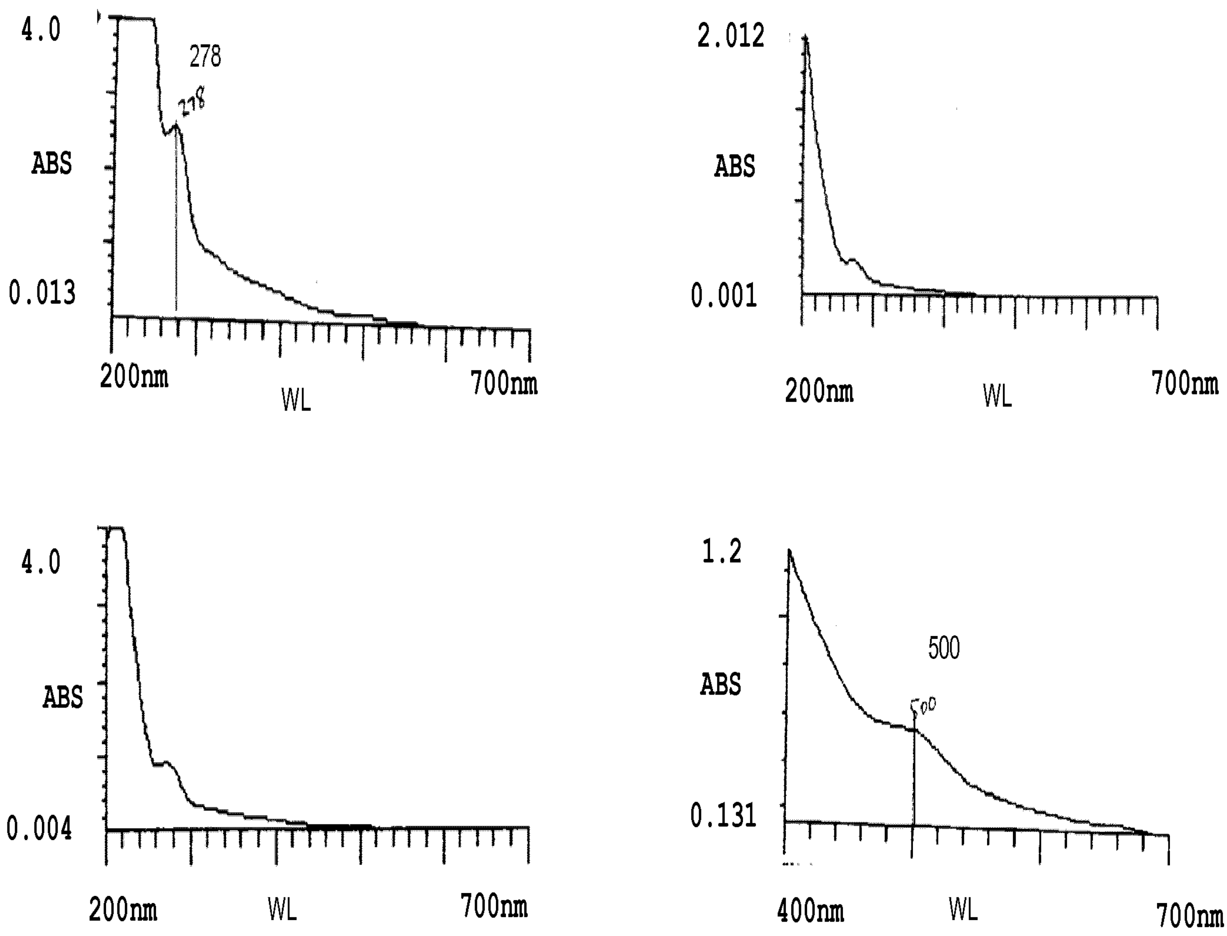
4.0
278
278
ABS
0.013
200nm
WL
700nm
2.012
ABS
0.001
200nm
WL
700nm
4.0
ABS
0.004
200nm
WL
700nm
1.2
500
500
ABS
0.131
400nm
WL
700nm

Figure 46
Effect of Compound X on the growth of OCAR-3 cells

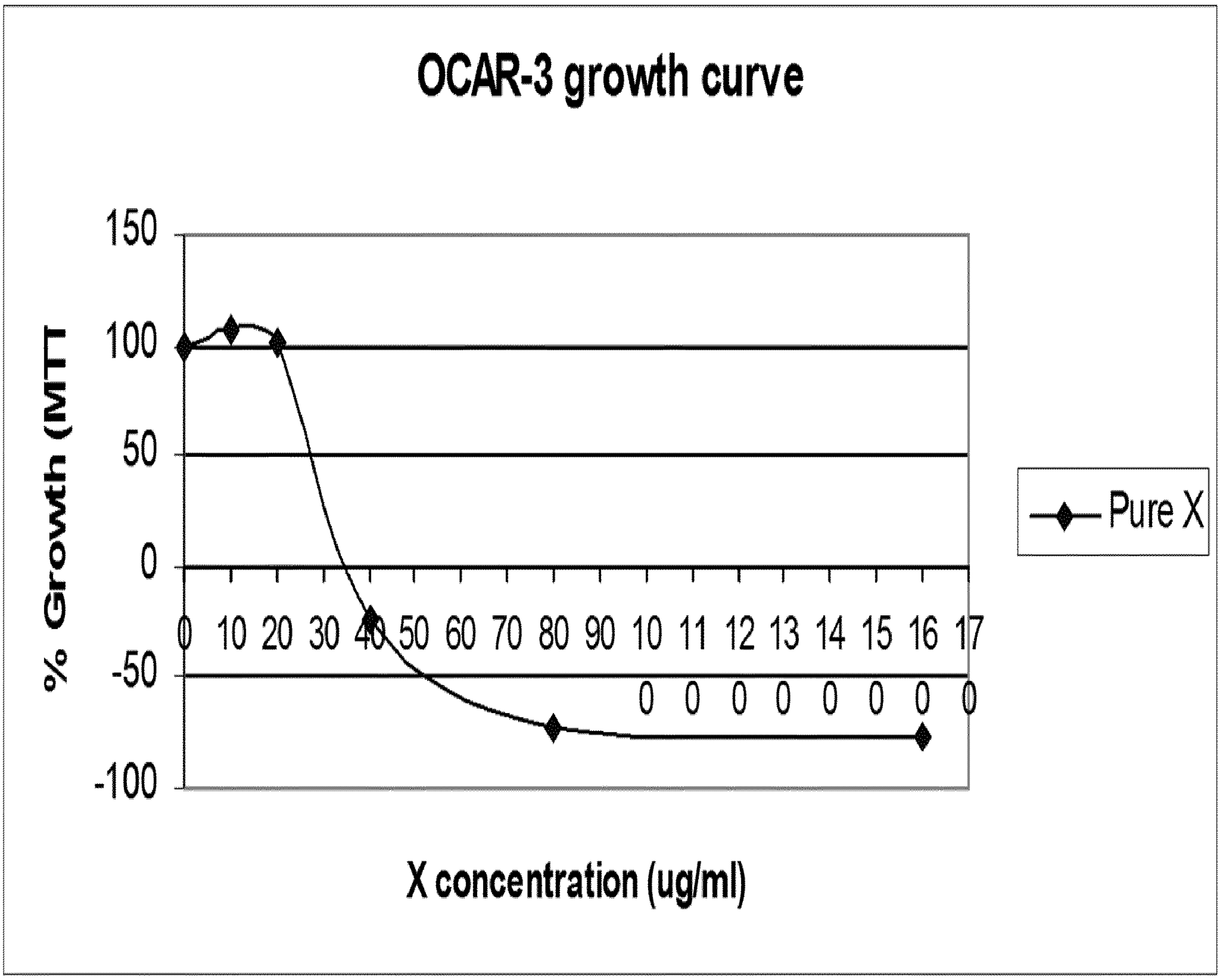

Figure 47
Chemical Structure of Compound X

$C_{58}H_{92}O_{22}$
Exact Mass: 1140.6080
Mol. Wt.: 1141.3379
C, 61.04; H, 8.12; O, 30.84

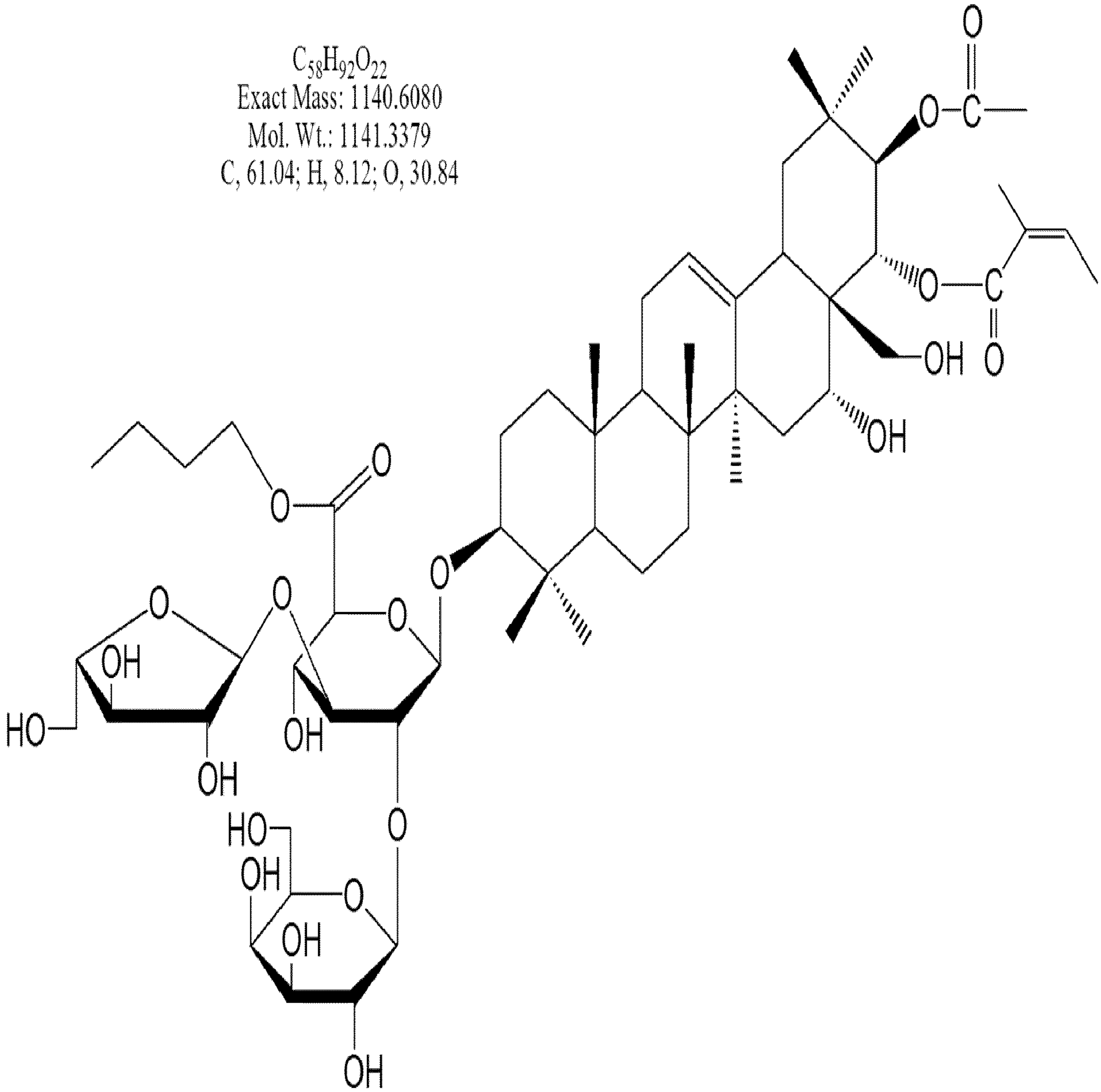

Figure 48
Haemolytic and Mtt activities of Compound Y

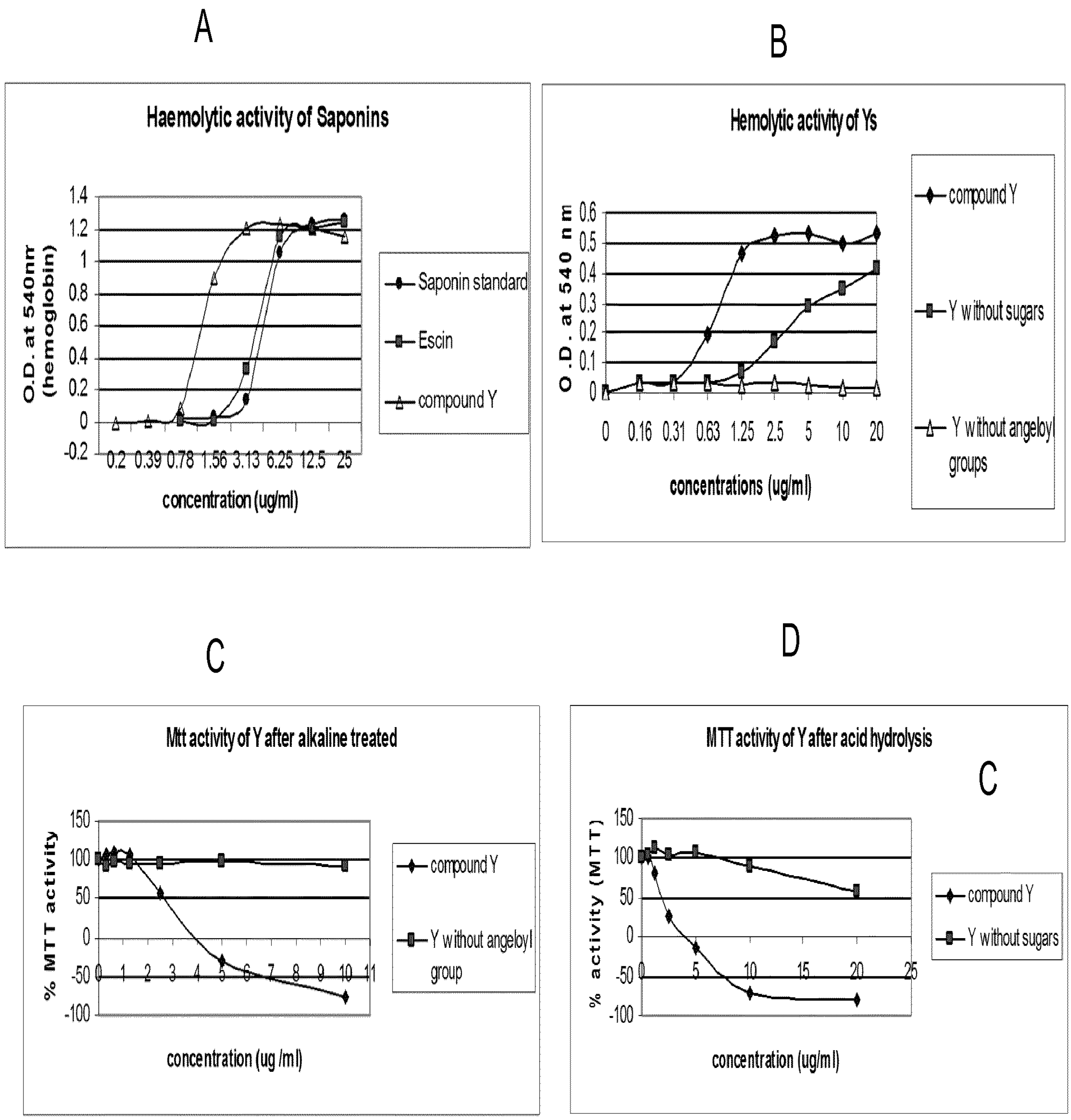

Figure 49

A

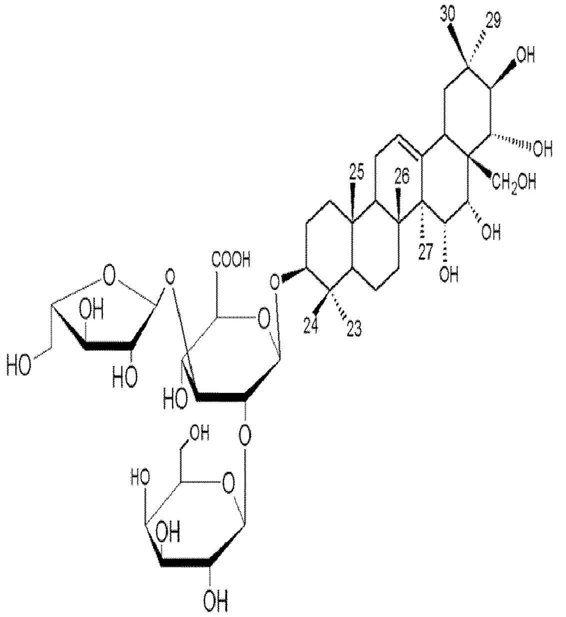

B

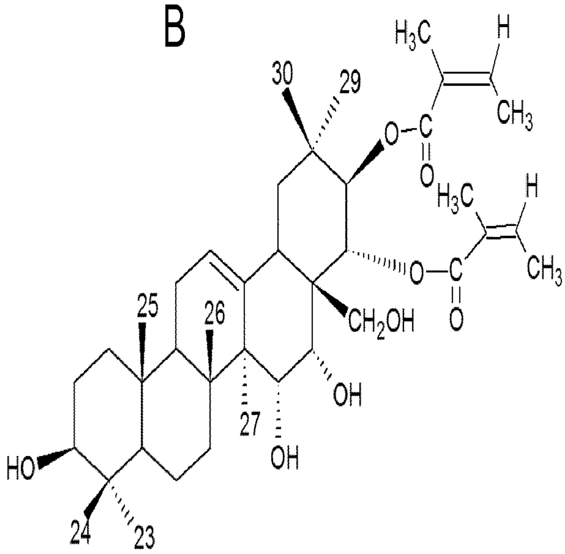

Figure 50
Structures of Compounds

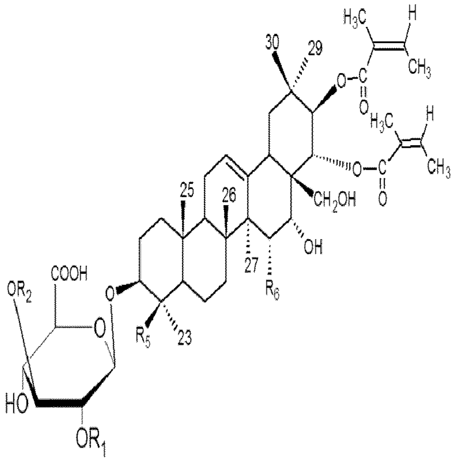

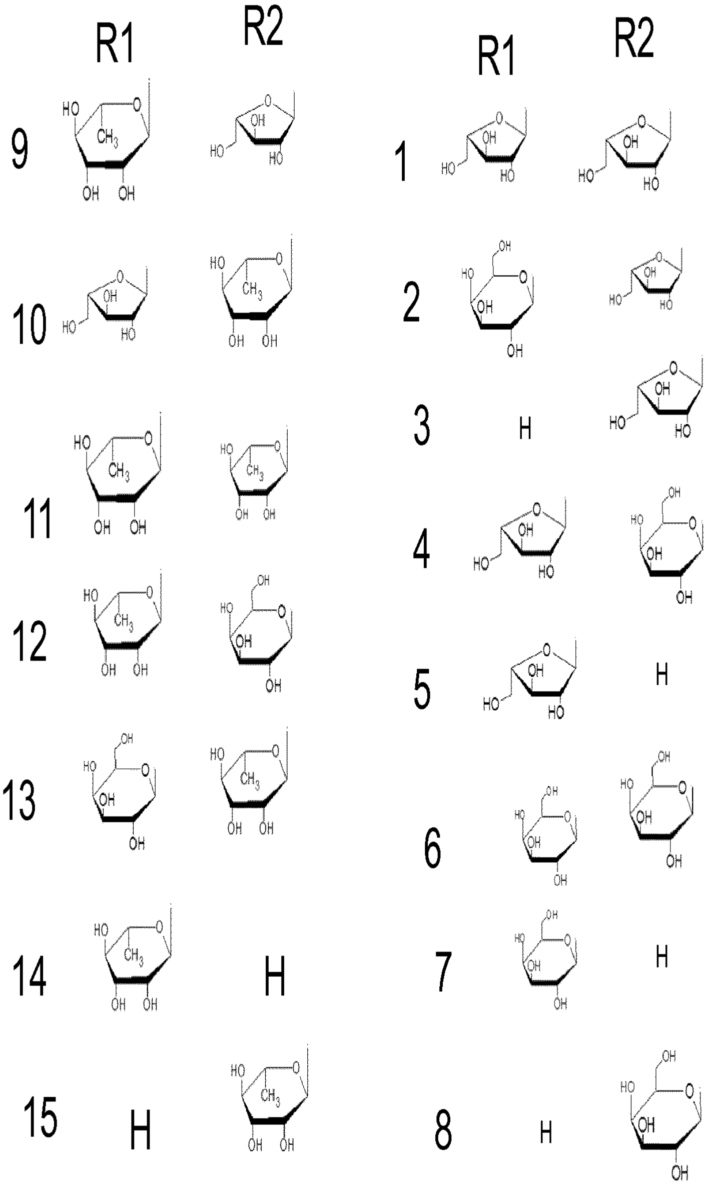

| Compound | R1 | R2 |
|---|---|---|
| 9 | | |
| 10 | | |
| 11 | | |
| 12 | | |
| 13 | | |
| 14 | | H |
| 15 | H | |
| 1 | | |
| 2 | | |
| 3 | H | |
| 4 | | |
| 5 | | H |
| 6 | | |
| 7 | | H |
| 8 | H | |

R6=H or OH

R5=CH3 or CH2OH

R1= D- glucose or D-galactose or L-rhamnose or L-arabinose or, D-xylose or alduronic acid or D- glucuronic acid or D-galacturonic acid

R2=D- glucose or D-galactose or L-rhamnose or L-arabinose or, D-xylose or alduronic acid or D- glucuronic acid or D-galacturonic acid

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 11131551 B
- US 11117760 B
- US 10906303 B
- US 0443465 W
- US 0433359 W
- US 60532101 B
- US 60509851 B
- US 60617379 B
- US 60613811 B
- US 60607858 B
- US 60675282 B
- US 60675284 B


### Non-patent literature cited in the description


- **YINGJIE CHEN ; TADAHIRO TAKEDA ; YUKIO OGIHARA.** *Chem. Pharm. Bull,* 1985, vol. 33 (4), 1387-1394
- **D. YINGJIE CHEN ; TADAHIRO TAKEDA ; YUKIO OGIHARA.** *Chem. Pharm. Bull,* 1985, vol. 33 (3), 1043-1048
- **YINGJIE CHEN ; TADAHIRO TAKEDA ; YUKIO OGIHARA.** *Chem. Pharm. Bull,* 1985, vol. 33 (1), 127-134
- **LAURENCE VOUTQUENNE ; CECILE KOKOUGAN ; CATHERINE LAVAUD ; ISABELLE POUNY ; MARC LITAUDON.** Triterpenoid saponins and Acylated prosapogenins from Harpullia austro-caledonica. *Phytochemistry,* 2002, vol. 59, 825-832
- **LAURENCE VOUTQUENNE et al.** Haemolytic acylated triterpenoil saponins from Harpullia austro-caledonica. *Phytochemistry,* 2005, vol. 66, 825-835
- **ZHONG JAING ; JEAN-FRANCOIS GALLARD ; MARIE-THERESE ADELINE ; VINCENT DUMONTET ; MAI VAN TRI ; THIERRY SEVENET ; MARY PAIS.** Six Triterpennoid Saponins from Maesa laxiflora. *J. Nat. Prod.,* 1999, vol. 62, 873-876
- **YOUNG SEO ; JOHN M. BERGER ; JENNINE HOCH ; KIM M NEDDERMANN ; ISIA BURSUKER ; STEVEN W. MAMBER ; DAVID G. KINGSTON.** A new Triterpene Saponin from Pittosporum viridiflorum from the Madagascar Rainforest. *J. Nat. Prod.,* 2002, vol. 65, 65-68
- **XIU-WEI YANG ; JING ZHAO ; XUE-HUI LUI ; CHAO-MEI MA ; MASAO HATTORI ; LI HE ZHANG.** Anti-HIV-1 Protease Triterpenoid Saponins from the Seeds of Aesculus chinensis. *J. Nat. Prod.,* 1999, vol. 62, 1510-1513
- **YI LU ; TATSUYA UMEDA ; AKIHITO YAGI ; KANZO SAKATA ; TIRTHANKAR CHAUDHURI ; D.K. GANGULY ; SECION SARMA.** Triterpenoid Saponins from the roots of the tea plant (Camellia sinensis var. Assamica). *Phytochchemistry,* 2000, vol. 53, 941-946
- **SANDRA APERS ; TESS E. DE BRUYNE ; MAGDA CLAEYS ; ARNOLD J. VILETINCK ; LUC A.C. PIETERS.** New acylated triterpenoid saponins from Maesa laceceolata. *Phytochemistry,* 1999, vol. 52, 1121-1131
- **ILARIA D'ACQUARICA ; MARIA CRISTINA ; DI GIOVANNI ; FRANCESCO GASPARRINI ; DOMENICO MISITI ; CLAUDIO D' ARRIGO ; NICOLINA FAGNANO ; DECIMO GUARNIERI ; GIOVANNI IACONO ; GIUSEPPE BIFULCO.** Isolation and structure elucidation of four new triterpenoid estersaponins from fruits of the Pittosporumtobira AIT. *Tetrahedron,* 2002, vol. 58, 10127-10136